# EUROPEAN PATENT APPLICATION

(11) **EP 3 581 095 A2**
(43) Date of publication of application: **18.12.2019**
(21) Application number: 18750669.6
(22) Date of filing: 07.02.2018
(51) Int. Cl.: A61B 5/00, A61B 5/11, A61B 5/091, G06Q 50/22

(54) **THORACIC MEASURING DEVICE, SCOLIOSIS CORRECTION SYSTEM, REMOTE SPINAL DIAGNOSTIC SYSTEM, AND WEARABLE MEASURING DEVICE**

(30) Priority: 08.02.2017 KR 20170017587; 12.05.2017 KR 20170059125; 26.07.2017 KR 20170094479; 30.11.2017 KR 20170162453
(71) Applicant: Bonebre Technology Co., Ltd, Busan 49241 (KR)
(72) Inventor: JUNG, Hwi Su, Busan 47616 (KR)
(74) Representative: Doherty, William
(86) International application number: PCT/KR2018/001663
(87) International publication number: WO 2018/147643

(57) **Abstract**

Provided is a chest measuring device configured to be attached to and detachable from a body and capable of inducing a correct posture of a subject and at the same time, correcting an abnormal alignment of the spine by analyzing measured values of left and right chests and generating vibrating motion to a chest that needs stimulation, a scoliosis correction system enabling a subject to conveniently measure his/her spinal condition alone without the help of others by including sensor units contacting left and right ribs and left and right transverse processes of lumbar vertebrae of the subject and also including a wearable internet of things (IoT) capable of detecting sensing values of muscles used to determine a spinal condition of the subject, a system for remotely diagnosing spine remotely diagnosing the spine of a patient by processing trunk movement data of the patient collected through a wearable measuring device, and a wearable measuring device enabling a subject to self-diagnose a spinal condition and correct a posture based on the result.

## Description

### TECHNICAL FIELD

One or more embodiments relate to a chest measuring device, a scoliosis correction system, a system for remotely diagnosing spine, and a wearable measuring device, and more particularly, to a chest measuring device measuring left and right chests of a body, a scoliosis correction system correcting scoliosis based on a measured value, a system for remotely diagnosing spine remotely diagnosing a spinal alignment state of a subject, and a wearable measuring device enabling a patient to self-treat by measuring a spinal condition.

### BACKGROUND

Scoliosis is defined as a symptom in which a spine is bent or twisted like a 'C'-shape or an 'S'-shape and thus a body is tilted or turned left or right, and is classified into functional scoliosis or structural scoliosis based on causes. Scoliosis induces lumbago and at the same time, affects a pulmonary function when an angle of curvature is higher than 70 to 80°, affects respiration when the angle of curvature is 90 to 100°, and causes a pulmonary heart disease due to lung capacity reduction when the angle of curvature is higher than 120°. Thus, interests in scoliosis are rapidly increasing every day.

Idiopathic scoliosis that has no known cause accounts for more than 80% of scoliosis, and scoliosis is caused by, in addition to genetic factors, environmental factors, such as a lifestyle, a bad posture, a desk inadequate to a body, and absence of health education. In particular, with the recent increase of working time at desks, the number of spine patients is increasing due to incorrect postures, and in this regard, various studies are being conducted to prevent spinal diseases by correcting incorrect postures in the early stage.

FIG. 1 is a flowchart of a system 100 for measuring spinal deformity disclosed in KR 10-1124144 (Title of Invention: System for Measuring Spinal Deformity).

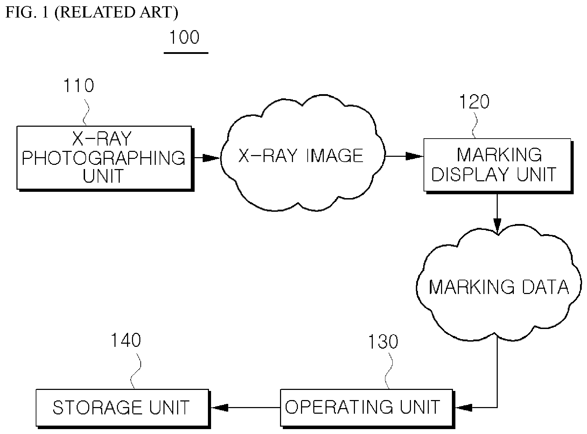

The system (hereinafter, referred to as a "first related art") 100 for measuring spinal deformity of FIG. 1 includes: an x-ray photographing unit 110 that generates a photographed result of a spine of an object as an x-ray image; a masking display unit 120 that receives and outputs, on a marking screen, the x-ray image generated by the x-ray photographing unit 110 and including a marking unit displaying reference vertical line generating points S1 and S2, or Sa and Sb, and measurement target reference points P1 and P2, or Pa and Pb on the marking screen; an operating unit 130 that generates reference vertical lines V1 and V2 based on the reference vertical line generating points S1 and S2, or Sa and Sb, and calculates relative location information of the measurement target reference points P1 and P2, or Pa and Pb or a measurement target point P3 or Pc derived from the measurement target reference points P1 and P2, or Pa and Pb based on the generated reference vertical lines V1 and V2; and a storage unit 140 that receives and stores the relative location information calculated by the operating unit 130.

The first related art 100 configured as such has a structural limitation of being applicable only to a hospital having the first related art 100 because an x-ray photographing unit that is expensive and difficult to be manipulated is essential to the first related art 100 in order to generate an x-ray image.

Also, even when the first related art 100 generates an x-ray image, an ordinary person who does not have medical knowledge of obtaining information about a spinal disease through the x-ray image is unable to use the x-ray image.

In addition, even when a doctor determines that the spine of a subject is deformed through data stored in the storage unit 140, the first related art 100 does not teach how to correct the deformed spine, and thus the first related art 100 is inconvenient and is unable to be used for personal purposes.

Accordingly, it is urgent to conduct studies on a spine correction system, in which 1) an apparatus for measuring spinal deformation is low-priced and simple to use, and 2) a correction value is automatically calculated based on measured data and an operation is performed based on the calculated correction value, such that an ordinary person who does not have medical knowledge conveniently uses the spine correction system.

FIG. 2 is a block diagram of system 200 for measuring scoliosis disclosed in KR 10-1043556 (Title of Invention: System and Method of Measuring Scoliosis).

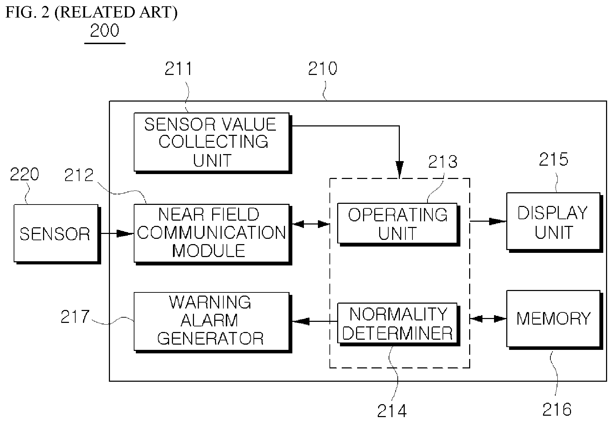

The system (hereinafter, referred to as a 'second related art') for measuring scoliosis of FIG. 2 includes a scoliosis measuring device 210 and a sensor 220. Here, the scoliosis measuring device 210 and the sensor 220 are configured to exchange data through a near field communication network.

The scoliosis measuring device 210 includes a sensor value collecting unit 211 collecting spinal curvature state information of a subject transmitted from the sensor 220, a near field communication module 212 supporting near field communication with the sensor 220, an operating unit 213 calculating a final Cobb's angle of the subject by analyzing the spinal curvature state information input from the sensor 220, a normality determiner 214 determining that a degree of lateral curvature of the spine is abnormal when the degree exceeds the final Cobb's angle of the subject calculated by the operating unit 213, a display unit 215 displaying normality, a memory 216 storing data, and a warning alarm generator 217 driven when the normality determiner 214 determines that the degree of lateral curvature of the spine is abnormal.

The second related art 200 configured as such may provide and transmit information that a spinal curvature state of the subject is abnormal through the normality determiner 214 and the warning alarm generator 217 when the spinal curvature state is abnormal. However, even when the subject recognizes that his/her spinal curvature state is abnormal, since the second related art 200 does not provide a function of how to correct the spine based on the final Cobb's angle, the subject merely straightens the posture or has to separately visit a medical facility to correct the spine.

Also, the final Cobb's angle 1) has different values based on locations of the spine to be measured, and 2) has remarkably low accuracy and precision when the final Cobb's angle is calculated through a sensor and the sensor is not attached to an accurate location corresponding to the central axis of the back. In other words, in the second related art 200, when the subject attaches the sensor alone or with an ordinary person who does not have medical knowledge, the sensor is attached by simply checking the central axis of the back by hands or naked eyes, and thus the accuracy of the final Cobb's angle actually calculated by the operating unit 213 may be low.

Accordingly, the second related art 200 may be used for personal purposes as it is configured to be attachable to and detachable from the body, but since the spinal curvature state is determined based on a Cobb's angle, the sensor needs to be attached to the accurate location corresponding to the central axis for accurate determination. Thus, it is practically difficult for an ordinary person who does not have medical knowledge to use the second related art 200.

### DISCLOSURE

### TECHNICAL PROBLEM

One or more embodiments include a chest measuring device configured to be attached to and detachable from a body and capable of inducing a correct posture of a subject and at the same time, correcting an abnormal alignment of the spine by analyzing measured values of left and right chests and generating vibrating motion to a chest that needs stimulation, a scoliosis correction system enabling a subject to conveniently measure his/her spinal condition alone without the help of others by including sensor units contacting left and right ribs and left and right transverse processes of lumbar vertebrae of the subject and also including a wearable internet of things (IoT) capable of detecting sensing values of muscles used to determine a spinal condition of the subject, a system for remotely diagnosing spine remotely diagnosing the spine of a patient by processing trunk movement data of the patient collected through a wearable measuring device, and a wearable measuring device enabling a subject to self-diagnose a spinal condition and correct a posture based on the result.

### TECHNICAL SOLUTION

According to one or more embodiments, a chest measuring device includes: first and second sensor units respectively including sensors detecting movement of left and right chests of a subject; a detaching unit configured to attach or detach the first and second sensor units to or from a chest of the subject; and a control unit configured to receive data measured by the sensor of the first sensor unit and the sensor of the second sensor unit, wherein the control unit includes: a first sensor unit processor configured to detect inhalation volume information of the left chest by analyzing data of the first sensor unit; a second sensor unit processor configured to detect inhalation volume information of the right chest by analyzing data of the second sensor unit; and a vibration determiner configured to calculate a difference between the inhalation volume information of the left chest detected by the first sensor unit processor and the inhalation volume information of the right chest detected by the second sensor unit processor, compare the calculated difference with a second threshold value that is defined as a largest difference between the inhalation volume information of the left and right chests during normal respiration, and determine that respiration is not normal due to inactivated a muscle of the chest caused by abnormal alignment of a spine when the difference is equal to or greater than the second threshold value.

The vibration determiner may further include: a left data detecting module configured to detect left data comprising the inhalation volume information of the lest chest by analyzing data input from the first sensor unit; a right data detecting module configured to detect right data including the inhalation volume information of the right chest by analyzing data input from the second sensor unit; and a third determining module configured to calculate the difference between the inhalation volume information of the left chest detected by the left data detecting module and the inhalation volume information of the right chest detected by the right data detecting module, compare the calculated difference with the second threshold value, and determine that the alignment of the spine is abnormal when the difference is equal to or greater than the second threshold value.

The left data detecting module may be configured to detect the left data further including exhalation volume information of the left chest and volume displacement value of the left chest during inhalation and exhalation, the right data detecting module may be configured to detect the right data further including exhalation volume information of the right chest and volume displacement value of the right chest during inhalation and exhalation, and the vibration determiner may further include: a first determining module configured to calculate a volume displacement value that is a difference between the inhalation volume information and the exhalation volume information of the left chest of the left data, compare the calculated volume displacement value with a first threshold value defined as a smallest value of a volume displacement value of inhalation and exhalation of the chest of which respiration is determined to be normal, and determine that respiration is not normal due to inactivated a muscle ofthe left chest caused by abnormal alignment of the spine when the calculated volume displacement value is smaller than the first threshold value; and a second determining module configured to calculate a volume displacement value that is a difference between the inhalation volume information and the exhalation volume information of the right chest of the right data, compare the calculated volume displacement value with the first threshold value, and determine that respiration is not normal due to inactivated a muscle of the right chest caused by abnormal alignment of the spine when the calculated volume displacement value is smaller than the first threshold value.

The first and second sensor units may each include: a gyrosensor configured to detect angular speeds of X-, Y-, and Z-axes that are perpendicular to each other; an acceleration sensor configured to detect acceleration of the X-, Y-, and Z-axes that are perpendicular to each other; and a geomagnetic sensor.

The first and second sensor units may each include a vibrator, wherein the control unit may be configured to output control data for vibrating the vibrator of the first sensor unit when the first determining module determines that respiration is not normal, output control data for vibrating the vibrator of the second sensor unit when the second determining module determines that respiration is not normal, and output control data to one of the first and second sensor units, which corresponds to a chest having smaller inhalation volume information when the third determining module determines that respiration is not normal, and the first and second sensor units may each operate the vibrators when the control data is received from the control unit.

The first and second sensor units may further include pressurizing units configured to selectively raise or lower the vibrators of the first and second sensor units, wherein the control unit may include a raised height detector driven when the vibration determiner determines that respiration is not normal and configured to detect a raised height corresponding to exhalation volume information of a chest of which respiration is determined to be abnormal by the vibration determiner by searching a reference table in which raised height of the pressurizing unit are matched per exhalation volume information of the chest, and the first and second sensor units may vibrate the vibrators after controlling the pressurizing units according to the raised height received from the control unit.

According to one or more embodiments, a scoliosis correction system includes: a wearable internet of things (IoT) including at least one sensor unit that includes a sensor and a vibrator, the sensor contacting a body of a subject and detecting movement of a muscle of the contacted body, a detaching unit configured to attach or detach the at least one sensor unit to or from the body of the subject, and a control unit configured to externally transmit a sensing value when the sensing value measured by the sensor of the at least one sensor unit is received; and a portable terminal in which a spine management application analyzing the sensing value received from the wearable IoT is installed, wherein the spine management application determines whether the muscle needs to be vibrated by analyzing the sensing value when the sensing value is received from the wearable IoT, and when it is determined that the muscle needs vibration, transmits vibration information to the wearable IoT by controlling the portable terminal.

The spine management application may include: a data analyzer configured to analyze the sensing value received from the at least one sensing unit via a pre-set analysis algorithm, and detect a motion vector of the muscle corresponding to a location where the at least one sensor unit is attached; and a vibration determiner configured to detect activity of the muscle by analyzing the motion vector detected by the data analyzer via a pre-set activity detection algorithm, compare the detected activity with a pre-set threshold value defined as a smallest value of muscle activity in which respiration is determined to be normal or the muscle is determined to be activated, and determine that the muscle needs to be vibrated when the detected activity is smaller than the pre-set threshold value, wherein the spine management application may transmit the vibration information to the wearable IoT by controlling the portable terminal when the vibration determiner determines that the muscle needs to be vibrated, and the wearable IoT may be configured to drive the vibrator of the at least one sensor unit when the vibration information is received from the portable terminal.

The at least one sensor unit may further include a pressurizing unit configured to selectively raise or lower the vibrator, the spine management application may further include a vibration information detector configured to be driven when the vibration determiner determines that the muscle needs to be vibrated, analyze the motion vector of the muscle detected by the data analyzer via a pre-set vibration intensity and height detection algorithm, and generate vibration information comprising optimum vibration intensity and an optimum raised height of the vibrator, which corresponds to the motion vector, and the wearable IoT may be further configured to enable the pressurizing unit to adjust a length of the vibrator based on the optimum raised height of the vibration information received from the spine management application, and vibrate the vibrator according to the optimum vibration intensity of the received vibration information.

The at least one sensor unit may include: a first sensor unit contacting the back of the subject corresponding to left ribs; a second sensor unit contacting the back of the subject corresponding to right ribs; a third sensor unit contacting the back of the subject corresponding to left transverse processes of lumbar vertebrae; and a fourth sensor unit contacting the back of the subject corresponding to right transverse processes of lumbar vertebrae, the data analyzer may further include: a first data detection module configured to analyze a sensing value measured by the first sensor unit; a second data detection module configured to analyze a sensing value measured by the second sensor unit; a third data detection module configured to analyze a sensing value measured by the third sensor unit; and a fourth data detection module configured to analyze a sensing value measured by the fourth sensor unit, the vibration determiner may be further configured to determine vibration of the first through fourth sensor units, the vibration information detector may be further configured to detect vibration information with respect to a sensor unit determined that vibration is needed by the vibration determiner, and the wearable IoT may be further configured to drive a vibrator of the sensor unit when the vibration information is received.

The first data detection module may be further configured to analyze the sensing value measured by the first sensor unit via the pre-set analysis algorithm and detect first inhalation detailed information indicating a motion vector of a muscle corresponding to the left ribs during inhalation, first exhalation detailed information indicating a motion vector of the muscle corresponding to the left ribs during exhalation, and first displacement information indicating a displacement vector of the first inhalation detailed information and the first exhalation detailed information, the second data detection module may be further configured to analyze the sensing value measured by the second sensor unit via the pre-set analysis algorithm and detect second inhalation detailed information indicating a motion vector of a muscle corresponding to the right ribs during inhalation, second exhalation detailed information indicating a motion vector of the muscle corresponding to the right ribs during exhalation, and second displacement information indicating a displacement vector of the second inhalation detailed information and the second exhalation detailed information, the third data detection module may be further configured to analyze the sensing value measured by the third sensor unit via the pre-set analysis algorithm and detect third inhalation detailed information indicating a motion vector of a muscle corresponding to the left transverse processes of lumbar vertebrae during inhalation, third exhalation detailed information indicating a motion vector of the muscle corresponding to the left transverse processes of lumbar vertebrae during exhalation, and third displacement information indicating a motion vector of the third inhalation detailed information and the third exhalation detailed information, and the fourth data detection module may be further configured to analyze the sensing value measured by the fourth sensor unit via the pre-set analysis algorithm and detect fourth inhalation detailed information indicating a motion vector of a muscle corresponding to the right transverse processes of lumbar vertebrae during inhalation, fourth exhalation detailed information indicating a motion vector of the muscle corresponding to the right transverse processes of lumbar vertebrae during exhalation, and fourth displacement information indicating a displacement vector of the fourth inhalation detailed information and the fourth exhalation detailed information.

The first through fourth sensor units may each include: a gyrosensor configured to detect an angular speed of X-, Y-, and Z-axes perpendicular to each other; an acceleration sensor configured to detect acceleration of the X-, Y-, and Z-axes perpendicular to each other; and a geomagnetic sensor.

The scoliosis correction system may further include a management server configured to, upon receiving information about the motion vectors of the first through fourth sensor units from the spine management application, detect a respiration pattern and a spinal alignment state of the subject by analyzing the received motion vectors of the muscles via a pre-set respiration pattern and spinal alignment state detection algorithm, detect optimum rotational angular breathing and an optimum training posture corresponding to the detected respiration pattern and spinal alignment state by searching a reference table in which rotational angular breathing and training postures are matched per respiration pattern and spinal alignment state, and transmit information about the detected optimum rotational angular breathing and training posture to the spine management application, wherein the spine management application may display, on a monitor of the portable terminal, the optimum rotational angular breathing and training posture received from the management server.

According to one or more embodiments, a system for remotely diagnosing spine includes: a patient interface unit configured to receive trunk movement data of a patient, which is generated when a wearable measuring device measures the patient, from a patient terminal; a database unit storing the trunk movement data; a diagnostic data generator configured to generate diagnostic data about the patient based on the trunk movement data ofthe patient; and a medical staff interface unit configured to provide the diagnostic data about the patient to a medical staff terminal.

The wearable measuring device may include: a first sensor unit configured to detect movement of a left chest of the patient; a second sensor unit configured to detect movement of a right chest of the patient; and a first near field communication unit configured to transmit data output from the first and second sensor units to the patient terminal.

The first and second sensor units may each include an acceleration sensor configured to detect acceleration with respect to at least one axis direction.

The patient terminal may be configured to generate a left chest displacement vector related to movement of the left chest of the patient based on the data output from the first sensor unit, and generate a right chest displacement vector related to movement of the right chest of the patient based on the data output from the second sensor unit.

The diagnostic data generator may be further configured to generate lung capacity data related to lung capacity of the left or right chest of the patient by receiving the left chest displacement vector or the right chest displacement vector of the patient.

The diagnostic data generator may be further configured to: detect a left or right chest inhalation displacement vector corresponding to inhalation of the patient and left or right chest exhalation displacement vector corresponding to exhalation of the patient, from among the received left or right chest displacement vector, calculate a first vector between the left or right chest inhalation displacement vector and the left or right chest exhalation displacement vector, and output half of a size of the first vector as the lung capacity data of the left or right chest.

The wearable measuring device may further include: a first stimulator configured to stimulate a left body portion of the patient; and a second stimulator configured to stimulate a right body portion of the patient, wherein the patient terminal may control the first stimulator to operate when the lung capacity data of the left chest is smaller than pre-set reference lung capacity, and control the second stimulator to operate when the lung capacity data of the right chest is smaller than the pre-set reference lung capacity.

The diagnostic data generator may be further configured to generate chest asymmetry data about asymmetry of the left chest and the right chest of the patient by receiving the left chest displacement vector and the right chest displacement vector of the patient.

The diagnostic data generator may be further configured to: detect a left chest inhalation displacement vector or a left chest exhalation displacement vector corresponding to inhalation or exhalation of the patient, from among the received left chest displacement vector, detect a right chest inhalation displacement vector or a right chest exhalation displacement vector corresponding to inhalation or exhalation of the patient, from among the received right chest displacement vector, calculate a second vector between the left chest inhalation displacement vector or the left chest exhalation displacement vector and the right chest inhalation displacement vector or the right chest exhalation displacement vector, and output a size of the second vector as the chest asymmetry data.

The wearable measuring device may further include: a first stimulator configured to stimulate a left body portion of the patient; and a second stimulator configured to stimulate a right body portion of the patient, wherein the patient terminal may be further configured to: control the first stimulator to operate when the chest asymmetry data is greater than a pre-set first threshold value and a size of the left chest inhalation displacement vector or the left chest exhalation displacement vector is smaller than a size of the right chest inhalation displacement vector or the right chest exhalation displacement vector, and control the second stimulator to operate when the chest asymmetry data is greater than the pre-set first threshold value and the size of the right chest inhalation displacement vector or the right chest exhalation displacement vector is smaller than the size of the left chest inhalation displacement vector or the left chest exhalation displacement vector.

The wearable measuring device may further include: a third sensor unit configured to detect movement of a left waist portion of the patient; and a fourth sensor unit configured to detect movement of a right waist portion of the patient, wherein the first near field communication unit may transmit data output from the third and fourth sensor units to the patient terminal.

The patient terminal may be further configured to: generate a left waist portion displacement vector about movement of the left waist portion of the patient based on the data output from the third sensor unit, and generate a right waist portion displacement vector about movement of the right waist portion of the patient based on the data output from the fourth sensor unit.

The diagnostic data generator may be further configured to generate waist portion asymmetry data about asymmetry between the left waist portion and the right waist portion of the patient by receiving the left waist portion displacement vector and the right waist portion displacement vector of the patient.

The diagnostic data generator may be further configured to: generate a third vector between the left waist portion displacement vector and the right waist portion displacement vector, and output a size of the third vector as the waist portion asymmetry data.

The patient terminal may be further configured to: control the first stimulator to operate when the waist portion asymmetry data is greater than a pre-set second threshold value and a size of the left waist portion displacement vector is smaller than a size of the right waist portion displacement vector, and control the second stimulator to operate when the waist portion asymmetry data is greater than the pre-set second threshold value and the size of the right waist portion displacement vector is smaller than the size of the left waist portion displacement vector.

The diagnostic data generator may be further configured to generate left or right trunk balance data about balance between the left or right chest and the left or right waist portion by receiving the left or right chest displacement vector and the left or right waist portion displacement vector of the patient.

The diagnostic data generator may be further configured to calculate a fourth vector between the left or right chest displacement vector and the left or right waist portion displacement vector, and output the fourth vector as the left or right trunk balance data.

The patient terminal may be further configured to: control the first stimulator to operate when the left trunk balance data is outside a pre-set reference vector range, and control the second stimulator to operate when the right trunk balance data is outside the pre-set reference vector range.

The patient terminal may include: a second near field communication unit configured to exchange data with the wearable measuring device; an input unit configured to receive a command for driving the patient terminal; a memory unit configured to store data received from the wearable measuring device and information about the wearable measuring device; a processor configured to generate spine health information indicating a spine health state of the patient based on the trunk movement data of the patient; and a display unit configured to display the spine health information of the patient.

The input unit may be further configured to receive a command for executing a posture measuring function from the patient, the processor may be further configured to receive data from the wearable measuring device through the second near field communication unit for a pre-set time, generate the trunk movement data of the patient based on the received data, and invoke spine health state data corresponding to the trunk movement data in preparation for spine health state instruction data stored in the memory unit and the display unit may be further configured to display the invoked spine health state data.

The spine health state instruction data may include the trunk movement data and the spine health state data matched to the trunk movement data.

The input unit may be further configured to receive at least one of a training time, a stimulation cycle, and a repetition cycle, and the processor may be further configured to: transmit a control signal to the wearable measuring device according to the training time such that the wearable measuring device is activated for the training time, transmit a control signal to the wearable measuring device such that duration of stimulation applied by the first and second stimulators changes according to the stimulation cycle, and transmit a control signal to the wearable measuring device according to the repetition cycle such that the wearable measuring device is repeatedly activated according to the repetition cycle.

The processor may be further configured to calculate a spine score of the patient based on the trunk movement data, and the display unit may be further configured to display the calculated spine score.

The processor may be further configured to calculate the spine score such that the spine score is high when the lung capacity data is large, and is high when the trunk asymmetry data is small.

The processor may be further configured to: calculate lung capacity data of the left and right chests by adding the lung capacity data of the left chest and the lung capacity data of the right chest, calculate the trunk asymmetry data by adding the chest asymmetry data and the waist portion asymmetry data, and calculate the spine score by dividing the lung capacity data of the left and right chests by the trunk asymmetry data.

According to one or more embodiments, a wearable measuring device includes: a first stretch sensor unit configured to detect movement of a left trunk of a patient; a second stretch sensor unit configured to detect movement of a right trunk of the patient; and a first near field communication unit configured to transmit data output from the first and second stretch sensor units to a patient terminal.

The first stretch sensor unit may be provided at a portion of an object worn on the patient, which interacts with the left trunk, and the second stretch sensor unit may be provided at a portion of the object worn on the patient, which interacts with the right trunk.

The first and second stretch sensor units may be installed at a chest band surrounding a chest on a top worn by the patient.

The first stretch sensor unit may be further configured to output, to the first near field communication unit, first stretching amount data indicating a left chest stretching amount according to movement of a left chest of the patient, and the second stretch sensor unit may be further configured to output, to the first near field communication unit, second stretching amount data indicating a right chest stretching amount according to movement of a right chest of the patient.

The patient terminal may be configured to obtain the left chest stretching amount based on the first stretching amount data and obtain the right chest stretching amount based on the second stretching amount data.

The wearable measuring device may further include: a first stimulator configured to stimulate a left body portion of the patient; and a second stimulator configured to stimulate a right boy portion of the patient, wherein the patient terminal may be further configured to: control the first stimulate to operate when the left chest stretching amount is smaller than a pre-set reference chest stretching amount, and control the second stimulator to operate when the right chest stretching amount is smaller than the pre-set reference chest stretching amount.

The wearable device may further include: a first stimulator configured to stimulate a left body portion of the patient; and a second stimulator configured to stimulate a right body portion of the patient, wherein the patient terminal may be further configured to: calculate a first difference between the left chest stretching amount and the right chest stretching amount, control the first stimulator to operate when the calculated first difference is greater than a pre-set first reference value and the left chest stretching amount is smaller than the right chest stretching amount, and control the second stimulator to operate when the calculated first difference is greater than the pre-set first reference value and the right chest stretching amount is smaller than the left chest stretching amount.

The wearable measuring device may further include: a third stretch sensor unit configured to detect movement of a left waist portion of the patient; a fourth stretch sensor unit configured to detect movement of a right waist portion of the patient; and an auxiliary near field communication unit configured to transmit data output from the third and fourth stretch sensor units to the patient terminal.

The third stretch sensor unit may be provided at a portion of the object worn on the patient, which interacts with the left waist portion, and a fourth stretch sensor unit may be provided at a portion of the object worn on the patient, which interacts with the right waist portion.

The third and fourth stretch sensor units may be installed at a waist band surrounding a waist portion on clothing worn by the patient.

The third stretch sensor unit may be further configured to output, to the auxiliary near field communication unit, third stretching amount data indicating a left waist portion stretching amount by movement of the left waist portion of the patient, and the fourth stretch sensor unit may be further configured to output, to the auxiliary near field communication unit, fourth stretching amount data indicating a right waist portion stretching amount by movement of the right waist portion of the patient.

The patient terminal may be further configured to obtain the left waist portion stretching amount based on the third stretching amount data, and obtain the right waist portion stretching amount based on the fourth stretching amount data.

The wearable measuring device may further include: a first stimulator configured to stimulate a left body portion of the patient; and a second stimulator configured to stimulate a right body portion of the patient, wherein the patient terminal is further configured to: calculate a second difference between the left waist portion stretching amount and the right waist portion stretching amount, control the first stimulator to operate when the calculated second difference is greater than a pre-set second reference value and the left waist portion stretching amount is smaller than the right waist portion stretching amount, and control the second stimulator to operate when the calculated second difference is greater than the pre-set second reference value and the right waist portion stretching amount is smaller than the left waist portion stretching amount.

The wearable measuring device may further include: a first stimulator configured to stimulate a left body portion of the patient; and a second stimulator configured to stimulate a right body portion of the patient, wherein the patient terminal is further configured to: calculate a third difference between the left chest stretching amount the left waist portion stretching amount, control the first stimulator to operate when the calculated third difference is outside a pre-set reference range, calculate a fourth difference between the right chest stretching amount and the right waist portion stretching amount, and control the second stimulator to operate when the calculated fourth difference is outside the pre-set reference range.

According to one or more embodiments, an application for executing a spine diagnosing method includes: receiving, from a wearable measuring device, first stretching amount data indicating a left chest stretching amount of a patient and second stretching amount data indicating a right chest stretching amount of the patient; obtaining the left and right chest stretching amounts respectively from the first and second stretching amount data; controlling a first stimulator of the wearable measuring device to operate when the left chest stretching amount is smaller than a pre-set reference chest stretching amount, the first simulator configured to stimulate a left body portion of the patient; and controlling a second stimulator of the wearable measuring device to operate when the right chest stretching amount is smaller than the pre-set reference chest stretching amount, the second stimulator configured to stimulate a right body portion of the patient.

The spine diagnosing method may further include: calculating a first difference between the left chest stretching amount and the right chest stretching amount; controlling the first stimulator to operate when the calculated first difference is greater than a pre-set first reference value and the left chest stretching amount is smaller than the right chest stretching amount; and controlling the second stimulator to operate when the calculated first difference is greater than the pre-set first reference value and the right chest stretching amount is smaller than the left chest stretching amount.

The spine diagnosing method may further include: receiving, from the wearable measuring device, third stretching amount data indicating a left waist portion stretching amount of the patient and fourth stretching amount data indicating a right waist portion stretching amount of the patient; obtaining the left and right waist portion stretching amounts respectively from the third and fourth stretching amount data; calculating a second difference between the left waist portion stretching amount and the right waist portion stretching amount; controlling the first stimulator to operate when the calculated second difference is greater than a pre-set second reference value and the left waist portion stretching amount is smaller than the right waist portion stretching amount; and controlling the second stimulator to operate when the calculated second difference is greater than the pre-set second reference value and the right waist portion stretching amount is smaller than the left waist portion stretching amount.

The spine diagnosing method may further include: calculating a third difference between the left chest stretching amount and the left waist portion stretching amount; controlling the first stimulator to operate when the calculated third difference is outside a pre-set reference range; calculating a fourth difference between the right chest stretching amount and the right waist portion stretching amount; and controlling the second stimulator to operate when the calculated fourth difference is outside the pre-set reference range.

### ADVANTAGEOUS EFFECTS

The chest measuring device according to a first embodiment of the present invention may be configured to be attached to and detached from a body and capable of analyzing the measured values of left and right chests and generating a vibrating motion to a chest that needs stimulation, thereby inducing a correct posture of a subject and simultaneously correcting an abnormal alignment of the spine.

The scoliosis correction system according to a second embodiment of the present invention may include sensor units attachable or detachable to or from the body, while contacting the left and right ribs and the left and right transverse processes of lumbar vertebrae of the subject, and also includes the wearable IoT capable of detecting the sensing values of muscles used to determine the spinal condition of the subject, thereby enabling the subject to conveniently measure his/her spinal condition alone without the help of others.

The system for remotely diagnosing spine according to a third embodiment of the present invention is capable of diagnosing a patient's spine in a remote place by processing the patient's trunk movement data generated in the patient terminal, which is based on the raw data collected by a wearable measuring device a patient wears.

A wearable measuring device according to a fourth embodiment of the present invention may enable a subject to self-diagnose a spinal condition and enable the subject wearing the wearable measuring device to correct a posture based on the result of diagnosing the spinal condition, thus eventually enabling the cognitive therapy.

### BRIEF DESCRIPTION OF THE DRAWINGS

FIG. 1 is a flowchart of a system for measuring spinal deformity disclosed in KR 10-1124144 (Title of Invention: System for Measuring Spinal Deformity);
FIG. 2 is a block diagram of system for measuring scoliosis disclosed in KR 10-1043556 (Title of Invention: System and Method of Measuring Scoliosis);
FIG. 3 is a perspective view of a chest measuring device according to an embodiment;
FIG. 4 is an exploded perspective view of a first sensor unit of FIG. 3;
FIG. 5 is a perspective view of a housing of FIG. 4 viewed from the bottom;
FIG. 6 is a lateral cross-sectional view of FIG. 4;
FIG. 7 is a diagram for describing a pressurizing unit included in the chest measuring device, according to an embodiment;
FIG. 8 is a diagram illustrating a case when the first sensor unit included in the chest measuring device changes from a descending state to an ascending state, according to an embodiment;
FIG. 9 is a block diagram of a control unit of the chest measuring device of FIG. 3;
FIG. 10 is a block diagram of a vibration determiner of FIG. 9;
FIG. 11 is a perspective view of a charging device for charging the chest measuring device, according to an embodiment;
FIG. 12 is a diagram of a configuration of a spine correction system to which the chest measuring device of FIG. 3 is applied;
FIG. 13 is a block diagram of a personal terminal of FIG. 12;
FIG. 14 is a block diagram for describing a spine correction application of FIG. 12;
FIG. 15 is a diagram of a configuration of a scoliosis correction system according to an embodiment;
FIG. 16 is a diagram for describing principles of rotational angular breathing (RAB) using the scoliosis correction system, according to an embodiment.
FIG. 17 is a perspective view of a wearable internet of things (IoT) of FIG. 15;
FIG. 18 illustrates the wearable IoT of FIG. 17 being worn on the back of a user;
FIG. 19 is a block diagram of a control unit of the wearable IoT of FIG. 17;
FIG. 20 is a block diagram of a portable terminal of FIG. 15;
FIG. 21 is a block diagram of a spine management application of FIG. 17;
FIG. 22 is a block diagram of a data analyzer of FIG. 21;
FIG. 23 is a block diagram of a vibration determiner of FIG. 21;
FIG. 24 is a diagram for describing processes of remotely diagnosing the spine of a patient by using a system for remotely diagnosing spine according to an embodiment;
FIG. 25 is a block diagram of a system for remotely diagnosing spine according to an embodiment;
FIG. 26 is a block diagram of a wearable measuring device according to an embodiment;
FIG. 27 is a diagram of the wearable measuring device being worn on a patient, according to an embodiment;
FIG. 28 is a diagram for describing processes of generating trunk movement data of a patient by using a sensor unit, according to an embodiment;
FIG. 29 is a flowchart of a method of generating diagnostic data about a patient, according to an embodiment;
FIG. 30 is a diagram for describing processes of calculating a first vector, according to an embodiment;
FIG. 31 is a flowchart of a method of controlling a wearable measuring device to stimulate a patient, according to an embodiment;
FIG. 32 is a flowchart of a method of generating diagnostic data about a patient, according to another embodiment;
FIG. 33 is a diagram for describing processes of calculating a second vector, according to an embodiment;
FIG. 34 is a flowchart of a method of controlling a wearable measuring device to stimulate a patient, according to another embodiment;
FIG. 35 is a diagram of a wearable measuring device being worn on a patient, according to an embodiment;
FIG. 36 is a flowchart of a method of generating diagnostic data about a patient, according to another embodiment;
FIG. 37 is a flowchart of a method of controlling a wearable measuring device to stimulate a patient, according to another embodiment;
FIG. 38 is a flowchart of a method of generating diagnostic data about a patient, according to another embodiment;
FIG. 39 is a flowchart of a method of controlling a wearable measuring device to stimulate a patient, according to another embodiment;
FIG. 40 is a block diagram of a patient terminal according to an embodiment;
FIG. 41 is a flowchart of a method of providing spine health state data to a patient by measuring a posture of the patient, according to an embodiment;
FIGS. 42 through 44 illustrate a patient terminal providing spine health state data to a patient by measuring a posture of the patient, according to an embodiment;
FIG. 45 illustrates spine health state instruction data stored in a memory, according to an embodiment;
FIG. 46 is a flowchart of a method, performed by a patient terminal, of controlling a wearable measuring device, according to an embodiment;
FIGS. 47 and 48 illustrate screens of a patient terminal receiving information for controlling a wearable measuring device from a patient, according to an embodiment;
FIG. 49 is a flowchart of a method of calculating a spine score of a patient, according to an embodiment;
FIG. 50 is a flowchart of a method of calculating a spine score of a patient based on lung capacity data and trunk asymmetry data of the patient, according to an embodiment;
FIG. 51 illustrates a screen in which a calculated spine score is provided to a patient, according to an embodiment;
FIGS. 52 and 53 are respectively a rear view and a front view of a patient wearing a wearable measuring device, according to an embodiment;
FIG. 54 is a flowchart of a spine diagnosing method performed by a patient terminal, according to an embodiment;
FIG. 55 is a flowchart of a spine diagnosing method performed by a patient terminal, according to another embodiment;
FIGS. 56 and 57 are respectively a rear view and a front view of a patient wearing a wearable measuring device, according to another embodiment;
FIG. 58 is a flowchart of a spine diagnosing method performed by a patient terminal, according to another embodiment; and
FIG. 59 is a flowchart of a spine diagnosing method performed by a patient terminal, according to another embodiment.

### BEST MODE

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

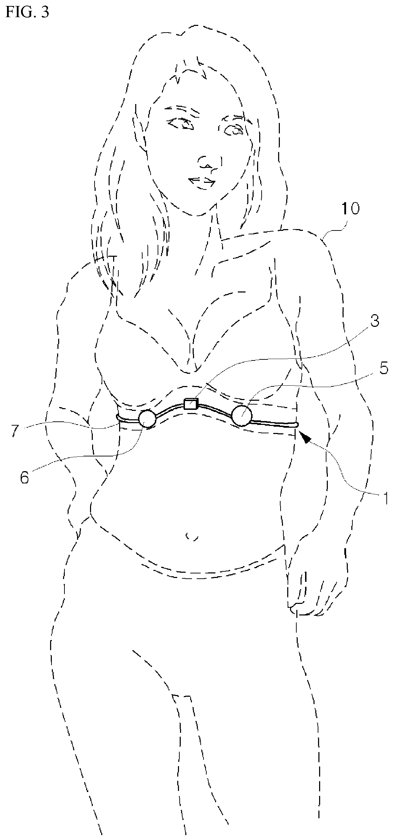

Hereinafter, a wearable chest measuring device 1 according to an embodiment will be described with reference to FIGS. 3 through 14.

The wearable chest measuring device 1 according to an embodiment measures displacement values of left and right chests of a body of a subject 10 and then determines normality of spinal alignment of the left and right chests by analyzing the measured displacement values, and detects vibration depth and intensity according to the displacement values of one of the left and right chests, which is determined to be abnormal, and then vibrates a vibrator based on the vibration depth and intensity to activate inactivated muscles and induce symmetric respiration of the left and right chests and abdominal respiration, such that the spine is aligned upright and at the same time, the abnormally aligned spine is corrected through the symmetric respiration and the abdominal respiration and the activation of the muscles.

In other words, the wearable chest measuring device 1 is an apparatus for providing not only a function of measuring the spinal alignment of the subject 10 by using sensors, but also a function of correcting the abnormally aligned spine by stimulating the inactivated muscles of the subject 10 using the Schroth theory widely-known as a scoliosis treatment.

As shown in FIG. 3, the wearable chest measuring device 1 includes first and second sensor units 5 and 6 measuring the displacement values of the left and right chests, a control unit 3 controlling and managing the first and second sensor units 5 and 6, and a detaching unit 7 combined to the first and second sensor units 5 and 6 and attaching or detaching the first and second sensor units 5 and 6 to or from the body of the subject 10.

The detaching unit 6 has a strap form having elasticity, and is combined to and supports the control unit 3 and the first and second sensor units 5 and 6. Here, the first and second sensor units 5 and 6 are combined to the detaching unit 7 in a manner that the first and second sensor units 5 and 6 are spaced apart from each other, and the control unit 3 is combined to the detaching unit 7 between the first and second sensor units 5 and 6. Accordingly, when the detaching unit 7 is attached to the body of the subject 10, the first and second sensor units 5 and 6 are disposed at locations respectively corresponding to the left and right chests, and the control unit 3 is disposed at a location adjacent to the center of the chest.

Also, the detaching unit 7 may have the strap form, wherein Velcro (not shown) for mutual detachment may be formed at two ends.

Here, in FIG. 3, the detaching unit 7 simply has the strap form, but alternatively, the detaching unit 7 may be configured in any one of well-known various forms and methods, such as a brassiere, so as to be attached or detached to or from the body.

The first and second sensor units 5 and 6 are combined to the detaching unit 7 while being spaced apart from each other such as to be disposed respectively at the left and right chests of the body of the subject 10 when attached to the body of the subject 10. Accordingly, the first and second sensor units 5 and 6 detect the displacement values of the left and right chests according to respiration of the subject 10.

In detail, the first and second sensor units 5 and 6 are attached between the seventh and ninth ribs of the left and right chests of the subject 10.

Also, the first and second sensor units 5 and 6 input the detected displacement values to the control unit 3.

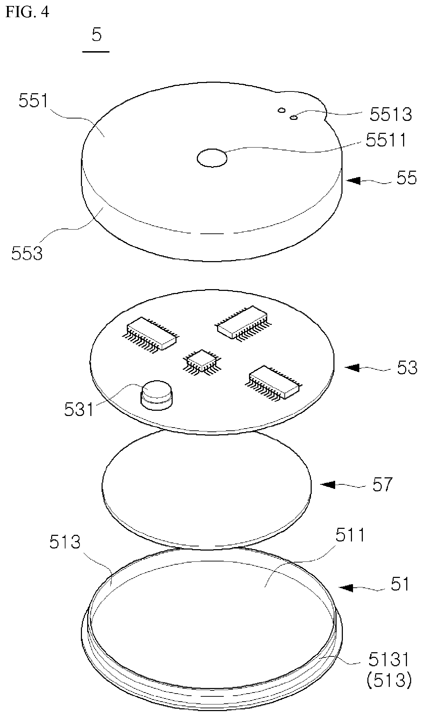

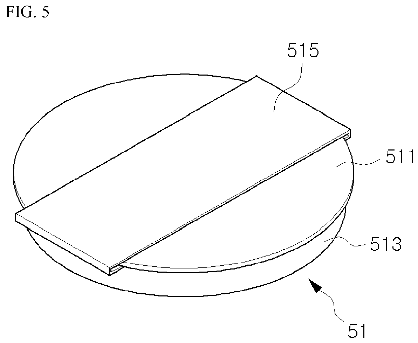

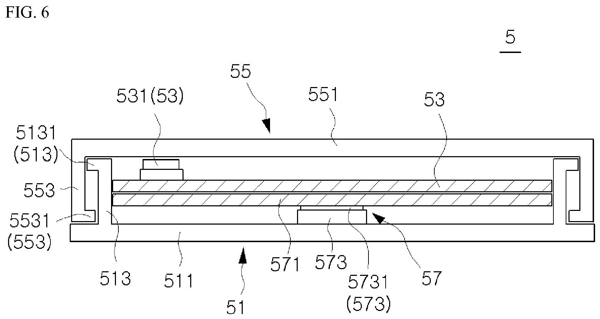

FIG. 4 is an exploded perspective view of the first sensor unit 5 of FIG. 3, FIG. 5 is a perspective view of a housing 51 of FIG. 4 viewed from the bottom, and FIG. 6 is a lateral cross-sectional view of FIG. 4.

As shown in FIGS. 4 through 6, the first sensor unit 5 includes the housing 51 having a space therein as the top is opened, a printed circuit board (PCB) 53 provided inside the housing 51, a case 55 combined to the housing 51 to seal a top opening ofthe housing 51, and a pressurizing unit 57 provided between the housing 51 and the PCB 53.

Here, the first sensor unit 5 is attached to the body of the subject 10 such that the case 55 contacts a chest.

The housing 51 has a disk shape with an opened top, and in detail, includes a bottom plate 511 having a disk shape and a side plate 513 perpendicularly connected to the bottom plate 511 at a location spaced apart inward from an outer circumference of the bottom plate 511.

Also, a protruding portion 5131 having an outer diameter expanding in a stepped manner is formed on an outer surface of the side plate 513 at a location spaced upward from the bottom portion of the side plate 513, wherein the protruding portion 5131 extends along an arc of the side plate 513.

Also, a clip 515 is provided on an outer surface of the bottom plate 511 having the disk shape, wherein the clip 515 is configured to be attached to or detached from the detaching unit 7 such that the first sensor unit 5 is attached to or detached from the detaching unit 7 via manipulation of the clip 515.

The PCB 53 includes an electric circuit and electric devices performing certain functions and operations of the first sensor unit 5, a power supply unit supplying driving power to the electric circuit and electronic devices, and a power storage unit charging or discharging charging power.

Also, the PCB 53 is configured to ascend or descend inside the housing 51 along the pressurizing unit 57 by being bolted to the pressurizing unit 57.

Also, a vibrator 531 is provided at one surface of the PCB 53, which faces the case 55, during assembly. Here, the vibrator 531 generates vibrating motion according to control of the control unit 3 to perform a function of dynamically correcting the abnormally aligned spine by simulating inactivated muscles of a chest.

Also, the vibrator 531 is provided such that an end of the vibrator 531 contacts the case 55. Accordingly, vibrating motion of the vibrator 531 is transmitted from the vibrator 531 to muscles of the chest through the case 55 and the body contacting the case 55, and thus the abnormally aligned spine is corrected as inactivated muscles of the chest is activated by the vibrating motion.

In other words, the vibrator 531 generates the vibrating motion when respiration is performed while the muscles of the chest are inactivated due to the abnormally aligned spine or an incorrect posture of the subject 10 and thus the chest does not smoothly expand and contract, thereby providing 1) a function of enabling the subject 10 to recognize that his/her posture and respiration are not normal through the vibrating motion and 2) a function of correcting the abnormally aligned spine by stimulating and activating the inactivated muscles of the chest through the vibrating motion to induce normal respiration

Also, although not illustrated in FIG. 4, the PCB 53 may include, on one surface, a gyrosensor, an acceleration sensor, and a geomagnetic sensor.

The acceleration sensor is a triaxial acceleration sensor and detects a velocity displacement vector per unit time. In other words, the acceleration sensor detects dynamic force, such as acceleration, vibration, and an impact.

Also, the acceleration sensor detects an acceleration vector with respect to 3 axes generated along X-, Y-, and Z-axes perpendicular to each other, based on gravitational acceleration.

Accordingly, the acceleration sensor detects the acceleration vector of the X-axis, the acceleration vector of the Y-axis, and the acceleration vector of the Z-axis.

The gyrosensor is a triaxial gyrosensor and detects rotational inertia by detecting an angular speed.

Also, the gyrosensor may detect an angular speed vector value, in which an object rotates in unit time in each direction of the X-, Y-, and Z-axes. Here, rotation with respect to the X-axis is referred to as roll, rotation with respect to the Y-axis is referred to as pitch, and rotation with respect to the Z-axis is referred to as yaw.

Accordingly, the gyrosensor detects an angular speed vector of the X-axis, an angular speed vector of the Y-axis, and an angular speed vector of the Z-axis.

The geomagnetic sensor detects geomagnetism of the left and right chests.

Since the acceleration sensor, the gyrosensor, and the geomagnetic sensor are widely used in various detecting apparatuses, details thereof are not provided herein.

However, in the present disclosure, the first sensor unit 51 contacts and is attached to the body corresponding to the chest so as to detect movement of the chest of the subject 10 according to respiration, with respect to the three axes.

Referring back to FIGS. 4 and 5, the case 55 includes a disk portion 551 having a disk shape and a side portion 553 perpendicularly connected to an outer circumference of the disk portion 551. Here, the side portion 553 has an outer diameter larger than that of the side plate 513 of the housing 51 such that the side plate 513 of the housing 51 is inserted into the side portion 553 during assembly.

Also, the side portion 553 includes an engaging portion 5531 protruding inward on an inner circumference adjacent to the bottom portion of the side portion 553 and extending along the arc. Here, the engaging portion 5531 has an inner diameter that is the same as the outer diameter of the side plate 513 of the housing 51 such that the inner end of the engaging portion 5531 is engaged with the outer circumference of the side plate 513 of the housing during assembly.

The side plate 513 of the housing 51 is inserted into the side portion 553 of the case 55 configured as such, during assembly. Here, the engaging portion 5531 of the side portion 553 of the case 55 has the inner diameter that is the same as the outer diameter of the side plate 513 of the housing 51, and thus the engaging portion 5531 of the side portion 553 is configured to be blocked by the protruding portion 5131 of the housing 51 but to be assembled or disassembled via forced fit.

Also, when assembly of the housing 51 and the case 55 is completed, the outer circumference of the side plate 513 of the housing 51 and the inner circumference of the side portion 553 of the case 55 are spaced apart from each other such that the case 55 is combined to the housing 51 while being slidable in a vertical direction.

Also, the case 55 is attached to the body of the subject 10 such that the front surface of the case 55 contacts the body, in detail, the body corresponding to the chest of the subject 10.

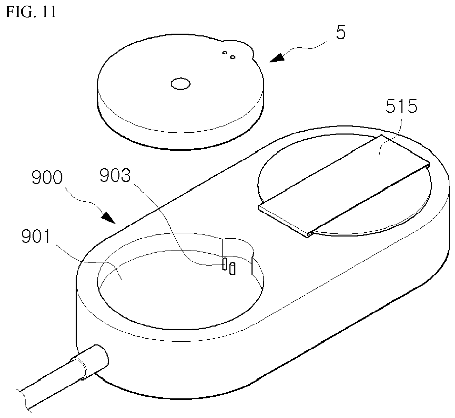

Also, a power supply button 5511 for turning on or off power and a terminal 5513 for receiving charging power from a charging device 900 of FIG. 11 described below are provided on a front surface of the disk portion 551 of the case 55.

The bottom of the pressurizing unit 57 is combined to the bottom plate 511 of the housing 51, and the top of the pressurizing unit 57 is combined to the PCB 53.

Also, the pressurizing unit 57 is configured to raise or lower the PCB 53 according to control of the control unit 3.

In other words, when the PCB 53 ascends by the pressurizing unit 57, the vibrator 531 provided in the PCB 53 also ascends, and accordingly, the case 55 pressurized upward by the vibrator 531 also ascends, and thus the subject 10 receives stronger vibrating motion than when the case 55 does not ascend.

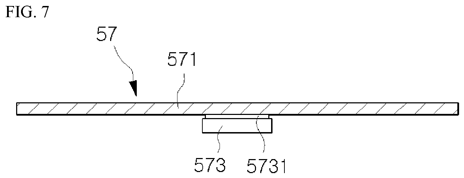

Here, any one of various well-known technologies and configurations for raising or lowering a particular element may be applied to the pressurizing unit 57, as will be described with reference to FIG. 7.

FIG. 7 is a diagram for describing the pressurizing unit 57 included in the chest measuring device 1, according to an embodiment.

As shown in FIG. 7, the pressurizing unit 57 includes a cylinder 573 provided at the bottom plate 511 of the housing 51, and a fixed plate 571 having a board shape, having a top surface on which the PCB 53 is mounted, and having a bottom surface to which a rod 5731 of the cylinder 573 is combined.

Also, as widely known, the cylinder 573 is configured such that the rod 5731 may linearly reciprocate in an up-and-down direction. Here, the fixed plate 571 is combined to an end of the rod 5731, and the PCB 53 is provided on the top surface of the fixed plate 571 such that ascending and descending movement of the rod 5731 is transmitted from the fixed plate 571 to the disk portion 551 of the case 55 through the PCB 53 and the vibrator 531, and thus the case 55 ascends or descend based on the movement of the rod 5731.

Since the second sensor unit 6 has the same configuration as the first sensor unit 5, details thereof are not provided again.

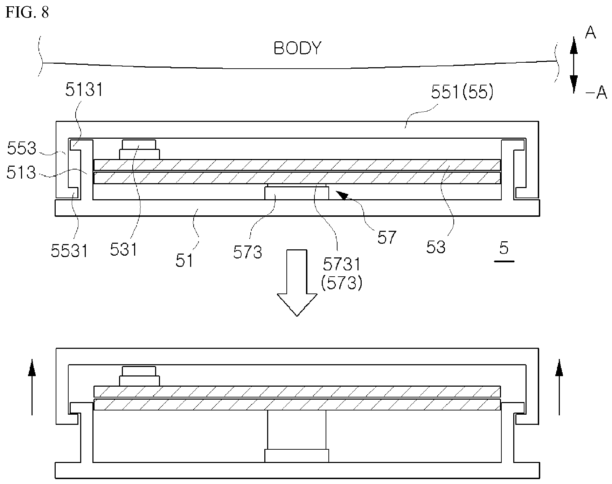

FIG. 8 is a diagram illustrating a case when the first sensor unit 5 changes from a descending state to an ascending state, according to an embodiment.

As shown in the first sensor unit 5 of FIG. 8, the case 55 is disposed in a direction -A away from the body of the subject 10 when the rod 5731 of the cylinder 573 of the pressurizing unit 57 is retracted.

At this time, when the rod 5731 of the cylinder 573 of the pressurizing unit 57 is drawn out, the PCB 53 and the vibrator 531 move together in a direction A towards the body according to the linear movement of the rod 5731, and the case 55 is moved in the direction A towards the body by the vibrator 531 contacting the disk portion 551.

Here, since the inner circumference of the side portion 553 is spaced apart from the side plate 513 of the housing 51, the case 55 moves in the direction A towards the body, and when such movement continues, the engaging portion 5531 of the side portion 553 of the case 55 is caught by the protruding portion 5131 of the side plate 513 of the housing 51 and is restricted from moving.

Also, when the vibrator 531 vibrates while the first sensor unit 5 is ascended (moved in the direction A towards the body), the subject 10 receives larger stimulation at the same vibration intensity compared to when the pressurizing unit 57 is descended.

Also, unlike the first sensor unit 5 shown in FIG. 8, when the rod 5731 of the cylinder 573 of the pressurizing unit 57 is drawn out, the case 55 is disposed in the direction A towards the body of the subject 10.

At this time, when the rod 5731 of the cylinder 573 of the pressurizing unit 57 is retracted, force supporting the case 55 is lost and accordingly, the case 55 receives force from the body according to elasticity of the detaching unit 7 described above.

Here, since the inner circumference of the side portion 553 is spaced apart from the side plate 513 of the housing 51, when the case 55 is pressurized by the body, the case 55 moves in the direction -A away from the body.

Also, when the vibrator 531 vibrates while the first sensor unit 5 is descended (moved in the direction -A away from the body), the subject 10 receives smaller stimulation at the same vibration intensity compared to when the pressurizing unit 57 is ascended.

In the wearable chest measuring device 1 according to an embodiment, the first and second sensor units 5 and 6 are respectively provided at the left and right chests of the body of the subject 10, and the gyrosensor, the acceleration sensor, and the geometric sensor of each of the first and second sensor units 5 and 6 detect and input, to the control unit 3, speed vectors with respect to three axes of the left and right chests.

Here, the control unit 3 analyzes and processes data input from the first and second sensor units 5 and 6 to determine whether the vibrator 531 of each of the first and second sensor units 5 and 6 is driven and determine elevation displacement of the pressurizing unit 57, and outputs determined control values to the first and second sensor units 5 and 6.

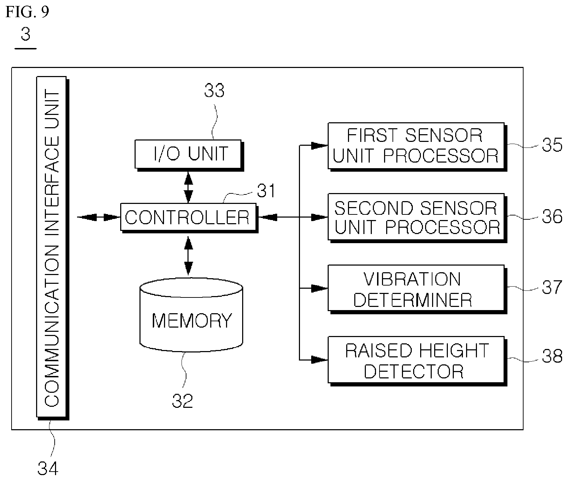

FIG. 9 is a block diagram of the control unit 3 of the wearable chest measuring device 1 of FIG. 3.

As shown in FIG. 9, the control unit 3 of the wearable chest measuring device 1 includes a controller 31, a memory 32, an input/output (I/O) unit 33, a communication interface unit 34, a first sensor unit processor 35, a second sensor unit processor 36, a vibration determiner 37, and a raised height detector 38.

Here, for convenience of description, it is described that the control unit 3 of the wearable chest measuring device 1 performs an operation of analyzing and processing detected data measured by the first and second sensor units 5 and 6, an operation of determining vibration, and an operation of determining a raised height of the pressurizing unit 57, but it would be obvious to one of ordinary skill in the art that the control unit 3 may be simply configured to externally transmit the detected data received from the first and second sensor units 5 and 6, and an external controller and a local server perform the above operations.

The controller 31 is an operating system (OS) of the control unit 3, and controls and manages the memory 32, the I/O unit 33, the communication interface unit 34, the first sensor unit processor 35, the second sensor unit processor 36, the vibration determiner 37, and the raised height detector 38.

Also, upon receiving data from the first and second sensor units 5 and 6 through the I/O unit 33, the controller 31 inputs the data received from the first sensor unit 5 to the first sensor unit processor 35 and inputs the data received from the second sensor unit 6 to the second sensor unit processor 36.

Also, the controller 31 inputs, to the vibration determiner 37, 3-axis acceleration values, angular speed values, and geomagnetic information of the first sensor unit 5 detected by the first sensor unit processor 35, and inputs, to the vibration determiner 37, 3-axis acceleration values, angular speed values, and geomagnetic information of the second sensor unit 6 detected by the second sensor unit processor 36.

Also, when the vibration determiner 37 determines that the first or second sensor unit 5 or 6 needs to vibrate, the controller 31 inputs, to the raised height detector 38, the 3-axis acceleration values, the angular speed values, and the geomagnetic information of the first and second sensor units 5 and 6 detected by the first and second sensor unit processors 35 and 36.

Also, when the raised height detector 38 determines a raised height of the pressurizing unit 57, the controller 31 controls the I/O unit 33 to output control data to the one of the first and second sensor units 5 and 6, which is determined that vibration is needed. Here, the control data contains the raised height determined by the raised height detector 38 and a signal for vibrating the vibrator 531.

The memory 32 stores identification (ID) information of the first and second sensor units 5 and 6.

Also, the memory 32 stores the 3-axis acceleration values, the angular speed values, and the geomagnetic information detected by the first sensor unit processor 35.

Also, the memory 32 stores the 3-axis acceleration values, the angular speed values, and the geomagnetic information detected by the second sensor unit processor 36.

Also, the memory 32 stores a pre-set volume detection algorithm. Here, the pre-set volume detection algorithm is an algorithm for detecting volume of a chest by using 3-axis acceleration values, angular speed values, and geomagnetic information.

Also, the memory 32 stores a reference table. Here, the reference table is defined by data to which a raised height of the pressurizing unit 57 is matched according to exhalation volume information of a chest.

The I/O unit 33 receives or transmits data from or to the first and second sensor units 5 and 6.

The communication interface unit 34 accesses a communication network to communicate with an external terminal and a server.

The first sensor unit processor 35 analyzes the detected data of the first sensor unit 5 received through the I/O unit 33 to detect angular speed values of the X-, Y-, and Z-axes measured by the gyrosensor of the first sensor unit 5, acceleration values of the X-, Y-, and Z-axes measured by the acceleration sensor of the first sensor unit 5, and geomagnetic values measured by the geomagnetic sensor of the first sensor unit 5.

The second sensor unit processor 36 analyzes the detected data of the second sensor unit 6 received through the I/O unit 33 to detect angular speed values of the X-, Y-, and Z-axes measured by the gyrosensor of the second sensor unit 6, acceleration values of the X-, Y-, and Z-axes measured by the acceleration sensor of the second sensor unit 6, and geomagnetic values measured by the geomagnetic sensor of the second sensor unit 6.

Also, data detected by the first and second sensor unit processors 35 and 36 is input to the vibration determiner 37 according to control of the controller 31.

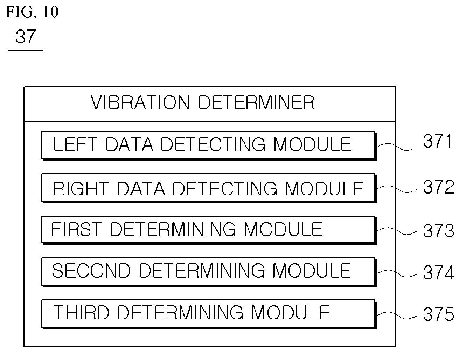

FIG. 10 is a block diagram of the vibration determiner 37 of FIG. 9.

As shown in FIG. 10, the vibration determiner 37 includes a left data detecting module 371, a right data detecting module 372, a first determining module 373, a second determining module 374, and a third determining module 375.

The left data detecting module 371 analyzes data detected by the first sensor unit processor 35 by using a pre-set volume detection algorithm to detect 1) inhalation volume information V1 of a left chest, 2) exhalation volume information V2 of the left chest, and 3) volume displacement value ΔV of the left chest during inhalation and exhalation.

Here, the pre-set volume detection algorithm is an algorithm for detecting volume of a chest by using 3-axis acceleration values, angular speed values, and geomagnetic information.

Hereinafter, data detected by the left data detecting module 371 will be referred to as left data.

The right data detecting module 372 analyzes data detected by the second sensor unit processor 36 by using the pre-set volume detection algorithm to detect 1) inhalation volume information V1' of a right chest, 2) exhalation volume information V2' of the right chest, and 3) volume displacement value ΔV' of the right chest during inhalation and exhalation.

Hereinafter, data detected by the right data detecting module 372 will be referred to as right data.

The first determining module 373 compares the volume displacement value ΔV of the left chest detected by the left data detecting module 371 with a first threshold value TH1. Here, the first threshold value TH1 is defined as a smallest value of a volume displacement value during inhalation and exhalation of a chest of which respiration is determined to be normal.

In other words, when the volume displacement value ΔV of the left chest is smaller than the first threshold value TH1, the first determining module 373 determines that the respiration of the left chest is not normal, and determines that inactivated muscles of the left chest needs to be stimulated via vibration of the first sensor unit 5.

The second determining module 374 compares the volume displacement value ΔV' of the right chest detected by the right data detecting module 372 with the first threshold value TH1 described above.

In other words, when the volume displacement value ΔV' of the right chest is smaller than the first threshold value TH1, the second determining module 374 determines that the respiration of the right chest is not normal, and determines that inactivated muscles of the right chest needs to be stimulated via vibration of the second sensor unit 6.

The third determining module 375 calculates a difference D between the inhalation volume information V1 of the left chest detected by the left data detecting module 371 and the inhalation volume information V1' of the right chest detected by the right data detecting module 372.

Also, the third determining module 375 compares the calculated difference D with a second threshold value TH2.

Here, the second threshold value TH2 is defined as a largest difference between the inhalation volume information V1 of the left chest and the inhalation volume information V1' of the right chests during normal respiration.

In other words, when the difference D is equal to or greater than the second threshold value TH2, the third determining module 375 determines that respiration of the left or right chest is not normal, in detail, determines that respiration of a chest having smaller inhalation volume information from among the left and right chests is not normal, and determines that inactivated muscles of the chest needs to be stimulated through vibration of a sensor unit corresponding to the chest of which respiration is not normal.

The raised height detector 38 is driven when the vibration determiner 37 of FIG. 10 described above determines that at least one of the first and second sensor units 5 and 6 needs to vibrate.

Also, the raised height detector 38 search the reference table stored in the memory 32 to detect a raised height H corresponding to the exhalation volume information V2 or V2' corresponding to a sensor unit determined that vibration is needed.

Here, the reference table is defined by data to which a raised height of the pressurizing unit 57 is matched according to exhalation volume information of a chest.

Also, when the raised height detector 38 detects the raised height H, the controller 31 outputs control data including information about the raised height H and the signal for vibrating the vibrator 531 to the corresponding sensor unit through the I/O unit 33. Upon receiving the control data from the controller 31, the corresponding sensor unit controls a raised height of the pressurizing unit 57 based on the raised height H and then generates vibrating motion of the vibrator 531 such that 1) the subject 10 recognizes, through vibration of the vibrator 531, that his/her respiration or posture is not normal and 2) the chest receives the vibrating motion having intensity suitable to a state of the chest and stimulating inactivated muscles of the chest, thereby aligning the abnormally aligned spine upright.

FIG. 11 is a perspective view of the charging device 900 for charging the wearable chest measuring device 1, according to an embodiment.

As shown in FIG. 11, the first and second sensor units 5 and 6 of the wearable chest measuring device 1 receive power from the charging device 900 when mounted on the charging device 900, and the received power is charged in a power storage unit (not shown).

The charging device 900 includes accommodating grooves 901 where the first and second sensor units 5 and 6 are respectively accommodated, and charging pins 903 for supplying charging power by being inserted into the terminals 5513 of the first and second units 5 and 6 are provided on the bottom surface of the accommodating grooves 901.

For convenience of description, it is described that the charging device 900 has a shape and configuration shown in FIG. 11, but the shape and configuration of the charging device 900 are not limited thereto, and may vary as long as power is supplied to a sensor unit.

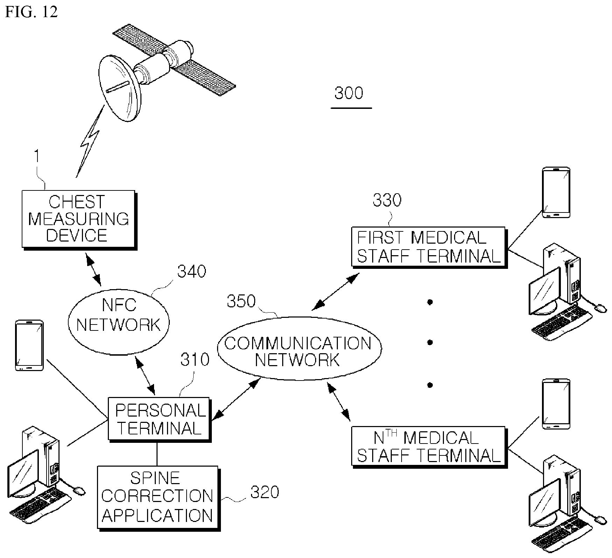

FIG. 12 is a diagram of a configuration of a spine correction system 300 to which the wearable chest measuring device 1 of FIG. 3 is applied.

The spine correction system 300 of FIG. 12 includes: the wearable chest measuring device 1 according to an embodiment described above with reference to FIGS. 3 through 10; a personal terminal 310 where a spine correction application 320 is installed, wherein the spine correction application 320 is an application program that receives the left data and the right data from the wearable chest measuring device 1 and provides response data by processing and analyzing the left data and the right data according to a user's request; medical staff terminals 330 that are terminals owned by medical staffs and perform remote treatment by connecting to the personal terminal 310 according to a request of the spine correction application 320; a short-range wireless communication (SWC) network 340 supporting data communication between the wearable chest measuring device 1 and the personal terminal 310; and a communication network 350 providing data movement paths between the personal terminal 310 and the medical staff terminals 330.

The communication network 350 provides data movement paths between the personal terminal 310 and the medical staff terminals 330, and in detail, may include a wired/wireless network such as a wide area network (WAN), or a mobile communication network.

The SWC network 340 provides a data movement path between the wearable chest measuring device 1 and the personal terminal 310, and in detail, may include Wi-Fi, Bluetooth, Zigbee, or a wired cable.

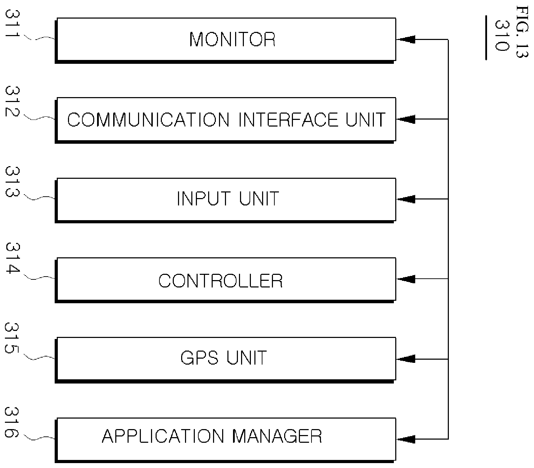

FIG. 13 is a block diagram of the personal terminal 310 of FIG. 12.

The personal terminal 310 is a terminal owned by the subject 10, and in detail, may be a desktop computer, a laptop computer, or a smart phone. For convenience of description, it is described that the personal terminal 310 is a smart phone.

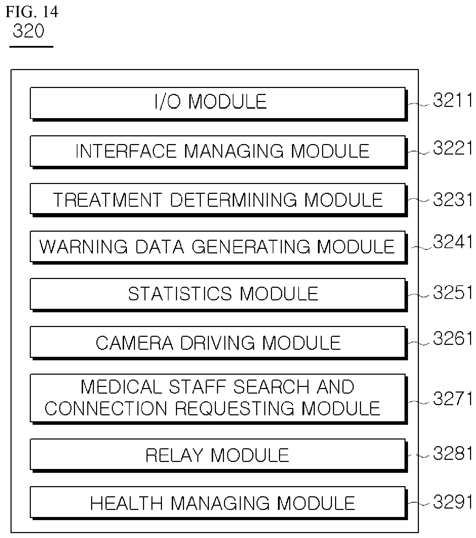

Also, as shown in FIG. 13, the personal terminal 310 includes: a monitor 311 commonly included in a smart phone and displaying content; a communication interface unit 312 supporting data communication with the medical staff terminal 330 or the wearable chest measuring device 1 by accessing the communication network 350 or the SWC network 340; an input unit 313 receiving a character or a symbol from a user; a controller 314 operating as an OS of the personal terminal 310 to control a control target; a global positioning system (GPS) unit 315 calculating a location of the personal terminal 310 by using a signal received from a GPS satellite; and an application manger 316 managing and controlling the spine correction application 320 of FIG. 14 described below.

FIG. 14 is a block diagram for describing the spine correction application 320 of FIG. 12.

The spine correction application 320 is an application program installed in the personal terminal 310.

Also, the spine correction application 320 includes: an I/O module 3211 receiving or transmitting data from or to the personal terminal 310; an interface managing module 3221 managing a pre-set graphic user interface (GUI); a treatment determining module 3231 determining whether to treat the subject 10 by analyzing the left data and the right data received from the wearable chest measuring device 1 through the personal terminal 310 and the I/O module 3211; a warning data generating module 3241 displaying warning data on the personal terminal 310 when the treatment determining module 3231 determines that the subject 10 needs to be treated; a statistics module 3251 generating statistic data per pre-set category by using the left data and the right data received from the wearable chest measuring device 1 during a pre-set cycle T; a camera driving module 3261 driving a camera of the personal terminal 310 by being driven when the warning data generating module 3241 generates the warning data; a medical staff search and connection requesting module 3271 requesting the medical staff terminal 330 located within a threshold range for connection by comparing location information of the personal terminal 310 and pre-set location information of the medical staff terminal 330, by being driven when the warning data generating module 3241 generates the warning data; a relay module 3281 relaying a video call between the medical staff terminal 330 and the personal terminal 310 connected to each other by the medical staff search and connection requesting module 3271; and a health managing module 3291 managing schedules, such as dates related to chest measurement of the subject 10 and treatment dates with a medical staff.

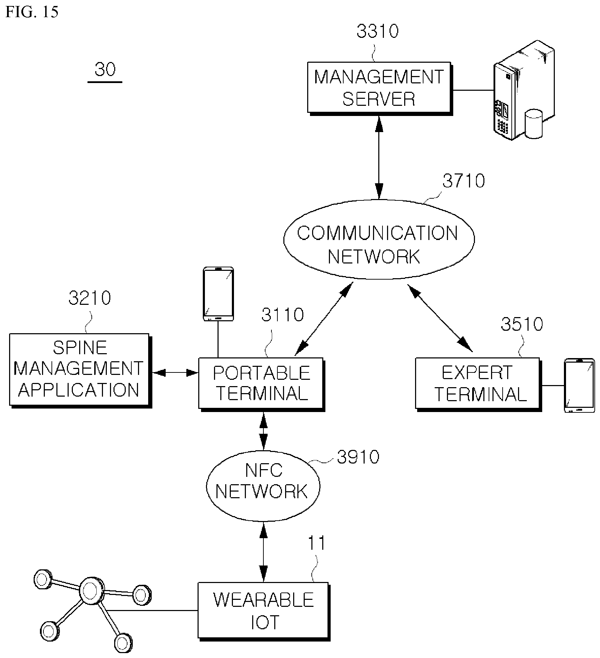

Hereinafter, a scoliosis correction system 30 according to an embodiment will be described with reference to FIGS. 15 through 23.

FIG. 15 is a diagram of a configuration of the scoliosis correction system 3- according to an embodiment.

The scoliosis correction system 30 according to an embodiment is used to, by using a wearable IoT 11 attached to or detached from a body of the subject 10, 1) correct the abnormally aligned spine by vibrating (stimulating) the inactivated muscles and 2) effectively correct scoliosis without the subject 10 having to visit a professional organization by providing information about rotational angular breathing (RAB) and a training posture corresponding to a respiration pattern and a spinal alignment state of the subject 10, based on sensing values of muscles corresponding to left and right ribs and left and right transverse processes of lumbar vertebrae according to respiration of the subject 10.

Also, as shown in FIG. 15, the scoliosis correction system 30 includes the wearable IoT 11, a management server 3310, a portable terminal 3110, a spine management application 3210, an expert terminal 3510, a communication network 3710, and an SWC network 3910.

The communication network 3710 provides data movement paths between the portable terminal 3110, the management server 3310, and the expert terminal 3510, and in detail, may include a wired/wireless network, such as WAN, a mobile communication network, or long-term evolution (LTE).

The SWC network 3910 supports data communication of the wearable IoT 11 and the portable terminal 3110 connected to the SWC network 3910, and in detail, may include Wi-Fi, Bluetooth, Zigbee, or a wired cable.

The portable terminal 3110 is a digital terminal owned by a user (the subject 10) who is to receive a spine correction service.

Also, the portable terminal 3110 supports connection with the communication network 3710 and the SWC network 3910, and in detail, may be a desktop computer, a laptop computer, or a smart phone, and for example, may be a smart phone.

Also, the spine management application 3210 is stored in the portable terminal 3110.

Also, upon receiving a sensing value from the wearable IoT 11 through the SWC network 3910, the portable terminal 3110 transmits the received sensing value to the spine management application 3210. Here, the spine management application 3210 1) detects motion vectors of muscles of the subject 10 (muscles corresponding to the left and right ribs and left and right transverse processes of lumbar vertebrae) by analyzing sensing values input by using a pre-set analysis algorithm, 2) detects activity of each muscle by analyzing the detected motion vectors by using a pre-set activity measuring algorithm and determines whether to vibrate each muscles based on the detected activity, and 3) when there is a muscle that needs to be vibrated, detects vibration information (a vibration depth and intensity) corresponding to a motion vector of the corresponding muscle.

Also, the portable terminal 3110 transmits information about the motion vector of each muscle to the management server 3310 through the communication network 3710, according to control of the spine management application 3210. Here, the management server 3310 detects the respiration pattern and the spinal alignment state of the subject 10 by analyzing the received motion vector of each muscle by using a pre-set respiration pattern and spinal alignment state detection algorithm, and detects and provides, to the subject 10, optimum RAB and training posture corresponding to the detected respiration pattern and spinal alignment state.

Also, the portable terminal 3110 transmits the detected vibration information to the wearable IoT 11 through the SWC network 3910 according to control of the spine management application 3210. Here, the wearable IoT 11 is configured to vibrate a sensor unit of the corresponding muscle based on the vibration information received from the portable terminal 3110, such that the inactivated muscles are stimulated by vibration and the abnormally aligned spine is corrected.

Also, upon receiving information about the optimum RAB and training posture from the management server 3310, the portable terminal 3110 inputs the information about the optimum RAB and training posture to the spine management application 3210. Here, upon detecting the information about the optimum RAB and training posture corresponding to the respiration pattern and the spinal alignment state of the subject 10, the management server 3310 generates and transmits, to the portable terminal 3110, a correction information interface, i.e., a GUI displaying the optimum RAB and training posture.

In other words, the subject 10 may not only automatically correct the abnormally aligned spine as the inactivated muscles are vibrated by the wearable IoT 11, but also correct symptoms of scoliosis, such as Rippental, Rippenberg, Lendental, and Lendenberg, via steady RAB and training by receiving the information about the optimum RAB and training posture corresponding to his/her respiration pattern and the spinal alignment state.

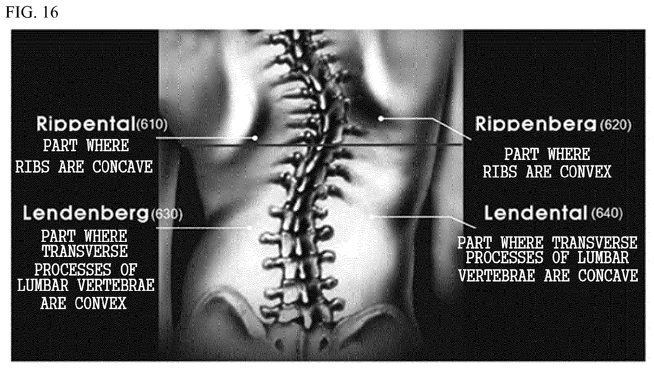

FIG. 16 is a diagram for describing principles of RAB using the scoliosis correction system 30, according to an embodiment.

Referring to RAB shown in FIG. 16, RAB is a basic concept of the Schroth theory, and is defined as a respiration pattern in which, by pushing respiration behind the back that is pressed as the body is narrowed during respiration and contracting the protruding back, respiration is balanced such that a contracted body part is expanded while a relaxed body part is not expanded.

Here, Rippental 610 is a part where ribs are concave, Rippenberg 620 is a part where ribs are convex, Lendental 640 is a part where transverse processes of lumbar vertebrae are concave, and Lendenberg 630 is a part where transverse processes of lumbar vertebrae are convex.

Referring back to FIG. 15, the wearable IoT 11 is configured to be attachable to or detachable from the back of the subject 10.

Also, the wearable IoT 11 includes four sensor units. Here, each sensor unit includes a gyrosensor, an acceleration sensor, and a geomagnetic sensor, and is configured to contact the back of the subject 10, in detail, each of muscles corresponding to the left and right ribs and the left and right transverse processes of lumbar vertebrae to detect sensing values (acceleration, an angular speed, and geomagnetism) of the each muscles.

Also, the wearable IoT 11 transmits the sensing values of the each muscle detected by the sensor units to the portable terminal 3110 through the SWC network 3910, and the portable terminal 3110 transmits the sensing values received from the wearable IoT 11 to the spine management application 3210.

Also, the sensor units of the wearable IoT 11 include a vibrator. Here, the vibrator is configured such that a protruding length and vibration intensity are adjustable. Accordingly, upon receiving the vibration information (the vibration depth and intensity) through the portable terminal 3110 according to control of the spine management application 3210, the wearable IoT 11 vibrates the vibrator of the sensor unit according to the received vibration depth and intensity such that the inactivated muscles are suitably stimulated and activated based on an inactivation degree of the subject 10.

The spine management application 3210 is an application program installed in the portable terminal 3110.

Also, upon receiving the sensing values of the each muscle (each of the muscles corresponding to the left and right ribs and the left and right transverse processes of lumbar vertebrae) from the wearable IoT 11 through the portable terminal 3110, the spine management application 3210 detects the motion vector of each muscle by analyzing the received sensing values by using the pre-set analysis algorithm, and determines a muscle that needs to be vibrated by using activity of each muscle detected by analyzing the detected motion vector of each muscle by using the pre-set activity measuring algorithm.

Also, when it is determined that there is a muscle that needs to be vibrated, the spine management application 3210 detects optimum vibration intensity and height of the vibrator by analyzing the motion vector of each muscle by using a pre-set vibration intensity and height detection algorithm.

Also, the spine management application 3210 transmits information about the motion vector of each muscle to the management server 3310 by controlling the portable terminal 3110.

Also, the spine management application 3210 transmits control data including information about the detected optimum vibration intensity and height to the wearable IoT 11 through the SWC network 3910 by controlling the portable terminal 3110.

Also, upon receiving, from the management server 3310, the correction information interface displaying the optimum RAB and training posture, the spine management application 3210 displays the received correction information interface on a monitor of the portable terminal 3110 such that the subject 10 receives the information about the optimum RAB and training posture suitable to his/her spinal condition.

Also, upon receiving information about a nearby professional organization (medical institution or medical care center) and a nearby expert from the management server 3310, the spine management application 3210 displays the received information on the monitor of the portable terminal 3110 such that, when the subject 10 needs to be treated, the subject 10 may conveniently receive the information about the nearby professional organization and the nearby expert.

The management server 3310 is a server that manages and controls the spine management application 3210.

Also, upon receiving the information about the motion vector of each muscle from the spine management application 3210, the management server 3310 analyzes the received motion vector of each muscle by using the pre-set respiration pattern and spinal alignment state detection algorithm, and detects the respiration pattern and the spinal alignment state of the subject 10.

Here, while detecting the respiration pattern and the spinal alignment state, the management server 3310 determines whether a spinal condition of the subject 10 includes Rippental, Rippenberg, Lendental, or Lendenberg.

Also, the management server 3310 stores a pre-set reference table in which RAB and a training posture are matched per respiration pattern and spinal alignment state, and when the respiration pattern and the spinal alignment state of the subject 10 are detected, detects the optimum RAB and training posture corresponding to the detected respiration pattern and spinal alignment state by searching the reference table.

Also, when the optimum RAB and the training posture are detected, the management server 3310 generates the correction information interface displaying the information about the detected optimum RAB and training posture and transmits the correction information interface to the spine management application 3210.

Also, the management server 3310 stores information (organization names, websites of professional organizations, phone numbers, locations, information about experts in professional organizations, and websites of experts) about professional organizations, such as medical instructions and medical care centers for treating and correcting scoliosis, and when the detected respiration pattern and spinal alignment state of the subject 10 are outside a pre-set threshold range, transmits information about a professional organization and an expert near the subject 10 to the spine management application 3210.

Also, when the respiration pattern and spinal alignment state received from the portable terminal 3110 are outside the pre-set threshold range, the management server 3310 transmits the respiration pattern and spinal alignment state to the expert terminal 3510, receives detailed diagnosis information from an expert, and transmits the detailed diagnosis information to the spine management application 3210.

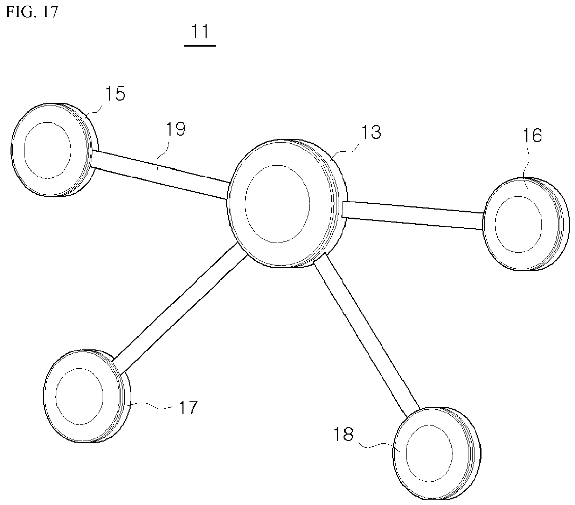

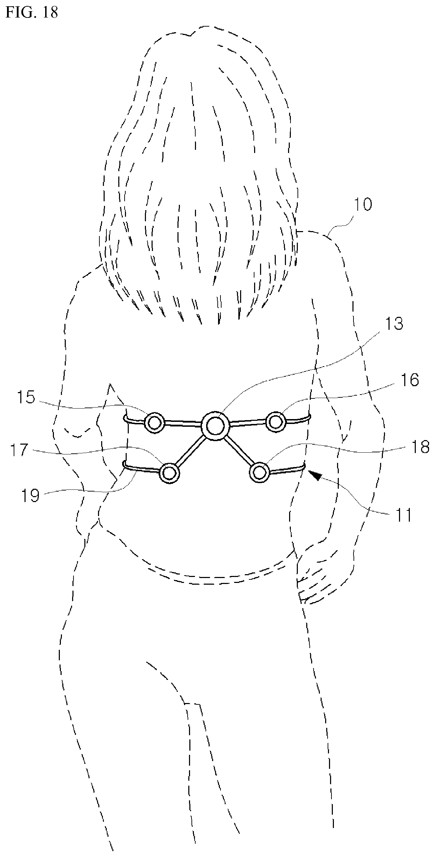

FIG. 17 is a perspective view of the wearable IoT 11 of FIG. 15, and FIG. 18 illustrates the wearable IoT 11 of FIG. 17 being worn on the back of the subject 10.

The wearable IoT 11 performs 1) a function of measuring sensor values at locations corresponding to the back of the subject 10 who is a target, in detail, the left and right ribs and the left and right transverse processes of lumbar vertebrae, by using first through fourth sensor units 15 through 18 contacting the locations, and 2) a function of correcting an abnormal spine alignment state by driving a vibrator according to a vibration depth and intensity input from the spine management application 3210 so as to stimulate the inactivated muscles.

In other words, the wearable IoT 11 is an apparatus that provides not only a function of diagnosing the respiration pattern and spinal alignment state through the sensing values of the left and right ribs and the left and right transverse processes of lumbar vertebrae of the subject 10 by using the first through fourth sensor units 15 through 18, but also a function of correcting the abnormally aligned spines by stimulating the inactivated muscles of the subject 10 by using the Schorth theory widely known as a scoliosis treatment.

As shown in FIGS. 17 and 18, the wearable IoT 11 includes: the first through fourth sensor units 15 through 18 measuring displacement values of the left and right ribs and the left and right transverse processes of lumbar vertebrae of the subject 10; a control unit 13 managing and controlling the first through fourth sensor units 15 through 18; and a detaching unit 19 combined to the control unit 13 and the first through fourth sensor units 15 through 18 and attaching or detaching the control unit 13 and the first through fourth sensor units 15 through 18 to or from the body of the subject 10.

The detaching unit 19 has a strap form having elasticity, and is combined to and supports the control unit 13 and the first through fourth sensor units 15 through 18. Here, the first through fourth sensor units 15 through 18 are combined to the detaching unit 19 while being spaced apart from each other, and the control unit 13 is provided at the center of the first through fourth sensor units 15 through 18 such that, when the detaching unit 19 is attached to the back of the subject 10, the first and second sensor units 15 and 16 are disposed at locations corresponding to the left and right ribs, the third and fourth sensor units 17 and 18 are disposed at locations corresponding to the left and right transverse processes of lumbar vertebrae, and the control unit 13 is disposed at the center.

Also, the detaching unit 19 may have a strap form while Velcro (not shown) for mutual detachment may be formed at two ends.

Here, in FIG. 18, it is described that the detaching unit 19 simply has the strap form, but alternatively, the detaching unit 19 may be configured in any one of well-known various forms and methods, such as a brassiere or a tank top.

The first and second sensor units 15 and 16 detect the displacement values of the left and right ribs according to respiration of the subject 10 by being combined to the detaching unit 19 opposite to each other and contacting the left and right ribs of the subject 10 when attached to the body of the subject 10.

The third and fourth sensor units 17 and 18 detect the displacement values of the left and right transverse processes of lumbar vertebrae according to respiration of the subject 10 by being combined to the detaching unit 19 opposite to each other below the first and second sensor units 5 and 6 and contacting the left and right transverse processes of lumbar vertebrae of the subject 10 when attached to the body of the subject 10.

Also, the first through fourth sensor units 15 through 18 inputs detected data to the control unit 13.

Meanwhile, since the first through fourth sensor units 15 through 18 included in the scoliosis correction system 30 have the same structure as the first and second sensor units 5 and 6 of included in the chest measuring device 1, details about the structures and operations of the first through fourth sensor units 15 through 18 are not provided again.

The wearable IoT 11 configured as such may detect the sensing values with respect to the left and right ribs and the left and right transverse processes of lumbar vertebrae as the first through fourth sensor units 15 through 18 respectively contact the locations corresponding to the left and right ribs and the left and right transverse vertebrae of lumbar vertebrae of the back of the subject 10 and each of the first through fourth sensor units 15 through 18 includes a gyrosensor, an acceleration sensor, and a geomagnetic sensor.

Here, the control unit 13 transmits the detected data input from the first through fourth sensor units 15 through 18 to the portable terminal 3110 through the SWC network 3910, and upon receiving vibration information from the spine management application 3210 through the portable terminal 3110, drives the vibrator 531 of each of the first through fourth sensor units 15 through 18 according to the received vibration information, thereby enabling the subject 10 to receive vibration (stimulation) suitable to his/her muscle activity.

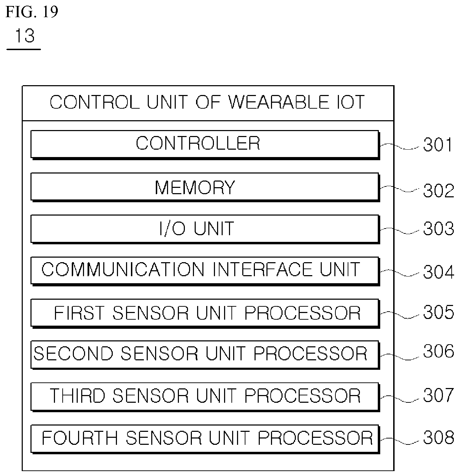

FIG. 19 is a block diagram of the control unit 13 of the wearable IoT 11 of FIG. 17.

As shown in FIG. 19, the control unit 13 of the wearable IoT 11 includes a controller 301, a memory 302, an I/O unit 303, a communication interface unit 304, a first sensor unit processor 305, a second sensor unit processor 306, a third sensor unit processor 307, and a fourth sensor unit processor 308.

The controller 301 is an OS of the control unit 13, and manages and controls control targets, i.e., the memory 302, the I/O unit 303, the communication interface unit 304, and the first through fourth sensor unit processors 305 through 308.

Also, upon receiving data from the first through fourth sensor units 15 through 18 through the I/O unit 303, the controller 301 inputs the data received from the first sensor unit 15 to the first sensor unit processor 305, inputs the data received from the second sensor unit 16 to the second sensor unit processor 306, inputs the data received from the third sensor unit 17 to the third sensor unit processor 307, and inputs the data received from the fourth sensor unit 18 to the fourth sensor unit processor 308.

Also, when sensing values are detected by the first through fourth sensor unit processors 305 through 308, the controller 301 controls the communication interface unit 304 to transmit the detected sensing values to the portable terminal 3110 through the SWC network 3910. Here, the sensing values include acceleration, an angular speed, and geomagnetism of each of the first through fourth sensor units 15 through 18.

Also, upon receiving the vibration information from the portable terminal 3110 through the communication interface unit 304, the controller 301 refers to ID information of the received vibration information to transmit the vibration information to a corresponding sensor unit processor.

For example, upon receiving vibration information including vibration depth and intensity of the second sensor unit 16 from the portable terminal 3110, the controller 301 transmits the vibration information to the second sensor unit processor 306.

The memory 302 stores ID information of each of the first through fourth sensor units 15 through 18.

Also, the memory 302 stores the sensing values (for example, a 3-axis acceleration value, an angular speed value, and geomagnetic information) of each of the first through fourth sensor units 15 through 18 detected by the first through fourth sensor unit processor 305 through 308.

The I/O unit 303 receives or transmits data from or to the first through fourth sensor units 15 through 18.

The communication interface unit 304 communicates with the portable terminal 3110 by supporting connection with the SWC network 3910.

The first through fourth sensor unit processors 305 through 308 detects the sensing values of the first through fourth sensor units 15 through 18 by analyzing the detected data of the first through fourth sensor units 15 through 18 received through the I/O unit 303. Here, the sensing values may include angular speed values of X-, Y-, and Z-axes measured by the gyrosensor, acceleration values of X-, Y-, and Z-axes measured by the acceleration sensor, and geomagnetic values measured by the geomagnetic sensor.

Also, the first through fourth sensor unit processors 305 through 308 generate control signals corresponding to the vibration information received from the portable terminal 3110, and outputs the generated control signals to the first through fourth sensor units 15 through 18 through the I/O unit 303. Here, the control signal includes a raised height of the rod 3731 of the cylinder 573 and the vibration intensity of the vibrator 531.

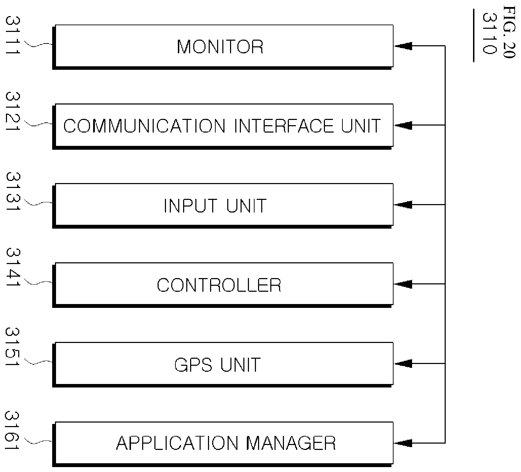

FIG. 20 is a block diagram of the portable terminal 3110 of FIG. 15.

As shown in FIG. 20, the portable terminal 3110 includes: a monitor 3111 commonly included in a smart phone and displaying content; a communication interface unit 3121 connecting to the communication network 3710 or the SWC network 3910 to exchange data with the management server 3310 or the wearable IoT 11; an input unit 3131 receiving a character and a symbol from a user; a controller 3141 operating as an OS of the portable terminal 3110 and controlling a control target; a GPS unit 3151 calculating a location of the portable terminal 3110 based on a signal received from a GPS satellite; and an application manager 3161 managing installation and driving of the spine management application 3210.

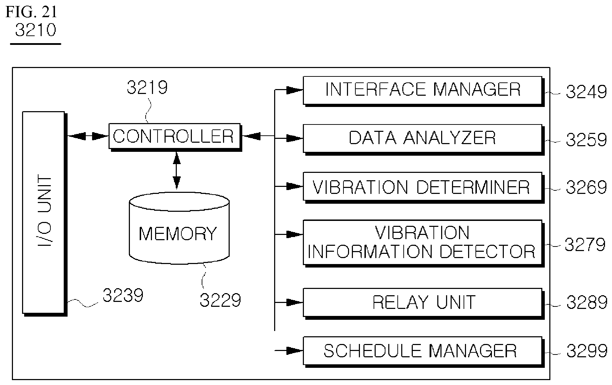

FIG. 21 is a block diagram of the spine management application 3210 of FIG. 17.

The spine management application 3210 is an application program installed in the portable terminal 3110.

Also, as shown in FIG. 21, the spine management application 3210 includes a controller 3219, a memory 3229, an I/O unit 3239, an interface manager 3249, a data analyzer 3259, a vibration determiner 3269, a vibration information detector 3279, a relay unit 3289, and a schedule manager 3299.

The controller 319 is an OS of the spine management application 3210, and manages and controls control targets, i.e., the memory 3229, the I/O unit 3239, the interface manager 3249, the data analyzer 3259, the vibration determiner 3269, the vibration information detector 3279, the relay unit 3289, and the schedule manager 3299.

Also, upon receiving, from the portable terminal 3110, the detected data transmitted from the wearable IoT 11, the controller 3219 transmits the detected data to the data analyzer 3259 and the vibration determiner 3269.

Also, when the data analyzer 3259 detects the respiration pattern and the spinal alignment state, the controller 3219 controls the portable terminal 3110 to transmit the detected respiration pattern and the spinal alignment state to the management server 3310.

Also, when the vibration determiner 3269 determines that the vibrator 531 of at least one of the first through fourth sensor units 15 through 18 needs to vibrate, the controller 3219 drives the vibration information detector 3279.

Also, when the vibration information detector 3279 detects the vibration information, the controller 3219 controls the portable terminal 3110 to transmit the detected vibration information to the wearable IoT 11 through the SWC network 3910.

Also, upon receiving the correction information interface, i.e., a GUI displaying RAB and a training posture, from the management server 3310, the controller 3219 inputs the correction information interface to the interface manager 3249. Here, the interface manager 3249 displays the correction information interface on the monitor 3111 of the portable terminal 3110.

Also, upon receiving a request for a video call with the expert terminal 3510 from a user, the controller 3219 drives the relay unit 3289.

The memory 3229 stores the sensing values received from the wearable IoT 11.

Also, the memory 3229 stores the pre-set analysis algorithm for detecting the motion vector of the muscle (muscle of the left or right ribs, or left or right transverse processes) by processing an operation using the sensing values received from the wearable IoT 11 as input values. Here, the pre-set analysis algorithm is used for operation processes of the data analyzer 3259.

Also, the memory 3229 stores the pre-set activity detection algorithm for detecting activity, i.e., an activation degree, of the muscle, based on the motion vector of the muscle. Here, the pre-set activity detection algorithm is used for operation processes of the vibration determiner 3269.

Also, the memory 3229 stores the pre-set vibration intensity and height detection algorithm for detecting the raised height H of the pressurizing unit 57 and the vibration intensity according to analysis results detected by the data analyzer 3259. Here, the vibration intensity and height detection algorithm is used for operation processes of the vibration information detector 3279.

The interface manager 3249 manages pre-manufactured GUIs. Here, any one of various well-known configurations may be applied to the GUI.

Also, upon receiving the correction information interface transmitted from the management server 3310, the interface manager 3249 displays the correction information interface on the monitor 3111 of the portable terminal 3110.

The data analyzer 3259 analyzes the sensing values by using the pre-set analysis algorithm to detect the motion vector of the muscle during inhalation and exhalation of the subject 10.

In other words, the data analyzer 3259 detects the motion vector of the left ribs during inhalation and exhalation by analyzing the sensing values measured by the first sensor unit 15, detects the motion vector of the right ribs during inhalation and exhalation by analyzing the sensing values measured by the second sensor unit 16, detects the motion vector of the left transverse processes of lumbar vertebrae during inhalation and exhalation by analyzing the sensing values measured by the third sensor unit 17, and detects the motion vector of the right transverse processes of lumbar vertebrae during inhalation and exhalation by analyzing the sensing values measured by the fourth sensor unit 18.

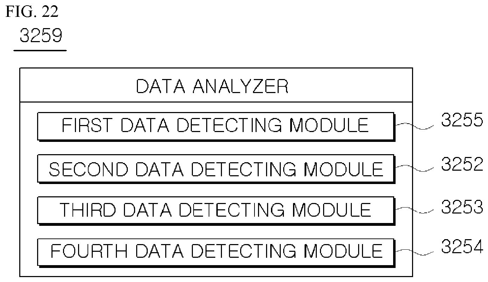

FIG. 22 is a block diagram of the data analyzer 3259 of FIG. 21.

As shown in FIG, 22, the data analyzer 3259 includes a first data detecting module 3255, a second data detecting module 3252, a third data detecting module 3253, and a fourth data detecting module 3254.

The first data detecting module 3255 detects, by analyzing the sensing values measured by the first sensor unit 15 by using the pre-set analysis algorithm, 1) first inhalation detailed information D1 indicating a motion vector of the left ribs during inhalation, 2) first exhalation detailed information D1' indicating a motion vector of the left ribs during exhalation, and 3) first displacement information ΔD1 indicating a displacement vector of the first inhalation detailed information D1 and the first exhalation detailed information D1'.

The second data detecting module 3252 detects, by analyzing the sensing values measured by the second sensor unit 16 by using the pre-set analysis algorithm, 1) second inhalation detailed information D2 indicating a motion vector of the right ribs during inhalation, 2) second exhalation detailed information D2' indicating a motion vector of the right ribs during exhalation, and 3) second displacement information ΔD2 indicating a displacement vector of the second inhalation detailed information D2 and the second exhalation detailed information D2'.

The third data detecting module 3253 detects, by analyzing the sensing values measured by the third sensor unit 17 by using the pre-set analysis algorithm, 1) third inhalation detailed information D3 indicating a motion vector of the left transverse processes of lumbar vertebrae during inhalation, 2) third exhalation detailed information D3' indicating a motion vector of the left transverse processes of lumbar vertebrae during exhalation, and 3) third displacement information ΔD3 indicating a displacement vector of the third inhalation detailed information D3 and the third exhalation detailed information D3'.

The fourth data detecting module 3254 detects, by analyzing the sensing values measured by the fourth sensor unit 18 by using the pre-set analysis algorithm, 1) fourth inhalation detailed information D4 indicating a motion vector of the right transverse processes of lumbar vertebrae during inhalation, 2) fourth exhalation detailed information D4' indicating a motion vector of the right transverse processes of lumbar vertebrae during exhalation, and 3) fourth displacement information ΔD4 indicating a displacement vector of the fourth inhalation detailed information D4 and the fourth exhalation detailed information D4'.

The first through fourth inhalation detailed information D1 through D4, the first through fourth exhalation detailed information D1' through D4', and the first through fourth displacement information ΔD1 through ΔD4 detected by the data analyzer 3259 are input to the vibration determiner 3269 according to control of the controller 3219.

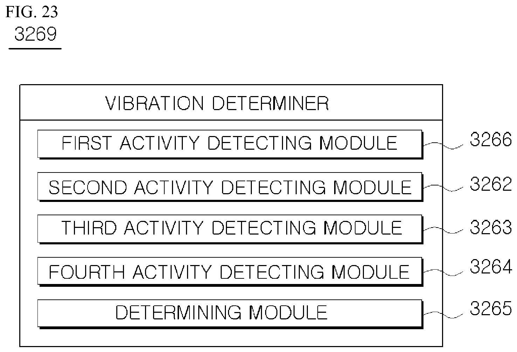

FIG. 23 is a block diagram of the vibration determiner 3269 of FIG. 21.

As shown in FIG. 23, the vibration determiner 3269 includes a first activity detecting module 3266, a second activity detecting module 3262, a third activity detecting module 3263, a fourth activity detecting module 3264, and a determining module 3265.

The first activity detecting module 3266 measures first activity A1, i.e., activity of muscles corresponding to the left ribs, by analyzing the first inhalation detailed information D1, the first exhalation detailed information D1', and the first displacement information ΔD1 input from the data analyzer 3259 by using the pre-set activity measuring algorithm.

The second activity detecting module 3262 measures second activity A2, i.e., activity of muscles corresponding to the right ribs, by analyzing the second inhalation detailed information D2, the second exhalation detailed information D2', and the second displacement information ΔD2 input from the data analyzer 3259 by using the pre-set activity measuring algorithm.

The third activity detecting module 3263 measures third activity A3, i.e., activity of muscles corresponding to the left transverse processes of lumbar vertebrae, by analyzing the third inhalation detailed information D3, the third exhalation detailed information D3', and the third displacement information ΔD3 input from the data analyzer 3259 by using the pre-set activity measuring algorithm.

The fourth activity detecting module 3264 measures fourth activity A4, i.e., activity of muscles corresponding to the right transverse processes of lumbar vertebrae, by analyzing the fourth inhalation detailed information D4, the fourth exhalation detailed information D4', and the fourth displacement information ΔD4 input from the data analyzer 3259 by using the pre-set activity measuring algorithm.

The determining module 3265 compares the first activity A1 detected by the first activity detecting module 3266 with a pre-set threshold value TH, and when the first activity A1 is equal to or greater than the threshold value TH, determines that the muscles corresponding to the left ribs do not need to be vibrated (stimulated), and when the first activity A1 is smaller than the threshold value TH, determines that the muscles need to be vibrated (stimulated).

Here, the threshold value TH is defined as a smallest value of muscle activity in which respiration is determined to be normal or the muscles are determined to be activated.

Also, the determining module 3265 compares the second activity A2 detected by the second activity detecting module 3262 with the threshold value TH, and when the second activity A2 is equal to or greater than the threshold value TH, determines that the muscles corresponding to the right ribs do not need to be vibrated (stimulated), and when the second activity A2 is smaller than the threshold value TH, determines that the muscles need to be vibrated (stimulated).

Also, the determining module 3265 compares the third activity A3 detected by the third activity detecting module 3263 with the threshold value TH, and when the third activity A3 is equal to or greater than the threshold value TH, determines that the muscles corresponding to the left transverse processes of lumbar vertebrae do not need to be vibrated (stimulated), and when the third activity A3 is smaller than the threshold value TH, determines that the muscles need to be vibrated (stimulated).

Also, the determining module 3265 compares the fourth activity A4 detected by the fourth activity detecting module 3264 with the threshold value TH, and when the fourth activity A4 is equal to or greater than the threshold value TH, determines that the muscles corresponding to the right transverse processes of lumbar vertebrae do not need to be vibrated (stimulated), and when the fourth activity A4 is smaller than the threshold value TH, determines that the muscles need to be vibrated (stimulated).

The vibration information detector 3279 is driven when the vibration determiner 3269 determines that at least one of the first through sensor units 15 through 18 needs to vibrate.

Also, the vibration information detector 3279 receives inhalation detailed information, exhalation detailed information, and displacement information of a sensor unit that is determined that vibration is needed.

Also, the vibration information detector 3279 detects vibration intensity and height of a vibrator by analyzing input data by using the pre-set vibration intensity and height detection algorithm.

Also, vibration information (the vibration intensity and height) detected by the vibration information detector 3279 is input to the wearable IoT 11 through the portable terminal 3110 according to control of the controller 3219.

Upon receiving a request for a video call with the expert terminal 3510 from a user, the relay unit 3289 requests the management server 3310 for relay, and relays the video call with the expert terminal 3510 matched by the management server 3310.

The schedule manager 3299 manages schedules, such as treatments and training dates of a professional organization.

As such, the scoliosis correction system 30 according to an embodiment enables the subject 10 to conveniently measure his/her spinal condition alone without the help of others by including the first through fourth sensor units 15 through 18 attachable or detachable to or from the body of the subject 10 while contacting the left and right ribs and the left and right transverse processes of lumbar vertebrae of the subject 10 and also including the wearable IoT 11 capable of detecting the sensing values of muscles used to determine the spinal condition of the subject 10.

Also, the scoliosis correction system 30 includes the spine management application 3210 that detects the activity of each muscle by analyzing the sensing values received from the wearable IoT 11 and then determines whether the muscle needs to be vibrated (stimulated) based on the detected activity, while wearable IoT 11 is configured such that each sensor unit includes the vibrator 531 and the vibrator 531 vibrates according to the vibration information received from the spine management application 3210. Accordingly, the scoliosis correction system 30 provides not only a function of diagnosing the spinal alignment state of the subject 10, but also a function of correcting the abnormally aligned spine by vibrating (stimulating) the inactivated muscles of the subject 10 by using the Schroth theory widely known as scoliosis treatment.

Also, the scoliosis correction system 30 is configured such that the subject 10 may self-diagnose and self-treat him/herself, and thus the subject 10 may correct him/herself without having to visit a separate professional organization. Accordingly, unnecessary time consumption and costs may be reduced and user convenience may be increased.

Also, the scoliosis correction system 30 is configured such that the length of the vibrator 531 of the wearable IoT 11 is changeable while the spine management application 3210 is configured such that the vibration intensity and height of the vibrator 531 are detected according to the sensing values of each muscle, and the wearable IoT 11 is configured such that vibration is performed according to the vibration intensity and height of the vibration information received from the spine management application 3210. Accordingly, each muscle is suitably vibrated (stimulated) based on the activity, and thus accuracy and precision of the correction may be increased.

Also, the management server 3310 detects the respiration pattern and the spinal alignment state of the subject 10 by analyzing the sensing values received from the spine management application 3210 and then detects and transmits, to the spine management application 3210, the optimum RAB and training posture corresponding to the detected respiration pattern and spinal alignment state, and the spine management application 3210 displays the optimum RAB and training posture received from the management server 3310 on the monitor 3111 of the portable terminal 3110. Accordingly, the scoliosis correction system 30 may enable the subject 10 to correct the respiration pattern and the spinal alignment state through the received optimum RAB and training posture without having to visit a separate medical care center or medical institution.

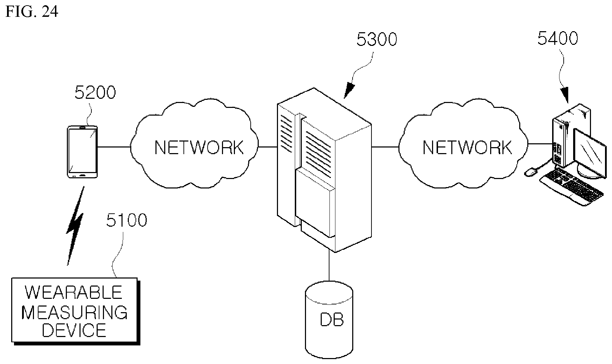

Hereinafter, a system for remotely diagnosing spine 5300 according to an embodiment will be described with reference to FIGS. 24 through 51.

FIG. 24 is a diagram for describing processes of remotely diagnosing the spine of a patient by using the system for remotely diagnosing spine 5300 according to an embodiment.

Referring to FIG. 24, a wearable measuring device 5100, a patient terminal 5200, the system for remotely diagnosing spine 5300, and a medical staff terminal 5400 may be used to remotely diagnose the spine of the patient.

The wearable measuring device 5100 is worn on the body of the patient and collects raw data required to diagnose the spine of the patient by measuring the patient. The wearable measuring device 5100 transmits the raw data to the patient terminal 5200 by being connected to the patient terminal 5200 via wireless communication.

The patient terminal 5200 is a terminal owned by the patient or a protector of the patient, and may be a mobile terminal, such as a smart device or a tablet PC.

According to an embodiment, the wearable measuring device 5100 and the patient terminal 5200 may exchange data by using short-distance wireless communication, such as Bluetooth, but the short-distance wireless communication is not limited to Bluetooth.

The patient terminal 5200 may generate trunk movement data of the patient based on the raw data received from the wearable measuring device 5100. Then, the patient terminal 5200 may transmit the trunk movement data to the system for remotely diagnosing spine 5300 through a network.

The system for remotely diagnosing spine 5300 may store the trunk movement data received from the patient terminal 5200, and generate diagnostic data about the patient based on the stored trunk movement data. The system for remotely diagnosing spine 5300 provides the generated diagnostic data to the medical staff terminal 5400 through a network such that a medical staff remotely diagnoses the patient.

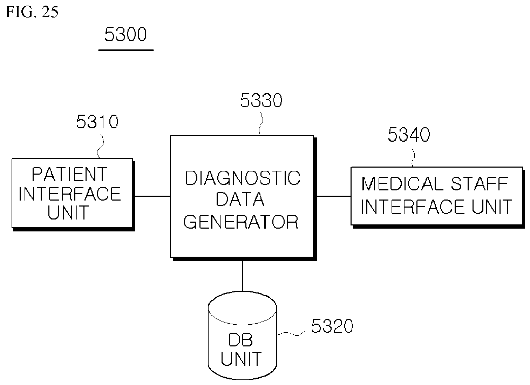

FIG. 25 is a block diagram of the system for remotely diagnosing spine 5300 according to an embodiment.

The system for remotely diagnosing spine 5300 is a computing apparatus including a processor (for example, a central processing unit (CPU) or a graphic processing unit (GPU)) processing data and a memory (for example, hard disk drive (HDD), a solid state disk (SSD), a random access memory (RAM), or a read-only memory (ROM) storing data, and for example, may be a server.

Referring to FIG. 25, the system for remotely diagnosing spine 5300 includes a patient interface unit 5310, a database (DB) unit 5320, a diagnostic data generator 5330, and a medical staff interface unit 5340.

The patient interface unit 5310 receives, from the patient terminal 5200, the trunk movement data of the patient generated as the wearable measuring device 5100 measures the patient. The patient interface unit 5310 may include a communication module capable of exchanging data with a client or server by connecting to a network.

The DB unit 5320 stores the trunk movement data. The DB unit 5320 is a storage device that stores data, and may include, for example, not only a mass storage device, such as HDD or SSD, but also a memory device, such as RAM, ROM, a cache, or a register.

The diagnostic data generator 5330 generates diagnostic data about the patient based on the trunk movement data. The diagnostic data generator 5330 includes a processor that processes data, such as CPU or GPU, and may generate the diagnostic data by processing the trunk movement data according to a pre-stored algorithm or program.

The medical staff interface unit 5340 provides the diagnostic data to the medical staff terminal 5400. Like the patient interface unit 5310, the medical staff interface unit 5340 includes a communication module capable of exchanging data with a client or server by connecting to a network, and exchanges data with the medical staff terminal 5400 through the network.

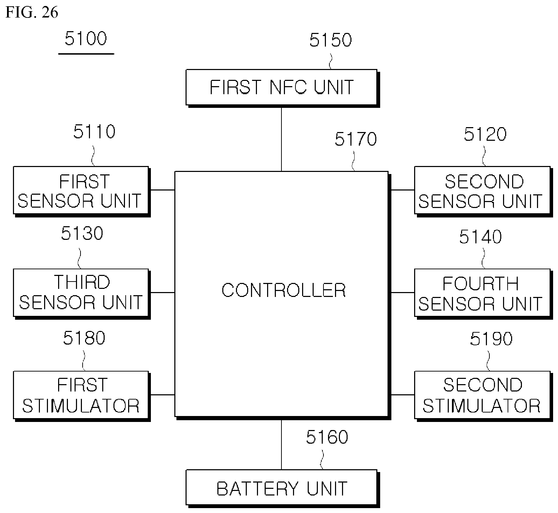

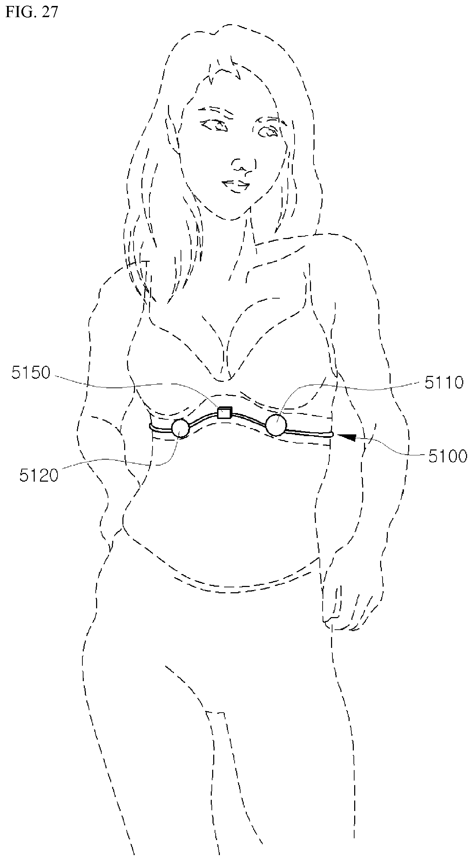

FIG. 26 is a block diagram of the wearable measuring device 5100 according to an embodiment, and FIG. 27 is a diagram of the wearable measuring device 5100 being worn on the patient, according to an embodiment.

According to an embodiment, the wearable measuring device 5100 is worn on the body of the patient and collects various types of raw data used to diagnose the spine of the patient. Referring to FIG. 26, the wearable measuring device 5100 may include a first sensor unit 5110, a second sensor unit 5120, and a first SWC unit 5150.

The first sensor unit 5110 detects movement of the left chest of the patient.

The second sensor unit 5120 detects movement of the right chest of the patient. The first SWC unit 5150 transmits data output from the first and second sensor units 5110 and 5120 to the patient terminal 5200.

Also, the wearable measuring device 5100 may include a battery unit 5160 and a controller 5170. The battery unit 5160 supplies power to each module of the wearable measuring device 5100. The controller 5170 controls operations of the wearable measuring device 5100.

As shown in FIG. 27, the wearable measuring device 5100 may be integrated into clothing such that the wearable measuring device 5100 is worn on the body of the patient when the patient wears the clothing.

According to an embodiment, the first and second sensor units 5110 and 5120 may include an acceleration sensor detecting acceleration with respect to at least one axial direction.

As shown in FIG. 27, the first sensor unit 5110 may be provided at a part of the clothing where the left chest of the patient is located so as to output an acceleration value according to movement of the left chest of the patient. Similarly, the second sensor unit 5120 may be provided at a part of the clothing where the right chest of the patient is located so as to output an acceleration value according to movement of the right chest of the patient.

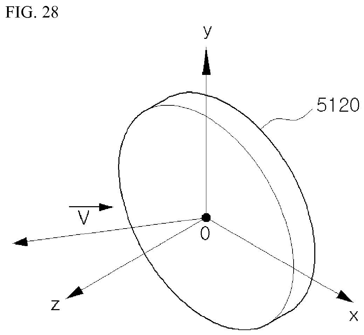

FIG. 28 is a diagram for describing processes of generating the trunk movement data of the patient by using the second sensor unit 5120, according to an embodiment.

As shown in FIG. 28, the second sensor unit 5120 may output data about a displacement vector V by detecting acceleration in x-, y-, and z-axis directions according to chest movement of the patient.

According to an embodiment, when the chest moves as the patient breathes, the first and second sensor units 5110 and 5120 may output data for defining the displacement vector V related to a direction and size of the chest movement.

Also, the patient terminal 5200 generates a left chest displacement vector related to the movement of the left chest based on data output from the first sensor unit 5110 and generates a right chest displacement vector related to the movement of the right chest based on data output from the second sensor unit 5120.

According to the current embodiment, the patient terminal 5200 generates displacement vectors indicating the chest movement of the patient by using the first and second sensor units 5110 and 5120 including the acceleration sensor, but according to an embodiment, the first and second sensor units 5110 and 5120 may include at least one of an acceleration sensor, a gyrosensor, and a geomagnetic sensor.

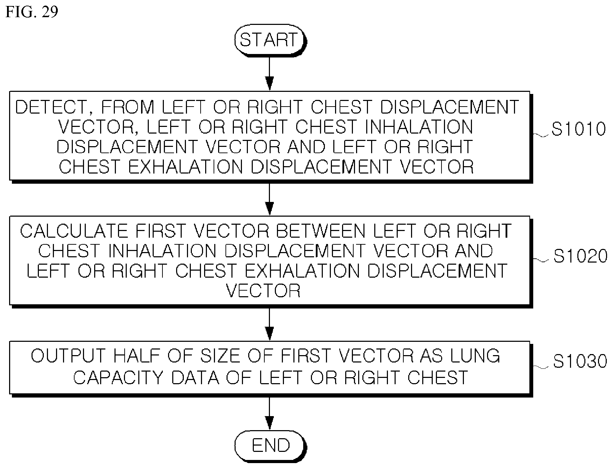

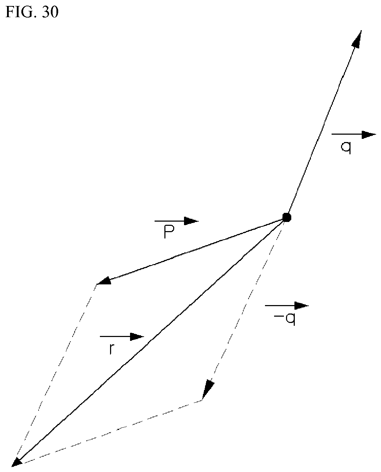

FIG. 29 is a flowchart of a method of generating diagnostic data about a patient, according to an embodiment, and FIG. 30 is a diagram for describing processes of calculating a first vector, according to an embodiment.

According to an embodiment, in the system for remotely diagnosing spine 5300, the diagnostic data generator 5330 may generate lung capacity data related to lung capacity of the left or right chest of the patient by receiving the left chest displacement vector or the right chest displacement vector of the patient.

Referring to FIGS. 29 and 30, the method, performed by the diagnostic data generator 5330, of generating diagnostic data about a patient may include detecting a left or right chest inhalation displacement vector P corresponding to inhalation of the patient and a left or right chest exhalation displacement vector q corresponding to exhalation of the patient from a received left or right chest displacement vector (operation S1010), calculating a first vector r between the left or right chest inhalation displacement vector P and the left or right chest exhalation displacement vector q (operation S1020), and outputting a half of the size of the first vector r as lung capacity data of the left or right chest (operation S1030).

According to an embodiment, the diagnostic data generator 5330 may determine, as the left or right chest inhalation displacement vector P corresponding to the inhalation of the patient, a displacement vector in a distal direction based on the body of the patient from among the left or right chest displacement vector received from the patient terminal 5200.

On the other hand, the diagnostic data generator 5330 may determine, as the left or right chest exhalation displacement vector q corresponding to the exhalation of the patient, a displacement vector in a proximal direction based on the body of the patient from among the left or right chest displacement vector received from the patient terminal 5200.

Then, the diagnostic data generator 5330 may calculate the first vector r between the left or right chest inhalation displacement vector P and the left or right chest exhalation displacement vector q, and generate lung capacity data of the left chest based on the size of the first vector r.

Similarly, the diagnostic data generator 5330 may calculate the first vector r between the left or right chest inhalation displacement vector P and the left or right chest exhalation displacement vector q, and generate the lung capacity data of the right chest based on the size of the first vector r.

For example, the diagnostic data generator 5330 may generate half of the size of the first vector r as the lung capacity data of the left or right chest, but the lung capacity data may not necessarily be the half of the size of the first vector r, and may be a pre-set ratio of the size of the first vector r.

Referring back to FIG. 26, the wearable measuring device 5100 may further include a first stimulator 5180 and a second stimulator 5190.

The first stimulator 5180 stimulates a left body part of the patient.

The second stimulator 5190 stimulates a right body part of the patient. According to an embodiment, the first and second stimulators 5180 and 1590 may include a vibrator that generates vibration, but the first and second stimulators 5180 and 5190 are not limited thereto, and may include, other than the vibrator, any device capable of stimulating a body part of the patient, such as an electrode applying electric stimulation.

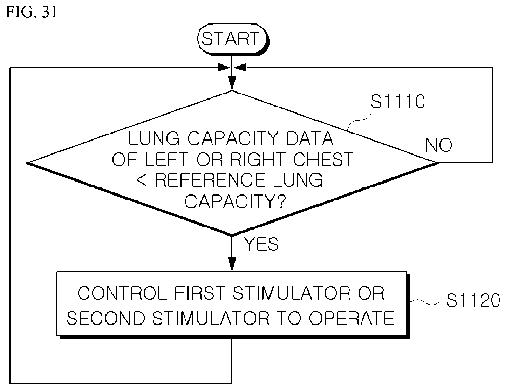

FIG. 31 is a flowchart of a method of controlling the wearable measuring device 5100 to stimulate the patient, according to an embodiment.

Referring to FIG. 31, the method, performed by the patient terminal 5200, of controlling the wearable measuring device 5100 may include controlling the first stimulator 5180 to operate (operation S1120) when it is determined that the lung capacity data of the left chest is smaller than pre-set reference lung capacity ('Yes' in operation S1110).

Also, the method may include controlling the second stimulator 5190 to operate (operation S1120) when it is determined that the lung capacity data of the right chest is smaller than the pre-set reference lung capacity ('Yes' in operation S1110).

According to the current embodiment, the patient terminal 5200 may control the first or second stimulator 5180 or 5190 to stimulate the left or right chest of the patient by using vibration or electric stimulation, when the lung capacity data of the left or right chest is smaller than the pre-set reference lung capacity, based on the lung capacity data of the left or right chest generated by the diagnostic data generator 5330.

As a result, the patient wearing the wearable measuring device 5100 may consciously and actively move a chest that has low lung capacity due to abnormal bending of the spine, by recognizing the chest through stimulation. Accordingly, the patient may obtain a treatment effect of correcting the spine by training inactivated muscles of the chest.

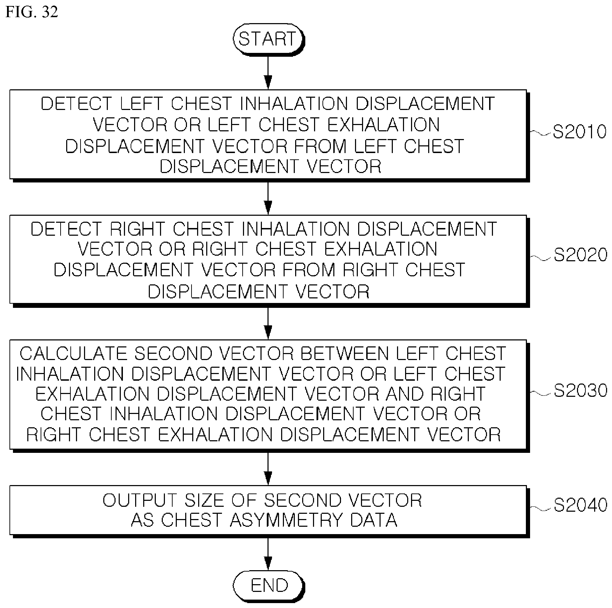

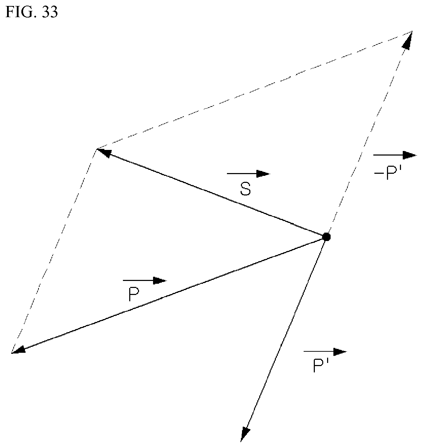

FIG. 32 is a flowchart of a method of generating the diagnostic data about the patient, according to another embodiment, and FIG. 33 is a diagram for describing processes of calculating a second vector, according to an embodiment.

According to an embodiment, the diagnostic data generator 5330 may generate chest asymmetry data about asymmetry between the left chest and the right chest of the patient by receiving the left chest displacement vector and the right chest displacement vector of the patient.

Referring to FIG. 32, the method, performed by the diagnostic data generator 5330, of generating the diagnostic data includes detecting a left chest inhalation displacement vector or a left chest exhalation displacement vector corresponding to inhalation or exhalation of the patient from a received left chest displacement vector (operation S2010), detecting a right chest inhalation displacement vector or a left chest exhalation displacement vector corresponding to inhalation or exhalation of the patient from a received right chest displacement vector (operation S2020), calculating a second vector between the left chest inhalation displacement vector or left chest exhalation displacement vector and the right chest inhalation displacement vector or right chest exhalation displacement vector (operation S2030), and outputting a size of the second vector as the chest asymmetry data (operation S2040).

For example, referring to FIG. 33, the diagnostic data generator 5330 may detect a left chest inhalation displacement vector P corresponding to inhalation of the patient from a left chest displacement vector of the patient received from the patient terminal 5200. Also, the diagnostic data generator 5330 may detect a right chest inhalation displacement vector P corresponding to inhalation of the patient from a right chest displacement vector of the patient received from the patient terminal 5200.

Then, the diagnostic data generator 5330 calculates a second vector S between the left chest inhalation displacement vector P and the right chest inhalation displacement vector P', and output a size of the second vector S as chest asymmetry data.

In FIG. 33, the chest asymmetry data is generated based on inhalation of the patient, but the diagnostic data generator 5330 may calculate a second vector between a left chest exhalation displacement vector and a right chest exhalation displacement vector based on exhalation of the patient, and output a size of the second vector as the chest asymmetry data.

Also, according to an embodiment, the diagnostic data generator 5330 may output a pre-set ratio of the size of the second vector as the chest asymmetry data instead of outputting the size of the second vector as the chest asymmetry data.

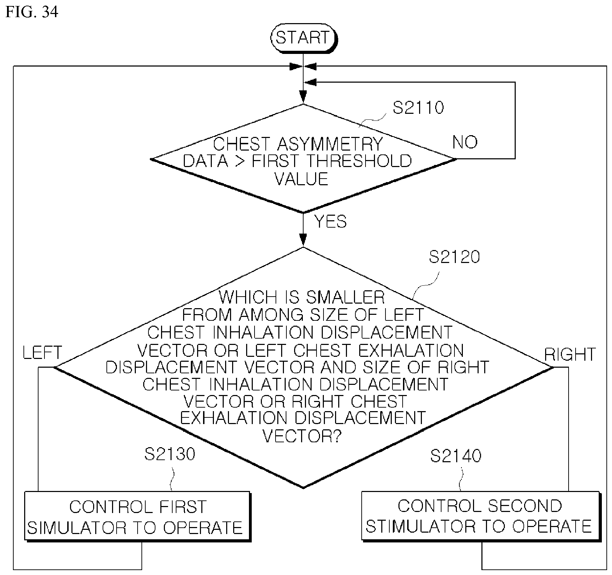

FIG. 34 is a flowchart of a method of controlling the wearable measuring device 5100 to stimulate the patient, according to another embodiment.

Referring to FIG. 34, the method, performed by the patient terminal 5200, of controlling the wearable measuring device 5100 may include controlling the first stimulator 5180 to operate (operation S2130) when it is determined that the chest asymmetry data is greater than a pre-set first threshold value ('Yes' in operation S2110) and that a size of a left chest inhalation displacement vector or left chest exhalation displacement vector is smaller than a size of a right chest inhalation displacement vector or right chest exhalation displacement vector ('Left' in operation S2120).

Also, the method may include controlling the second stimulator 5190 to operate (operation S2140) when it is determined that the chest asymmetry data is greater than the pre-set first threshold value ('Yes' in operation S2110) and that the size of the right chest inhalation displacement vector or right chest exhalation displacement vector is smaller than the size of the left chest inhalation displacement vector or left chest exhalation displacement vector ('Right' in operation S2120).

According to the current embodiment, the patient terminal 5200 may control the first or second stimulator 5180 or 5190 to stimulate one of the left and right chests of the patient, which moves less during respiration, by using vibration or electric stimulation, when chest asymmetry data is greater than the pre-set first threshold value based on the chest asymmetry data generated by the diagnostic data generator 5330.

As a result, the patient wearing the wearable measuring device 5100 may consciously and actively move one of the left and right chests, which moves less during respiration, by recognizing the corresponding chest through stimulation when the left and right chests are severely asymmetric due to abnormal bending of the spine. Accordingly, the patient may obtain a treatment effect of correcting the spine by training inactivated muscles of the corresponding chest.

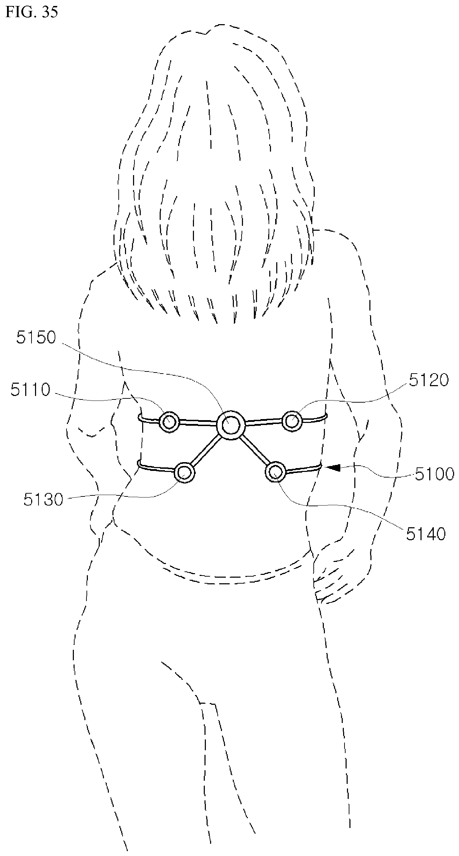

FIG. 35 is a diagram of the wearable measuring device 5100 being worn on the patient, according to an embodiment.

Unlike the wearable measuring device 5100 including the first and second sensor units 5110 and 5120 described above, the wearable measuring device 5100 according to the current embodiment may further include a third sensor unit 5130 and a fourth sensor unit 5140 detecting movement of a left waist portion of the patient, as shown in FIG. 26.

In this case, the first SWC unit 5150 may further transmit data output from the third and fourth sensor units 5130 and 5140 to the patient terminal 5200, in addition to data output from the first and second sensor units 5110 and 5120.

According to the current embodiment, the patient terminal 5200 may generate a left waist portion displacement vector related to movement of the left waist portion of the patient based on the data output from the third sensor unit 5130, and generate a right waist portion displacement vector related to movement of a right waist portion of the patient based on the data output from the fourth sensor unit 5140.

As described above with reference to FIG. 28, like the first and second sensor units 5110 and 5120, the third and fourth sensor units 5130 and 5140 may include an acceleration sensor detecting acceleration with respect to at least one axial direction. In this case, the third and fourth sensor units 5130 and 5140 may output data related to the displacement vector V by detecting acceleration in x-, y-, and z-axis directions according to waist portion movement of the patient.

Also, the patient terminal 5200 may generate the left waist portion displacement vector related to movement of the left waist portion of the patient based on the data output from the third sensor unit 5130, and generate the right waist portion displacement vector related to movement of the right waist portion of the patient based on the data output from the fourth sensor unit 5140.

Like the first and second sensor units 5110 and 5120, the third and fourth sensor units 5130 and 5140 may include at least one of an acceleration sensor, a gyrosensor, and a geomagnetic sensor so as to generate a displacement vector indicating waist portion movement of the patient.

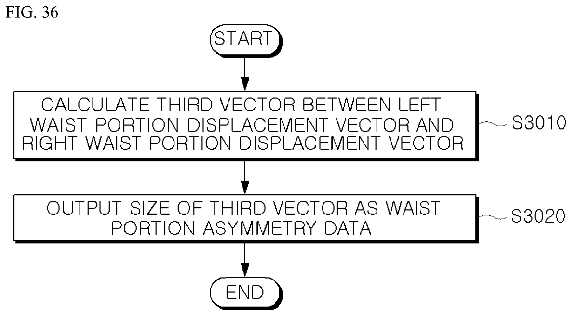

FIG. 36 is a flowchart of a method of generating the diagnostic data about the patient, according to another embodiment.

According to an embodiment, the diagnostic data generator 5330 may generate a waist portion asymmetry data about asymmetry between the left waist portion and the right waist portion of the patient by receiving the left waist portion displacement vector and the right waist portion displacement vector of the patient.

Referring to FIG. 36, the method, performed by the diagnostic data generator 5330, of generating the diagnostic data may include calculating a third vector between the left waist portion displacement vector and the right waist portion displacement vector (operation S3010), and outputting a size of the third vector as the waist portion asymmetry data (operation S3020).

According to an embodiment, the diagnostic data generator 5330 may output a pre-set ratio of the size of the third vector as the waist portion asymmetry data instead of outputting the size of the third vector as the waist portion asymmetry data.

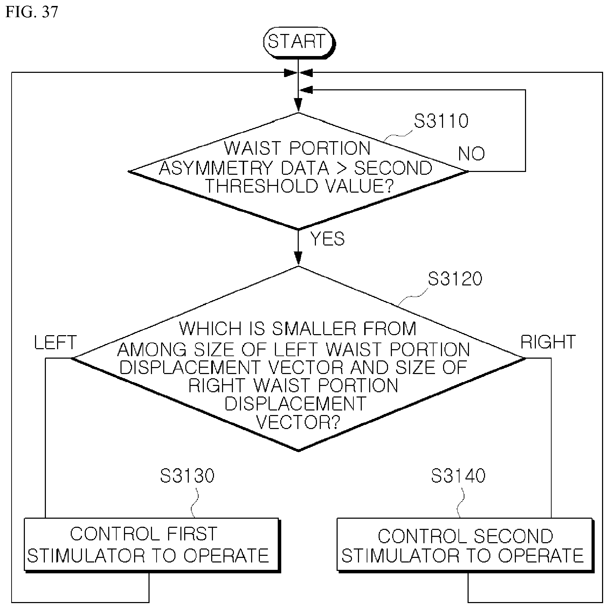

FIG. 37 is a flowchart of a method of controlling the wearable measuring device 5100 to stimulate the patient, according to another embodiment.

Referring to FIG. 37, the method, performed by the patient terminal 5200, of controlling the wearable measuring device 5100 may include controlling the first stimulator 5180 to operate (operation S3130) when it is determined that the waist portion asymmetry data is greater than a pre-set second threshold value ('Yes' in operation S3110) and that the size of the left waist portion displacement vector is smaller than the size of the right waist portion displacement vector ('Left' in operation S3120).

Also, the method may include controlling the second stimulator 5190 to operate (operation S3140) when it is determined that the waist portion asymmetry data is greater than a pre-set second threshold value ('Yes' in operation S3110) and that the size of the right waist portion displacement vector is smaller than the size of the left waist portion displacement vector ('Right' in operation S3120).

According to the current embodiment, the patient terminal 5200 may control the first or second stimulator 5180 or 5190 to stimulate one of the left and right waist portions of the patient, which moves less, by using vibration or electric stimulation, when the waist portion asymmetry data is greater than the pre-set second threshold value based on the waist portion asymmetry data generated by the diagnostic data generator 5330.

As a result, the patient wearing the wearable measuring device 5100 may consciously and actively move one of the left and right waist portions, which moves less, by recognizing the corresponding waist portion through stimulation when the left and right waist portions are severely asymmetric due to abnormal bending of the spine. Accordingly, the patient may obtain a treatment effect of correcting the spine by training inactivated muscles of the corresponding waist portion.

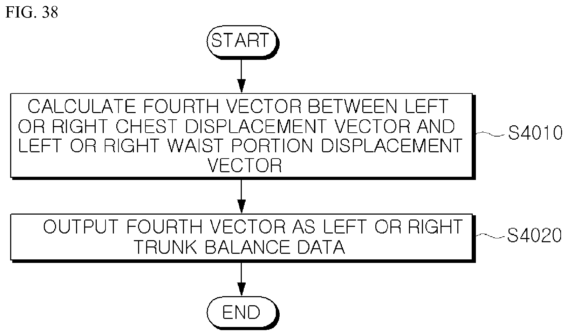

FIG. 38 is a flowchart of a method of generating the diagnostic data about the patient, according to another embodiment.

According to an embodiment, the diagnostic data generator 5330 may generate left or right trunk balance data about balance between the left or right chest and the left or right waist portion of the patient by receiving the left or right chest displacement vector and the left or right waist portion displacement vector of the patient.

Referring to FIG. 38, the method, performed by the diagnostic data generator 5330, of generating the diagnostic data may include calculating a fourth vector between the left or right chest displacement vector and the left or right waist portion displacement vector (operation S4010) and outputting the fourth vector as the left or right trunk balance data (operation S4020).

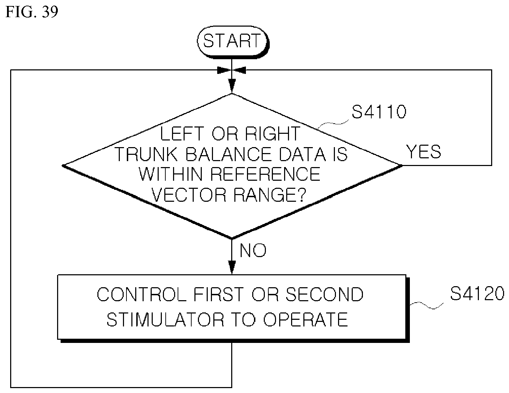

FIG. 39 is a flowchart of a method of controlling the wearable measuring device 5100 to stimulate the patient, according to another embodiment.

Referring to FIG. 39, the method, performed by the patient terminal 5200, of controlling the wearable measuring device 5100 may include controlling the first stimulator 5180 to operate (operation S4120) when it is determined that the left trunk balance data is outside a pre-set reference vector range ('No' in operation S4110).

Also, the method may include controlling the second stimulator 5190 to operate (operation S4120) when it is determined that the right trunk balance data is outside the pre-set reference vector range ('No' in operation S4110).

For example, the patient terminal 5200 may operate the first stimulator 5180 such that the left body part of the patient is stimulated when the fourth vector between the left chest displacement vector and the left waist portion displacement vector respectively collected from the left chest and the left waist portion of the patient is outside a pre-set vector size range and a vector direction range.

Similarly, the patient terminal 5200 may operate the second stimulator 5190 such that the right body part of the patient is stimulated when the fourth vector between the right chest displacement vector and the right waist portion displacement vector respectively collected from the right chest and the right waist portion of the patient is outside the pre-set vector size range and the vector direction range.

In the current embodiment, unlike the left or right chest lung capacity data, the chest asymmetry data between the left and right chests, and the waist portion asymmetry data between the left and right waist portions described above, a movement relationship between one chest and one waist portion is indicated as vector-based trunk balance data based on displacement vectors of the one chest and one waist portion of the patient, and the normality of the spine may be diagnosed by comparing the vector-based trunk balance data with a reference vector range.

In the current embodiment, the patient terminal 5200 may control the first or second stimulator 5180 or 5190 to stimulate the left or right body part of the patient by using vibration or electric stimulation, when the left or right trunk balance data is outside the reference vector range.

As a result, the patient wearing the wearable measuring device 5100 may consciously and actively move a chest and waist portion of one of the left and right trunks, in which movement at the chest and waist portion is outside a normal range due to abnormal bending of the spine, by recognizing the corresponding trunk through stimulation. Accordingly, the patient may obtain a treatment effect of correcting the spine by training inactivated muscles of the chest or waist portion of the corresponding trunk

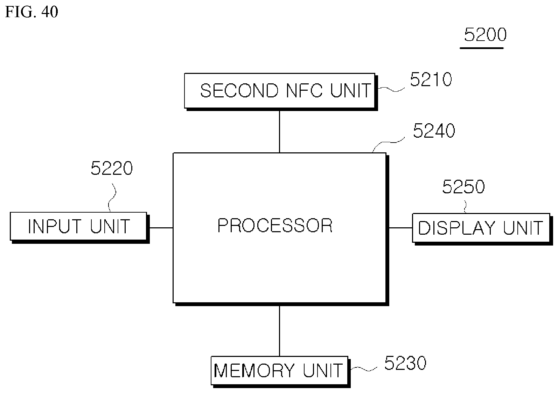

FIG. 40 is a block diagram of the patient terminal 5200 according to an embodiment.

Referring to FIG. 40, the patient terminal 5200 includes a second SWC unit 5210, an input unit 5220, a memory unit 5230, a processor 5240, and a display unit 5250.

The second SWC unit 5210 exchanges data with the wearable measuring device 5100. The input unit 5220 receives a command for driving the patient terminal 5200. The memory unit 5230 stores information about the wearable measuring device 5100 and data received from the wearable measuring device 5100. The processor 5240 generates spine health information indicating a spine health state of the patient based on trunk movement data of the patient. The display unit 5250 displays the spine health information of the patient.

As described above, the patient terminal 5200 may be a mobile terminal owned by the patient or the protector of the patient, such as a smart device or a tablet PC.

The second SWC unit 5210 is connected to the first SWC unit 5150 included in the wearable measuring device 5100 via SWC, and exchanges data with the first SWC unit 5150.

The first and second SWC units 5150 and 5210 may exchange measurement data or control data based on a Bluetooth communication protocol, but an SWC protocol is not limited thereto.

The input unit 5220 is an input device receiving a command from a user using the patient terminal 5200, and may include a touch screen or a keypad. The memory unit 5230 is a storage device storing data, and may include, for example, RAM, ROM, a cache, or a register. The processor 5240 is a processor processing data, and may include, for example, an access point (AP). The display unit 5250 is a display device displaying data on a screen, and may include, for example, a liquid crystal display (LCD).

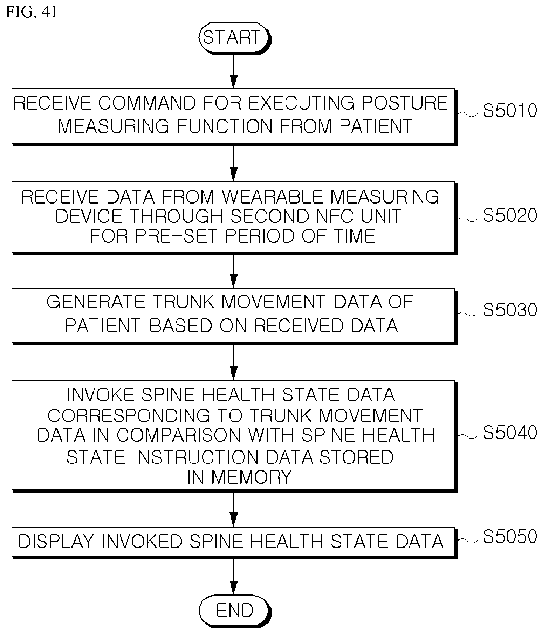

FIG. 41 is a flowchart of a method of providing spine health state data to a patient by measuring a posture of the patient, according to an embodiment.

Referring to FIG. 41, the input unit 5220 receives a command for executing a posture measuring function from the patient, in operation S5010. Then, the processor 5240 receives data from the wearable measuring device 5100 through the second SWC unit 5210 for a pre-set period of time in operation S5020, generates trunk movement data of the patient based on the received data in operation S5030, and invokes spine health state data corresponding to the trunk movement data in preparation for spine health state instruction data stored in the memory 5230 in operation S5040. Then, the display unit 5250 displays the invoked spine health state data in operation S5050.

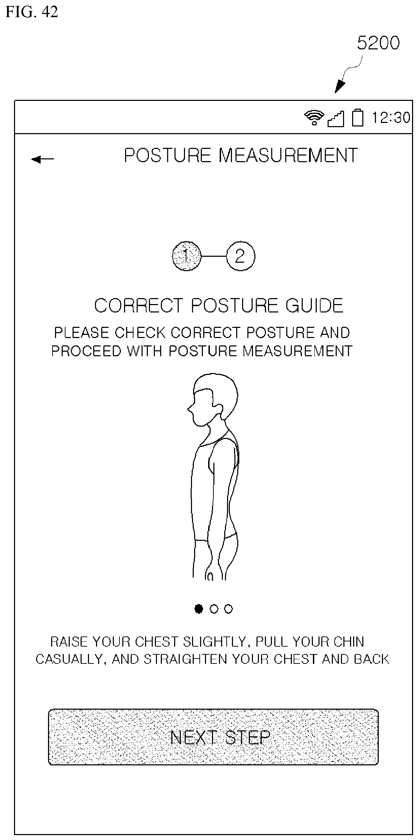

FIGS. 42 through 44 illustrate the patient terminal 5200 providing the spine health state data to the patient by measuring the posture of the patient, according to an embodiment.

In order for the user of the patient terminal 5200 to measure the posture of the patient and determine the spine health state data, the user first inputs the command of executing the posture measuring function to the patient terminal 5200 through the input unit 5220.

When the posture measuring function is executed in the patient terminal 5200, the patient terminal 5200 may display, on the display unit 5250, correct posture guide information showing a correct posture for posture measurement, as shown in FIG. 42.

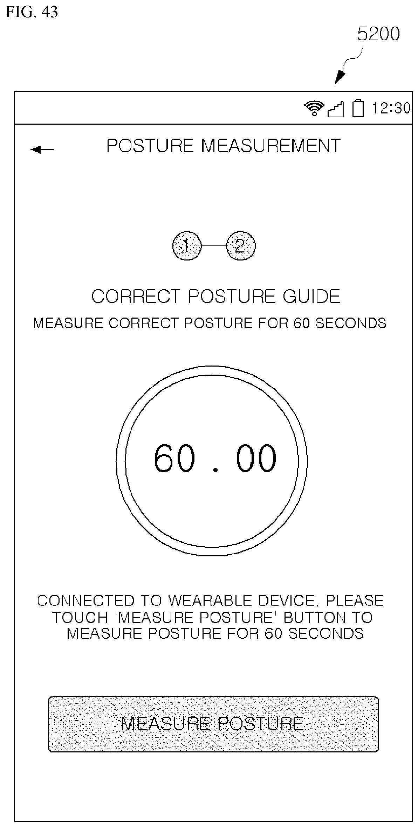

Then, when the user presses a 'next step' button, the patient terminal 5200 may start the posture measurement. At this time, the processor 5240 receives data collected by the wearable measuring device 5100 by measuring the patient for a pre-set period of time, through the second SWC unit 5210 connected to the first SWC unit 5150 of the wearable measuring device 5100 in via SWC. The posture measurement is performed on a screen of the patient terminal 5200 of FIG. 43 for 60 seconds, and the patient terminal 5200 receives raw data (for example, an acceleration value output by an acceleration sensor) for generating the trunk movement data of the patient from the wearable measuring device 5100.

According to an embodiment, the first SWC unit 5150 of the wearable measuring device 5100 and the second SWC unit 5210 of the patient terminal 5200 may be pre-connected via pairing.

Then, the processor 5240 may generate, based on the raw data received from the wearable measuring device 5100, the trunk movement data, such as the chest displacement vector, the lung capacity data, the chest asymmetry data, the waist portion displacement vector, the waist portion asymmetry data, and the trunk balance data.

Next, the processor 5240 invokes the spine health state data corresponding to the trunk movement data in preparation for the spine health state instruction data stored in the memory unit 5230.

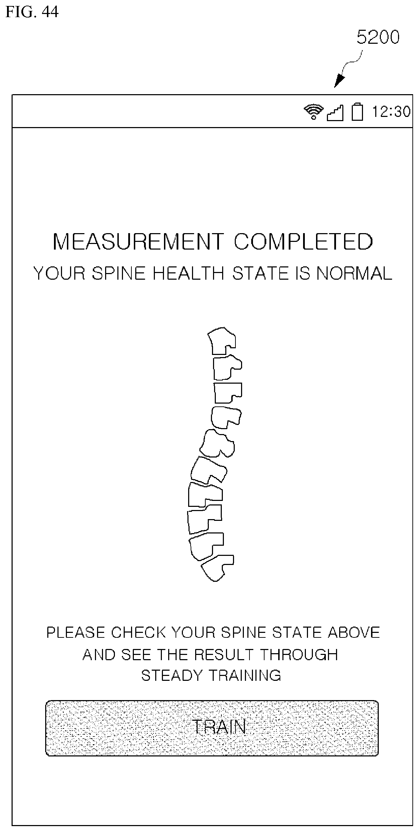

Then, as shown in FIG. 44, the patient terminal 5200 may display the spine health state data on the display unit 5250 and provide the spine health state data to the user.

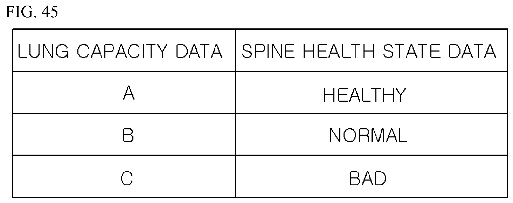

FIG. 45 illustrates the spine health state instruction data stored in the memory unit 5230, according to an embodiment.

According to an embodiment, the spine health state instruction data may include the trunk movement data and the spine health state data matched to the trunk movement data.

For example, as shown in FIG. 45, the spine health state instruction data stored in the memory unit 5230 may include a column of the lung capacity data, i.e., the trunk movement data, and a column of the spine health state data matched to the lung capacity data.

In FIG. 45, the lung capacity data is displayed in A, B, and C, but may be, in practice, defined as a lowest value and a highest value indicating a size range of a vector. The lung capacity data may be matched to any one of healthy, normal, and bad indicated by the spine health state data to configure the spine health state instruction data.

When the lung capacity data generated by measuring the posture of the patient in the screen shown in FIG. 43 belongs to 'B' in FIG. 45, the patient terminal 5200 may display 'Normal' as the spine health state data as shown in FIG. 44.

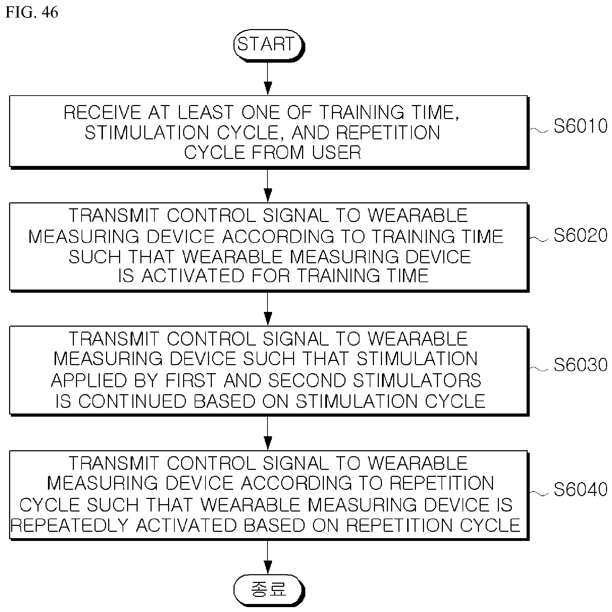

FIG. 46 is a flowchart of a method, performed by the patient terminal 5200, of controlling the wearable measuring device 5100, according to an embodiment.

Referring to FIG. 46, the input unit 5220 receives at least one of a training time, a stimulation cycle, and a repetition cycle from the user, in operation S6010. Then, the processor 5240 transmits a control signal to the wearable measuring device 5100 according to the training time such that the wearable measuring device 5100 is activated for the training time, in operation S6020. Also, the processor 5240 transmits a control signal to the wearable measuring device 5100 such that duration of stimulation applied by the first and second stimulators 5180 and 5190 is changed according to the stimulation cycle, in operation S6030. Also, the processor 5240 transmits a control signal to the wearable measuring device 5100 according to the repetition cycle such that the wearable measuring device 5100 is repeatedly activated based on the repetition cycle, in operation S6040.

As such, the user may adjust spine diagnosis processes including the posture measurement and spine correction using the wearable measuring device 5100, by inputting, to the patient terminal 5200, various types of setting information related to driving of the wearable measuring device 5100.

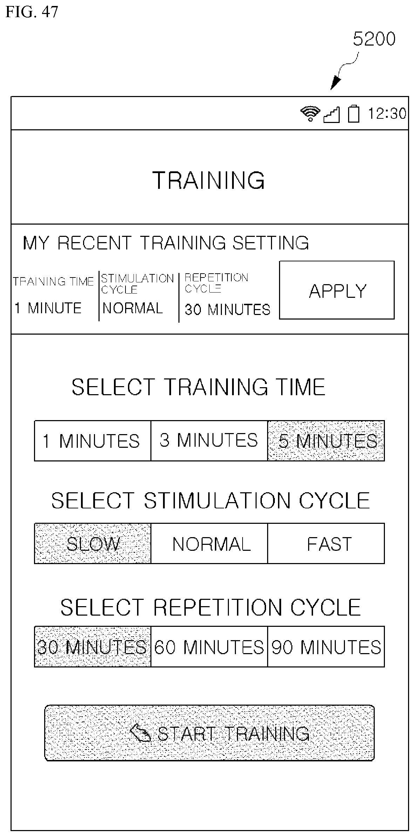

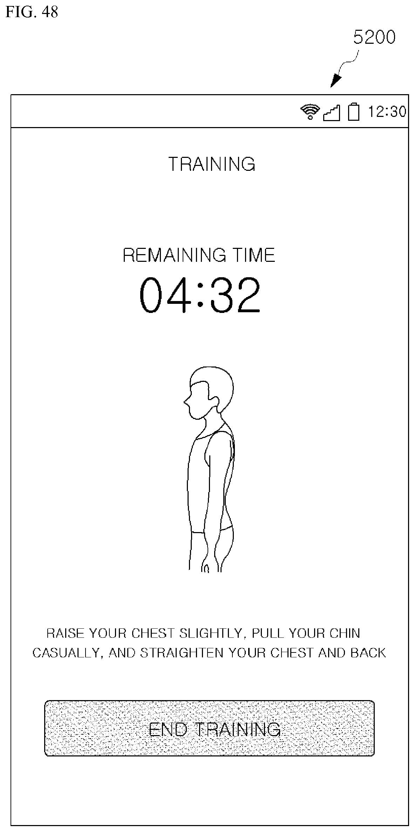

FIGS. 47 and 48 illustrate screens of the patient terminal 5200 receiving information for controlling the wearable measuring device 5100 from the patient, according to an embodiment.

As shown in FIG. 47, according to an embodiment, the patient terminal 5200 may provide a screen for receiving setting information related to driving of the wearable measuring device 5100 through the display unit 5250.

The training time indicates a period of time where the wearable measuring device 5100 collects data by measuring the patient. In FIG. 47, the user may select one of 1 minute, 3 minutes, and 5 minutes as the training time to adjust a driving time of the wearable measuring device 5100.

The stimulation cycle indicates duration of the first and second stimulators 5180 and 5190 of the wearable measuring device 5100 stimulating a body part of the patient. When the stimulation cycle is slow, the duration of stimulation applied to the body part is long.

On the other hand, when the stimulation cycle is fast, the duration of stimulation applied to the body part is short. In FIG. 47, the user may select one of 'slow', 'normal', and 'fast' as the stimulation cycle to adjust the duration of stimulation provided by the wearable measuring device 5100 to the patient.

The repetition cycle indicates a cycle where the wearable measuring device 5100 is driven. The wearable measuring device 5100 is repeatedly driven based on the repetition cycle as the wearable measuring device 5100 is continuously driven for the training time. In FIG. 47, the user may select any one of 30 minutes, 60 minutes, and 90 minutes as the repetition cycle to adjust the repetition cycle in which the wearable measuring device 5100 is repeatedly driven.

The training time, the stimulation cycle, and the repetition cycle shown in FIG. 47 are only examples, and may vary according to an embodiment.

As shown in FIG. 47, when the setting information related to driving of the wearable measuring device 5100 is input, the patient terminal 5200 may transmit a control signal to the wearable measuring device 5100 via SWC.

Also, as shown in FIG. 48, the patient terminal 5200 may receive data from the wearable measuring device 5100 by driving the wearable measuring device 5100 according to the setting information input by the user, and generate the trunk movement data based on the received data, thereby controlling the wearable measuring device 5100 to stimulate the body part of the patient.

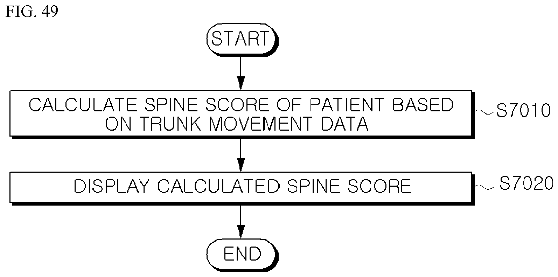

FIG. 49 is a flowchart of a method of calculating a spine score of the patient, according to an embodiment.

The patient terminal 5200 may calculate the spine score of the patient based on the trunk movement data of the patient, and provide the spine score to the user.

Referring to FIG. 49, the processor 5240 calculates the spine score based on the trunk movement data in operation S7010, and the display unit 5250 displays the calculated spine score in operation S7020.

According to an embodiment, the spine score may be calculated based on the lung capacity data and the trunk asymmetry data. In detail, the processor 5240 may calculate the spine score such that the spine score is high when the lung capacity data is large and the spine score is high when the trunk asymmetry data is small.

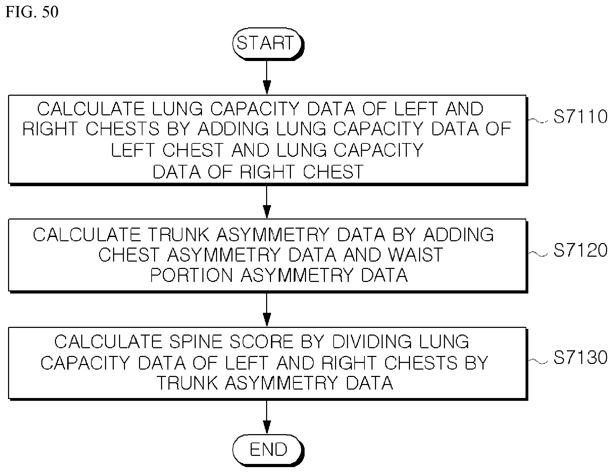

FIG. 50 is a flowchart of a method of calculating the spine score of the patient based on the lung capacity data and the trunk asymmetry data of the patient, according to an embodiment.

Referring to FIG. 50, the processor 5240 may calculate the lung capacity data of the left and right chests by adding the lung capacity data of the left chest and the long capacity data of the right chest, in operation S7110. Then, the processor 5240 may calculate the trunk asymmetry data by adding the chest asymmetry data and the waist portion asymmetry data, in operation S7120. Then, the processor 5240 may calculate the spine score by dividing the lung capacity data of the left and right chests by the trunk asymmetry data, in operation S7130.

As such, the patient terminal 5200 may generate the trunk movement data of the patient based on measurement data of the patient received from the wearable measuring device 5100 and calculate the spine score based on the trunk movement data, wherein the spine score may be proportional to the lung capacity data and inversely proportional to the trunk asymmetry data.

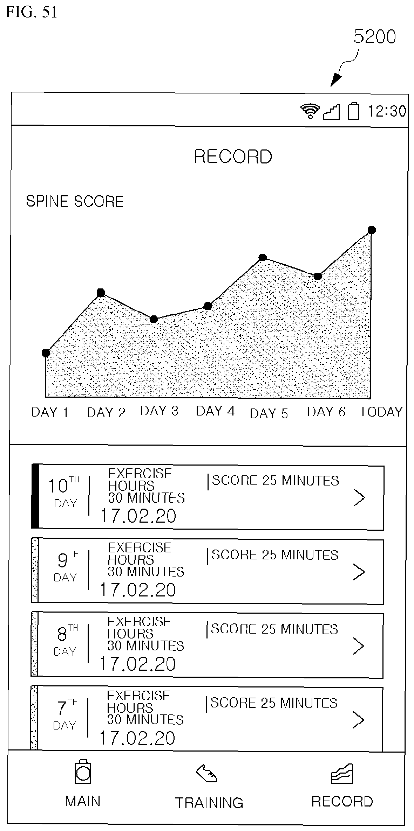

FIG. 51 illustrates a screen in which the calculated spine score is provided to the patient, according to an embodiment.

According to an embodiment, the patient terminal 5200 may calculate the spine score based on the trunk movement data of the patient, and store the spine score in the memory unit 5230.

In addition, the patient terminal 5200 may display and provide, to the user, a graph of a change of the spine score according to time, by repeatedly calculating the spine score.

For example, as shown in FIG. 51, the patient termianl5 200 may calculate and store the spine score of the patient every day, and generate and display, on the screen, a spine score graph in which a horizontal axis indicates time and a vertical axis indicates a spine score.

In the embodiments described above, the wearable measuring device 5100 transmits the raw data, such as an acceleration value, to the patient terminal 5200 by measuring the patient, the patient terminal 5200 generates the trunk movement data of the patient, such as a displacement vector, based on the raw data, and the system for remotely diagnosing spine 5300 generates the diagnostic data, such as the lung capacity data, the trunk asymmetry data, and the left or right trunk balance data, by receiving the displacement vector from the patient terminal 5200, but the present disclosure is not limited thereto.

For example, the wearable measuring device 5100 may further generate the trunk movement data, such as the displacement vector, by processing the raw data obtained by measuring the patient, and in addition, further generate the diagnostic data, such as the lung capacity data, the trunk asymmetry data, and the left or right trunk balance data, based on the displacement vector.

Similarly, the patient terminal 5200 may generate not only the trunk movement data, such as the displacement vector, but also the diagnostic data, such as the lung capacity data, the trunk asymmetry data, and the left or right trunk balance data, upon receiving the raw data, such as an acceleration value, from the wearable measuring device 5100.

Also, the system for remotely diagnosing spine 5300 may directly generate the trunk movement data, such as the displacement vector, upon receiving the raw data, such as an acceleration value, collected by the wearable measuring device 5100, through the patient terminal 5200.

In other words, the trunk movement data and the diagnostic data may be generated by any one of the wearable measuring device 5100, the patient terminal 5200, and the system for remotely diagnosing spine 5300.

Processes for operations ofthe wearable measuring device 5100, the patient terminal 5200, and the system for remotely diagnosing spine 5300, according to embodiments may be embodied as computer-executable programs on a computer-readable recording medium. The computer-readable recording medium is any data storage device that can store data which can be thereafter read by a computer system. Examples of the computer-readable recording medium include ROM, RAM, CD-ROMs, magnetic tapes, floppy disks, optical data storage devices, etc. Alternatively, the processes may be embodied as computer programs stored in a medium to be combined to and executed by a computer.

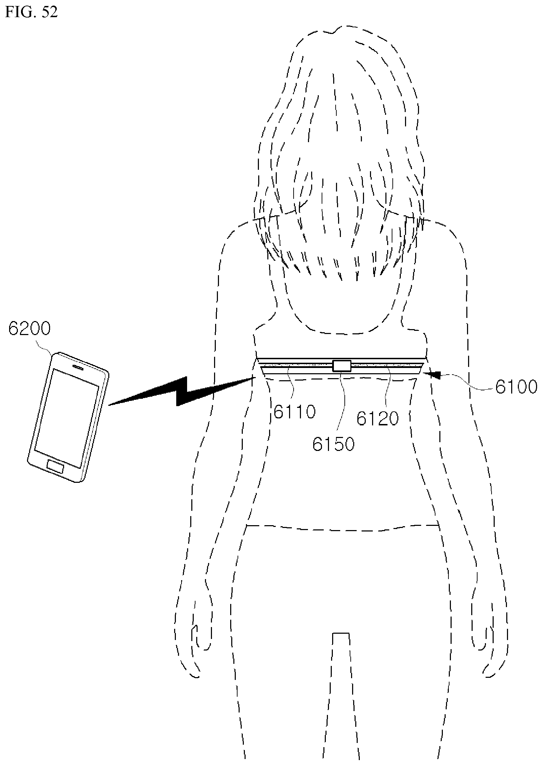

Here, after, a wearable measuring device 6100 according to another embodiment will be described with reference to FIGS. 52 through 59.

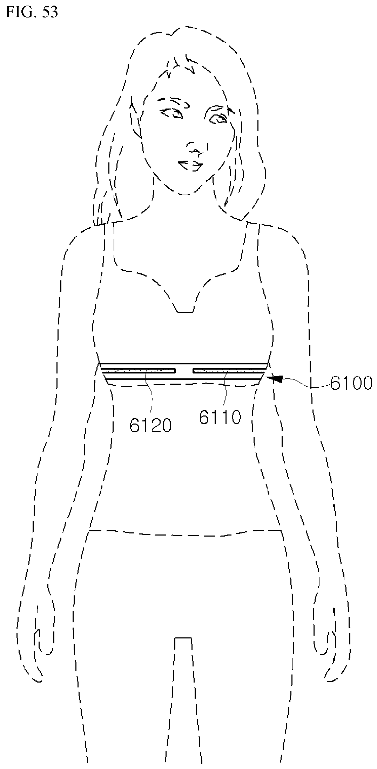

FIGS. 52 and 53 are respectively a rear view and a front view of a patient wearing the wearable measuring device 6100, according to an embodiment.

The wearable measuring device 6100 includes a first stretch sensor unit 6110, a second stretch sensor unit 6120, and a first SWC unit 6150.

The first stretch sensor unit 6110 detects movement of a left trunk of the patient. The second stretch sensor unit 6120 detects movement of a right trunk of the patient.

The first SWC unit 6150 transmits data output from the first and second stretch sensor units 6110 and 6120 to a patient terminal 6200.

According to an embodiment, the first stretch sensor unit 6110 is provided at a portion of an object worn on the patient, which interacts with the left trunk, and the second stretch sensor unit 6120 is provided at a portion of the object worn on the patient, which interacts with the right trunk.

For example, the first and second stretch sensor units 6110 and 6120 may be provided at a chest band surrounding the chest of the patient on a top worn by the patient. As shown in FIGS. 52 and 53, the first and second stretch sensor units 6110 and 6120 may be formed of a stretchable material, and provided at a band portion of underwear or sportswear top worn on the chest of a female.

As such, the first and second stretch sensor units 6110 and 6120 may be integrated into the object worn by the patient, but alternatively, may be provided to be replaceable. For example, the first and second stretch sensor units 6110 and 6120 formed of fabric may be provided to be replaceable in clothing worn by the patient.

In the current embodiment, the patient wearing the wearable measuring device 6100 is not limited to a female, and the first and second stretch sensor units 6110 and 6120 may be provided at a portion of the top of a male surrounding the chest. In addition, the wearable measuring device 6100 does not necessarily have to be clothing, and may be embodied in any one of various objects, such as a gear and an accessory, as long as the first and second stretch sensor units 6110 and 6120 detect the movements of the left and right trunks by being provided at portions respectively interacting with the left and right trunks of the patient.

The first stretch sensor unit 6110 may output, to the first SWC unit 6150, first stretching amount data indicating a left chest stretching mount according to the movement of the left chest of the patient. Also, the second stretch sensor unit 6120 may output, to the first SWC unit 6150, second stretching amount data indicating a right chest stretching amount according to the movement of the right chest of the patient.

For example, the first and second stretch sensor units 6110 and 6120 may output an electric signal according to a resistance value of a sensor, which changes according to expansion or contraction. Here, the left chest stretching amount of the patient may be a changed resistance value of the first stretch sensor unit 6110, and the right chest stretching amount may be a changed resistance value of the second stretch sensor unit 6120. However, based on types of the first and second stretch sensor units 6110 and 6120, the left and right chest stretching amounts may be indicated by another type of physical amount other than a resistance value of a sensor.

The first SWC unit 6150 exchanges data with the patient terminal 6200 via SWC. For example, the first SWC unit 6150 may exchange data with the patient terminal 6200 via Bluetooth. However, an SWC protocol used by the first SWC unit 6150 and the patient terminal 6200 to exchange data is not limited to a Bluetooth protocol.

The patient terminal 6220 may obtain the left chest stretching amount based on the first stretching amount data, and obtain the right chest stretching amount based on the second stretching amount data. According to an embodiment, the patient terminal 6200 may be a mobile terminal, such as a smart device, and process data by executing an application, by including a processor, such as an AP, and a memory.

According to an embodiment, the wearable measuring device 6100 may further include a first stimulator and a second stimulator. The first stimulator stimulates a left body part of the patient, and the second stimulator stimulates a right body part of the patient.

The first and second stimulators may include a vibrator generating vibration. However, the configuration of the first and second stimulators is not limited thereto, and the first and second stimulators may include, in addition to the vibrator, various devices capable of stimulating a body part of the patient, such as an electrode applying electric stimulation.

Although not shown in FIGS. 52 and 53, like the first and second stretch sensor units 6110 and 6120, the first and second stimulators may also be provided at portions of the object worn by the patient, which respectively interact with the left and right trunks of the patient.

For example, the first and second stimulators may be provided at the chest band surrounding the chest on the top worn by the patient. However, the first and second stimulators may be provided at other objects, aside from the chest band of the top, as long as the first and second stimulators stimulate the left and right body parts of the patient.

Also, the first and second stimulators do not necessarily need to be provided at the same object as the first and second stretch sensor units 6110 and 6120, and according to an embodiment, the first and second stretch sensor units 6110 and 6120 may be provided at the top and the first and second stimulators may be embodied as separate accessories.

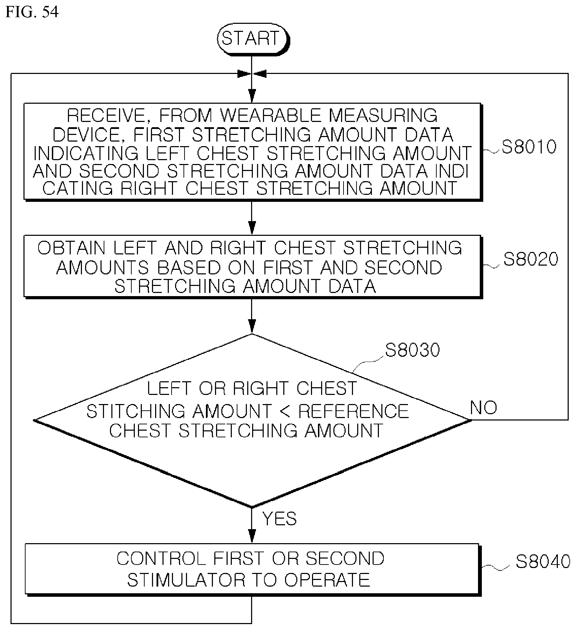

FIG. 54 is a flowchart of a spine diagnosing method performed by the patient terminal 6100, according to an embodiment.

According to an embodiment, the patient terminal 6200 receives, from the wearable measuring device 6100, the first stretching amount data indicating the left chest stretching amount and the second stretching amount data indicating the right chest stretching amount in operation S8010, obtains the left and right chest stretching amounts based on the first and second stretching amount data in operation S8020, and when it is determined that the left chest stretching amount is smaller than a pre-set reference chest stretching amount in operation S8030, controls the first stimulator to operate in operation S8040, and when it is determined that the right chest stretching amount is smaller than the pre-set reference chest stretching amount in operation S8030, controls the second stimulator to operate in operation S8040.

According to the current embodiment, the patient wearing the wearable measuring device 6100 may consciously and actively move one of the left and right chests, which has low stretching amount due to abnormal bending of the spine, by recognizing the corresponding chest through stimulation. Accordingly, the patient may obtain a treatment effect of correcting the spine by training inactivated muscles of the corresponding chest.

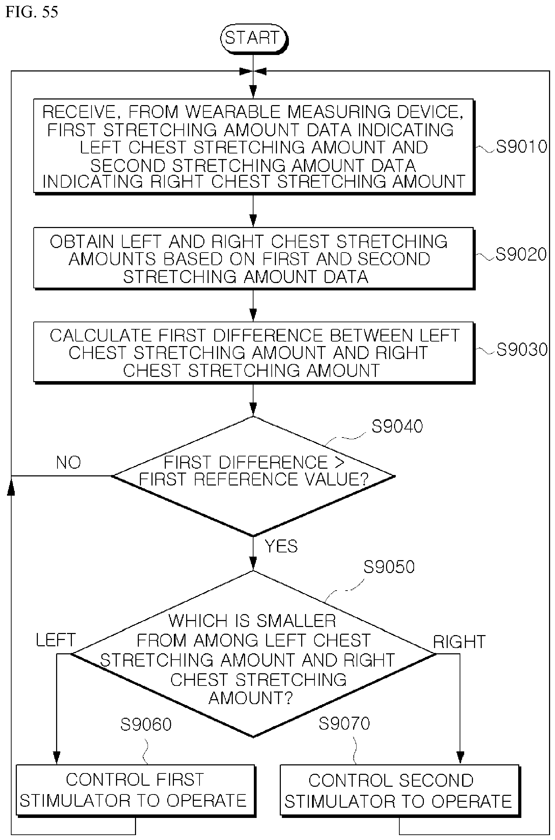

FIG. 55 is a flowchart of a spine diagnosing method performed by the patient terminal 6200, according to another embodiment.

According to an embodiment, the patient terminal 6200 receives, from the wearable measuring device 6100, the first stretching amount data indicating the left chest stretching amount of the patient and the second stretching amount data indicating the right chest stretching amount of the patient in operation S9010, obtains the left and right chest stretching amounts based on the first and second stretching amount data in operation S9020, calculates a first difference between the left chest stretching amount and the right chest stretching amount in operation S9030, and when it is determined that the calculated first difference is greater than a pre-set first reference value in operation S9040 and that the left chest stretching amount is smaller than the right chest stretching amount in operation S9050, controls the first stimulator to operate in operation S9060, and when it is determined that the calculated first difference is greater than the pre-set first reference value in operation S9040 and that the right chest stretching amount is smaller than the left chest stretching amount in operation S9050, controls the second stimulator to operate in operation S9070.

According to the current embodiment, the patient wearing the wearable measuring device 6100 may consciously and actively move one of the left and right chests, which moves less during respiration, by recognizing the corresponding chest through stimulation when the left and right chests are severely asymmetric due to abnormal bending of the spine. Accordingly, the patient may obtain a treatment effect of correcting the spine by training inactivated muscles of the corresponding chest.

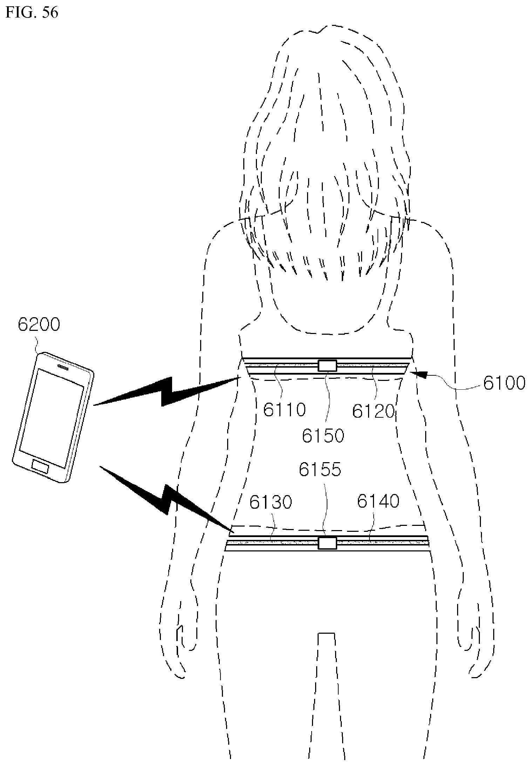

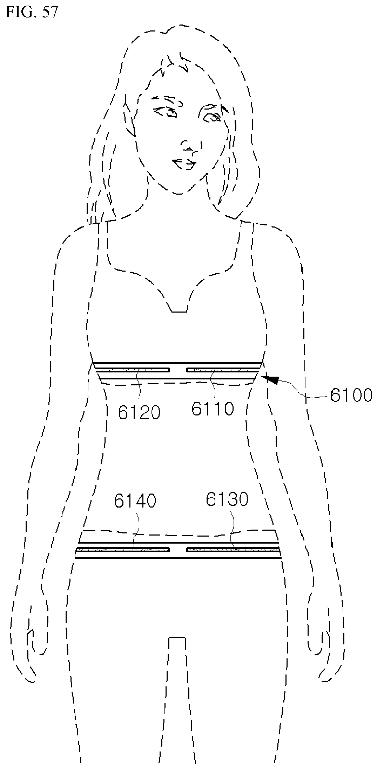

FIGS. 56 and 57 are respectively a rear view and a front view of a patient wearing the wearable measuring device 6100, according to another embodiment.

According to an embodiment, the wearable device 6100 may further include a third stretch sensor unit 6130, a fourth stretch sensor unit 6140, and an auxiliary SWC unit 6155.

The third stretch sensor unit 6130 detects movement of a left waist portion of the patient. The fourth stretch sensor unit 6140 detects movement of a right waist portion of the patient. The auxiliary SWC unit 6155 transmits data output from the third and fourth stretch sensor units 6130 and 6140 to the patient terminal 6200.

According to an embodiment, the third stretch sensor unit 6130 may be provided at a portion of an object worn by the patient, which interacts with the left waist portion, and the fourth stretch sensor unit 6150 may be provided at a portion of the object worn by the patient, which interacts with the right waist portion.

For example, the third and fourth stretch sensor units 6130 and 6140 may be provided at a waist band surrounding a waist portion on clothing worn by the patient. As shown in FIGS. 56 and 57, the third and fourth stretch sensor units 6130 and 6140 may be formed of a stretchable material, and provided at a waist band portion of underwear or sportswear bottom.

As such, the third and fourth stretch sensor units 6130 and 6140 may be integrated into the object worn by the patient, but alternatively, may be provided to be replaceable. For example, the third and fourth stretch sensor units 6130 and 6140 formed of fabric may be provided to be replaceable in clothing worn by the patient.

In addition, the wearable measuring device 6100 may be embodied in any one of various objects, such as a gear and an accessory, as long as the third and fourth stretch sensor units 6130 and 6140 detect the movements of the left and right waist portions by being provided at portions respectively interacting with the left and right waist portions of the patient.

The third stretch sensor unit 6130 may output, to the auxiliary SWC unit 6155, third stretching amount data indicating a left waist portion stretching amount according to movement of the left waist portion of the patient. Also, the fourth stretch sensor unit 6140 may output, to the auxiliary SWC unit 6155, fourth stretching amount data indicating a right waist portion stretching amount according to movement of the right waist portion of the patient.

For example, the third and fourth stretch sensor units 6130 and 6140 may output an electric signal according to a resistance value of a sensor, which changes according to expansion or contraction. Here, the left waist portion stretching amount of the patient may be a changed resistance value of the third stretch sensor unit 6130, and the right waist portion stretching amount may be a changed resistance value of the fourth stretch sensor unit 6140. However, based on types of the third and fourth stretch sensor units 6130 and 6140, the left and right waist portion stretching amounts may be indicated by another type of physical amount other than a resistance value of a sensor.

The auxiliary SWC unit 6155 exchanges data with the patient terminal 6200 via SWC. For example, the auxiliary SWC unit 6155 may exchange data with the patient terminal 6200 via Bluetooth. However, an SWC protocol used by the auxiliary SWC unit 6155 and the patient terminal 6200 to exchange data is not limited to a Bluetooth protocol.

The patient terminal 6220 may obtain the left waist portion stretching amount based on the third stretching amount data, and obtain the right waist portion stretching amount based on the fourth stretching amount data.

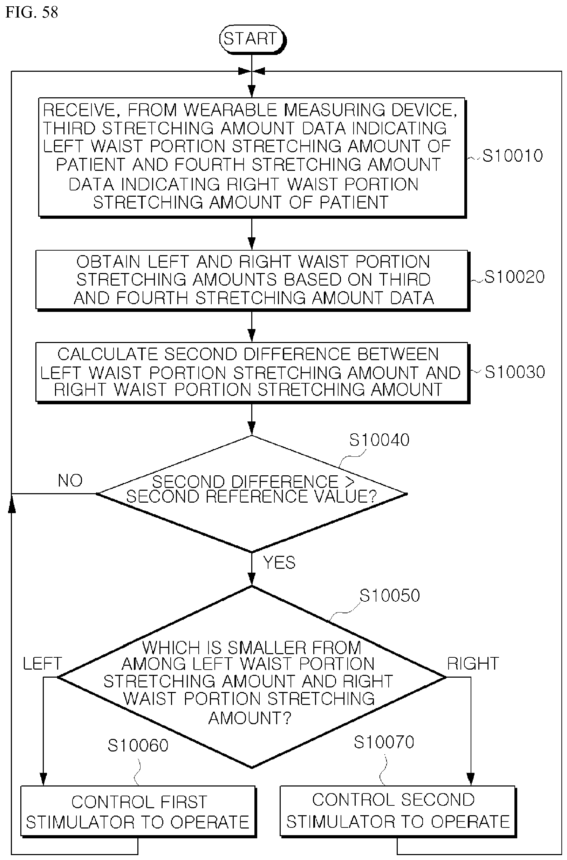

FIG. 58 is a flowchart of a spine diagnosing method performed by the patient terminal 6200, according to another embodiment.

According to an embodiment, the patient terminal 6200 receives, from the wearable measuring device 6100, the third stretching amount data indicating the left waist portion stretching amount of the patient and the fourth stretching amount data indicating the right waist portion stretching amount of the patient, in operation S10010, obtains the left and right waist portion stretching amounts based on the third and fourth stretching amount data in operation S10020, calculates a second difference between the left waist portion stretching amount and the right waist portion stretching amount in operation S10030, and when it is determined that the calculated second difference is greater than a pre-set second reference value in operation S10040 and that the left waist portion stretching amount is smaller than the right waist portion stretching amount in operation S10050, controls the first stimulator to operate in operation S10060, and when it is determined that the calculated second difference is greater than the pre-set second reference value in operation S10040 and that the right waist portion stretching amount is smaller than the left waist portion stretching amount in operation S10050, controls the second stimulator to operate in operation S10070.

According to the current embodiment, the patient wearing the wearable measuring device 6100 may consciously and actively move one of the left and right waist portions, which moves less, by recognizing the corresponding waist portion through stimulation when the left and right waist portions are severely asymmetric due to abnormal bending of the spine. Accordingly, the patient may obtain a treatment effect of correcting the spine by training inactivated muscles of the corresponding waist portion.

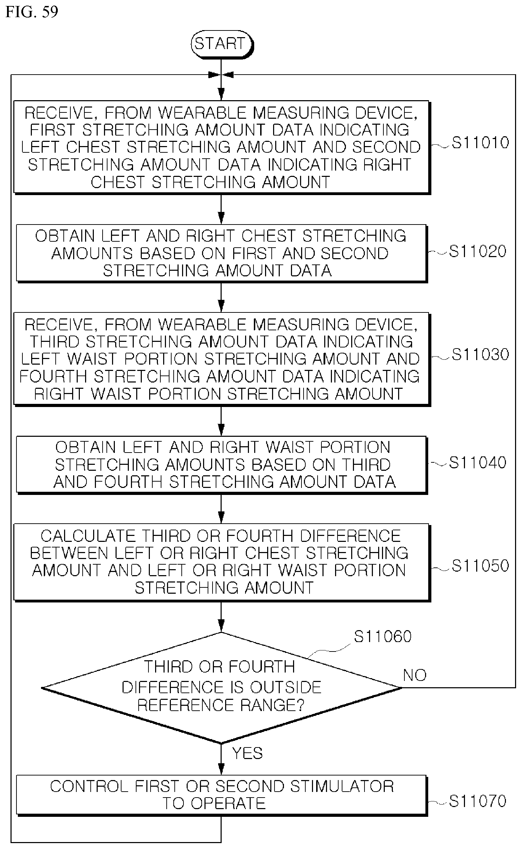

FIG. 59 is a flowchart of a spine diagnosing method performed by the patient terminal 6200, according to another embodiment.

According to an embodiment, the patient terminal 6200 receives, from the wearable measuring device 6100, the first stretching amount data indicating the left chest stretching amount and the second stretching amount data indicating the right chest stretching amount in operation S11010, obtaining the left and right chest stretching amounts based on the first and second stretching amount data in operation S11020, receiving, from the wearable measuring device 6100, the third stretching amount data indicating the left waist portion stretching amount and the fourth stretching amount data indicating the right waist portion stretching amount in operation S11030, obtaining the left and right waist portion stretching amounts based on the third and fourth stretching amount data in operation S11040, calculating a third difference between the left chest stretching amount and the left waist portion stretching amount in operation S11050, controlling the first stimulator to operate in operation S11070 when it is determined that the calculated third difference is outside a pre-set reference range in operation S11060, calculating a fourth difference between the right chest stretching amount and the right waist portion stretching amount in operation S11050, and controlling the second stimulator to operate in operation S11070 when it is determined that the calculated fourth difference is outside the pre-set reference range in operation S11060.

In the current embodiment, unlike the left or right chest stretching mount, the first difference between the left and right chest stretching amounts, and the second difference between the left and right waist portion stretching amounts, abnormality of the spine may be diagnosed by indicating a movement relationship between one side chest and one side waist portion as the third or fourth difference between a chest stretching amount of the one side chest and a waist portion stretching amount of the one side waist portion, based on stretching amounts of the one side chest and the one side waist portion, and comparing the third or fourth difference with a reference range.

According to the current embodiment, the patient wearing the wearable measuring device 6100 may consciously and actively move a chest and waist portion of one of the left and right trunks, in which movement at the chest and waist portion is outside a normal range due to abnormal bending of the spine, by recognizing the corresponding trunk through stimulation. Accordingly, the patient may obtain a treatment effect of correcting the spine by training inactivated muscles of the chest or waist portion of the corresponding trunk

The spine diagnosing method according to the embodiments may be embodied as an application stored in a medium to be executed by a mobile terminal. A computer-readable recording medium is any data storage device that can store data which can be thereafter read by a computer system. Examples of the computer-readable recording medium include ROM, RAM, CD-ROMs, magnetic tapes, floppy disks, optical data storage devices, etc.

While one or more embodiments have been described with reference to the figures, it will be understood by those of ordinary skill in the art that various changes in form and details may be made therein without departing from the spirit and scope of the disclosure as defined by the following claims.

## Claims

1. A chest measuring device comprising:
first and second sensor units respectively comprising sensors detecting movement of left and right chests of a subject;
a detaching unit configured to attach or detach the first and second sensor units to or from a chest of the subject; and
a control unit configured to receive data measured by the sensor of the first sensor unit and the sensor of the second sensor unit,
wherein the control unit comprises:
a first sensor unit processor configured to detect inhalation volume information of the left chest by analyzing data of the first sensor unit;
a second sensor unit processor configured to detect inhalation volume information of the right chest by analyzing data of the second sensor unit; and
a vibration determiner configured to calculate a difference between the inhalation volume information of the left chest detected by the first sensor unit processor and the inhalation volume information of the right chest detected by the second sensor unit processor, compare the calculated difference with a second threshold value that is defined as a largest difference between the inhalation volume information of the left and right chests during normal respiration, and determine that respiration is not normal due to inactivated a muscle of the chest caused by abnormal alignment of a spine when the difference is equal to or greater than the second threshold value.

2. The chest measuring device of claim 1, wherein the vibration determiner further comprises:
a left data detecting module configured to detect left data comprising the inhalation volume information of the lest chest by analyzing data input from the first sensor unit;
a right data detecting module configured to detect right data comprising the inhalation volume information of the right chest by analyzing data input from the second sensor unit; and
a third determining module configured to calculate the difference between the inhalation volume information of the left chest detected by the left data detecting module and the inhalation volume information of the right chest detected by the right data detecting module, compare the calculated difference with the second threshold value, and determine that the alignment of the spine is abnormal when the difference is equal to or greater than the second threshold value.

3. The chest measuring device of claim 2, wherein the left data detecting module is configured to detect the left data further comprising exhalation volume information of the left chest and volume displacement value of the left chest during inhalation and exhalation,
the right data detecting module is configured to detect the right data further comprising exhalation volume information of the right chest and volume displacement value of the right chest during inhalation and exhalation, and
the vibration determiner further comprises:
a first determining module configured to calculate a volume displacement value that is a difference between the inhalation volume information and the exhalation volume information of the left chest of the left data, compare the calculated volume displacement value with a first threshold value defined as a smallest value of a volume displacement value of inhalation and exhalation of the chest of which respiration is determined to be normal, and determine that respiration is not normal due to inactivated a muscle ofthe left chest caused by abnormal alignment of the spine when the calculated volume displacement value is smaller than the first threshold value; and
a second determining module configured to calculate a volume displacement value that is a difference between the inhalation volume information and the exhalation volume information of the right chest of the right data, compare the calculated volume displacement value with the first threshold value, and determine that respiration is not normal due to inactivated a muscle of the right chest caused by abnormal alignment of the spine when the calculated volume displacement value is smaller than the first threshold value.

4. The chest measuring device of claim 3, wherein the first and second sensor units each comprise:
a gyrosensor configured to detect angular speeds of X-, Y-, and Z-axes that are perpendicular to each other;
an acceleration sensor configured to detect acceleration of the X-, Y-, and Z-axes that are perpendicular to each other; and
a geomagnetic sensor.

5. The chest measuring device of claim 3 or 4, wherein the first and second sensor units each comprise a vibrator,
wherein the control unit is configured to output control data for vibrating the vibrator of the first sensor unit when the first determining module determines that respiration is not normal, output control data for vibrating the vibrator of the second sensor unit when the second determining module determines that respiration is not normal, and output control data to one of the first and second sensor units, which corresponds to a chest having smaller inhalation volume information when the third determining module determines that respiration is not normal, and
the first and second sensor units each operate the vibrators when the control data is received from the control unit.

6. The chest measuring device of claim 5, wherein the first and second sensor units further comprise pressurizing units configured to selectively raise or lower the vibrators of the first and second sensor units,
wherein the control unit comprises a raised height detector driven when the vibration determiner determines that respiration is not normal and configured to detect a raised height corresponding to exhalation volume information of a chest of which respiration is determined to be abnormal by the vibration determiner by searching a reference table in which raised height of the pressurizing unit are matched per exhalation volume information of the chest, and
the first and second sensor units vibrate the vibrators after controlling the pressurizing units according to the raised height received from the control unit.

7. A scoliosis correction system comprising:
a wearable internet of things (IoT) comprising at least one sensor unit that comprises a sensor and a vibrator, the sensor contacting a body of a subject and detecting movement of a muscle of the contacted body, a detaching unit configured to attach or detach the at least one sensor unit to or from the body of the subject, and a control unit configured to externally transmit a sensing value when the sensing value measured by the sensor of the at least one sensor unit is received; and
a portable terminal in which a spine management application analyzing the sensing value received from the wearable IoT is installed,
wherein the spine management application determines whether the muscle needs to be vibrated by analyzing the sensing value when the sensing value is received from the wearable IoT, and when it is determined that the muscle needs vibration, transmits vibration information to the wearable IoT by controlling the portable terminal.

8. The scoliosis correction system of claim 7, wherein the spine management application comprises:
a data analyzer configured to analyze the sensing value received from the at least one sensing unit via a pre-set analysis algorithm, and detect a motion vector of the muscle corresponding to a location where the at least one sensor unit is attached; and
a vibration determiner configured to detect activity of the muscle by analyzing the motion vector detected by the data analyzer via a pre-set activity detection algorithm, compare the detected activity with a pre-set threshold value defined as a smallest value of muscle activity in which respiration is determined to be normal or the muscle is determined to be activated, and determine that the muscle needs to be vibrated when the detected activity is smaller than the pre-set threshold value,
wherein the spine management application transmits the vibration information to the wearable IoT by controlling the portable terminal when the vibration determiner determines that the muscle needs to be vibrated, and the wearable IoT is configured to drive the vibrator of the at least one sensor unit when the vibration information is received from the portable terminal.

9. The scoliosis correction system of claim 8, wherein the at least one sensor unit further comprises a pressurizing unit configured to selectively raise or lower the vibrator,
the spine management application further comprises a vibration information detector configured to be driven when the vibration determiner determines that the muscle needs to be vibrated, analyze the motion vector of the muscle detected by the data analyzer via a pre-set vibration intensity and height detection algorithm, and generate vibration information comprising optimum vibration intensity and an optimum raised height of the vibrator, which corresponds to the motion vector, and
the wearable IoT is further configured to enable the pressurizing unit to adjust a length of the vibrator based on the optimum raised height of the vibration information received from the spine management application, and vibrate the vibrator according to the optimum vibration intensity of the received vibration information.

10. The scoliosis correction system of claim 9, wherein the at least one sensor unit comprises:
a first sensor unit contacting the back of the subject corresponding to left ribs;
a second sensor unit contacting the back of the subject corresponding to right ribs;
a third sensor unit contacting the back of the subject corresponding to left transverse processes of lumbar vertebrae; and
a fourth sensor unit contacting the back of the subject corresponding to right transverse processes of lumbar vertebrae,
the data analyzer further comprises:
a first data detection module configured to analyze a sensing value measured by the first sensor unit;
a second data detection module configured to analyze a sensing value measured by the second sensor unit;
a third data detection module configured to analyze a sensing value measured by the third sensor unit; and
a fourth data detection module configured to analyze a sensing value measured by the fourth sensor unit,
the vibration determiner is further configured to determine vibration of the first through fourth sensor units,
the vibration information detector is further configured to detect vibration information with respect to a sensor unit determined that vibration is needed by the vibration determiner, and
the wearable IoT is further configured to drive a vibrator of the sensor unit when the vibration information is received.

11. The scoliosis correction system of claim 10, wherein the first data detection module is further configured to analyze the sensing value measured by the first sensor unit via the pre-set analysis algorithm and detect first inhalation detailed information indicating a motion vector of a muscle corresponding to the left ribs during inhalation, first exhalation detailed information indicating a motion vector of the muscle corresponding to the left ribs during exhalation, and first displacement information indicating a displacement vector of the first inhalation detailed information and the first exhalation detailed information,
the second data detection module is further configured to analyze the sensing value measured by the second sensor unit via the pre-set analysis algorithm and detect second inhalation detailed information indicating a motion vector of a muscle corresponding to the right ribs during inhalation, second exhalation detailed information indicating a motion vector of the muscle corresponding to the right ribs during exhalation, and second displacement information indicating a displacement vector of the second inhalation detailed information and the second exhalation detailed information,
the third data detection module is further configured to analyze the sensing value measured by the third sensor unit via the pre-set analysis algorithm and detect third inhalation detailed information indicating a motion vector of a muscle corresponding to the left transverse processes of lumbar vertebrae during inhalation, third exhalation detailed information indicating a motion vector of the muscle corresponding to the left transverse processes of lumbar vertebrae during exhalation, and third displacement information indicating a motion vector of the third inhalation detailed information and the third exhalation detailed information, and
the fourth data detection module is further configured to analyze the sensing value measured by the fourth sensor unit via the pre-set analysis algorithm and detect fourth inhalation detailed information indicating a motion vector of a muscle corresponding to the right transverse processes of lumbar vertebrae during inhalation, fourth exhalation detailed information indicating a motion vector of the muscle corresponding to the right transverse processes of lumbar vertebrae during exhalation, and fourth displacement information indicating a displacement vector of the fourth inhalation detailed information and the fourth exhalation detailed information.

12. The scoliosis correction system of claim 11, wherein the first through fourth sensor units each comprise:
a gyrosensor configured to detect an angular speed of X-, Y-, and Z-axes perpendicular to each other;
an acceleration sensor configured to detect acceleration of the X-, Y-, and Z-axes perpendicular to each other; and
a geomagnetic sensor.

13. The scoliosis correction system of claim 11, further comprising a management server configured to, upon receiving information about the motion vectors of the first through fourth sensor units from the spine management application, detect a respiration pattern and a spinal alignment state of the subject by analyzing the received motion vectors of the muscles via a pre-set respiration pattern and spinal alignment state detection algorithm, detect optimum rotational angular breathing and an optimum training posture corresponding to the detected respiration pattern and spinal alignment state by searching a reference table in which rotational angular breathing and training postures are matched per respiration pattern and spinal alignment state, and transmit information about the detected optimum rotational angular breathing and training posture to the spine management application,
wherein the spine management application displays, on a monitor of the portable terminal, the optimum rotational angular breathing and training posture received from the management server.

14. A system for remotely diagnosing spine comprising:
a patient interface unit configured to receive trunk movement data of a patient, which is generated when a wearable measuring device measures the patient, from a patient terminal;
a database unit storing the trunk movement data;
a diagnostic data generator configured to generate diagnostic data about the patient based on the trunk movement data of the patient; and
a medical staff interface unit configured to provide the diagnostic data about the patient to a medical staff terminal.

15. The system for remotely diagnosing spine of claim 14, wherein the wearable measuring device comprises:
a first sensor unit configured to detect movement of a left chest of the patient;
a second sensor unit configured to detect movement of a right chest of the patient; and
a first near field communication unit configured to transmit data output from the first and second sensor units to the patient terminal.

16. The system for remotely diagnosing spine of claim 15, wherein the first and second sensor units each comprise an acceleration sensor configured to detect acceleration with respect to at least one axis direction.

17. The system for remotely diagnosing spine of claim 16, wherein the patient terminal is configured to generate a left chest displacement vector related to movement of the left chest of the patient based on the data output from the first sensor unit, and generate a right chest displacement vector related to movement of the right chest of the patient based on the data output from the second sensor unit.

18. The system for remotely diagnosing spine of claim 17, wherein the diagnostic data generator is further configured to generate lung capacity data related to lung capacity of the left or right chest of the patient by receiving the left chest displacement vector or the right chest displacement vector of the patient.

19. The system for remotely diagnosing spine of claim 18, wherein the diagnostic data generator is further configured to:
detect a left or right chest inhalation displacement vector corresponding to inhalation of the patient and left or right chest exhalation displacement vector corresponding to exhalation of the patient, from among the received left or right chest displacement vector,
calculate a first vector between the left or right chest inhalation displacement vector and the left or right chest exhalation displacement vector, and
output half of a size of the first vector as the lung capacity data of the left or right chest.

20. The system for remotely diagnosing spine of claim 19, wherein the wearable measuring device further comprises:
a first stimulator configured to stimulate a left body portion of the patient; and
a second stimulator configured to stimulate a right body portion of the patient,
wherein the patient terminal controls the first stimulator to operate when the lung capacity data of the left chest is smaller than pre-set reference lung capacity, and controls the second stimulator to operate when the lung capacity data of the right chest is smaller than the pre-set reference lung capacity.

21. The system for remotely diagnosing spine of claim 18, wherein the diagnostic data generator is further configured to generate chest asymmetry data about asymmetry of the left chest and the right chest of the patient by receiving the left chest displacement vector and the right chest displacement vector of the patient.

22. The system for remotely diagnosing spine of claim 21, wherein the diagnostic data generator is further configured to:
detect a left chest inhalation displacement vector or a left chest exhalation displacement vector corresponding to inhalation or exhalation of the patient, from among the received left chest displacement vector,
detect a right chest inhalation displacement vector or a right chest exhalation displacement vector corresponding to inhalation or exhalation of the patient, from among the received right chest displacement vector,
calculate a second vector between the left chest inhalation displacement vector or the left chest exhalation displacement vector and the right chest inhalation displacement vector or the right chest exhalation displacement vector, and
output a size of the second vector as the chest asymmetry data.

23. The system for remotely diagnosing spine of claim 22, wherein the wearable measuring device further comprises:
a first stimulator configured to stimulate a left body portion of the patient; and
a second stimulator configured to stimulate a right body portion of the patient,
wherein the patient terminal is further configured to:
control the first stimulator to operate when the chest asymmetry data is greater than a pre-set first threshold value and a size of the left chest inhalation displacement vector or the left chest exhalation displacement vector is smaller than a size of the right chest inhalation displacement vector or the right chest exhalation displacement vector, and
control the second stimulator to operate when the chest asymmetry data is greater than the pre-set first threshold value and the size of the right chest inhalation displacement vector or the right chest exhalation displacement vector is smaller than the size of the left chest inhalation displacement vector or the left chest exhalation displacement vector.

24. The system for remotely diagnosing spine of claim 23, wherein the wearable measuring device further comprises:
a third sensor unit configured to detect movement of a left waist portion of the patient; and
a fourth sensor unit configured to detect movement of a right waist portion of the patient,
wherein the first near field communication unit transmits data output from the third and fourth sensor units to the patient terminal.

25. The system for remotely diagnosing spine of claim 24, wherein the patient terminal is further configured to:
generate a left waist portion displacement vector about movement of the left waist portion of the patient based on the data output from the third sensor unit, and
generate a right waist portion displacement vector about movement of the right waist portion of the patient based on the data output from the fourth sensor unit.

26. The system for remotely diagnosing spine of claim 25, wherein the diagnostic data generator is further configured to generate waist portion asymmetry data about asymmetry between the left waist portion and the right waist portion of the patient by receiving the left waist portion displacement vector and the right waist portion displacement vector of the patient.

27. The system for remotely diagnosing spine of claim 26, wherein the diagnostic data generator is further configured to:
generate a third vector between the left waist portion displacement vector and the right waist portion displacement vector, and
output a size of the third vector as the waist portion asymmetry data.

28. The system for remotely diagnosing spine of claim 27, wherein the patient terminal is further configured to:
control the first stimulator to operate when the waist portion asymmetry data is greater than a pre-set second threshold value and a size of the left waist portion displacement vector is smaller than a size of the right waist portion displacement vector, and
control the second stimulator to operate when the waist portion asymmetry data is greater than the pre-set second threshold value and the size of the right waist portion displacement vector is smaller than the size of the left waist portion displacement vector.

29. The system for remotely diagnosing spine of claim 25, wherein the diagnostic data generator is further configured to generate left or right trunk balance data about balance between the left or right chest and the left or right waist portion by receiving the left or right chest displacement vector and the left or right waist portion displacement vector of the patient.

30. The system for remotely diagnosing spine of claim 29, wherein the diagnostic data generator is further configured to calculate a fourth vector between the left or right chest displacement vector and the left or right waist portion displacement vector, and output the fourth vector as the left or right trunk balance data.

31. The system for remotely diagnosing spine of claim 30, wherein the patient terminal is further configured to:
control the first stimulator to operate when the left trunk balance data is outside a pre-set reference vector range, and
control the second stimulator to operate when the right trunk balance data is outside the pre-set reference vector range.

32. The system for remotely diagnosing spine of claim 26, wherein the patient terminal comprises:
a second near field communication unit configured to exchange data with the wearable measuring device;
an input unit configured to receive a command for driving the patient terminal;
a memory unit configured to store data received from the wearable measuring device and information about the wearable measuring device;
a processor configured to generate spine health information indicating a spine health state of the patient based on the trunk movement data of the patient; and
a display unit configured to display the spine health information of the patient.

33. The system for remotely diagnosing spine of claim 32, wherein the input unit is further configured to receive a command for executing a posture measuring function from the patient,
the processor is further configured to receive data from the wearable measuring device through the second near field communication unit for a pre-set time, generate the trunk movement data of the patient based on the received data, and invoke spine health state data corresponding to the trunk movement data in preparation for spine health state instruction data stored in the memory unit, and
the display unit is further configured to display the invoked spine health state data.

34. The system for remotely diagnosing spine of claim 33, wherein the spine health state instruction data comprises the trunk movement data and the spine health state data matched to the trunk movement data.

35. The system for remotely diagnosing spine of claim 32, wherein the input unit is further configured to receive at least one of a training time, a stimulation cycle, and a repetition cycle, and
the processor is further configured to:
transmit a control signal to the wearable measuring device according to the training time such that the wearable measuring device is activated for the training time,
transmit a control signal to the wearable measuring device such that duration of stimulation applied by the first and second stimulators changes according to the stimulation cycle, and
transmit a control signal to the wearable measuring device according to the repetition cycle such that the wearable measuring device is repeatedly activated according to the repetition cycle.

36. The system for remotely diagnosing spine of claim 32, wherein the processor is further configured to calculate a spine score of the patient based on the trunk movement data, and
the display unit is further configured to display the calculated spine score.

37. The system for remotely diagnosing spine of claim 36, wherein the processor is further configured to calculate the spine score such that the spine score is high when the lung capacity data is large, and is high when the trunk asymmetry data is small.

38. The system for remotely diagnosing spine of claim 37, wherein the processor is further configured to:
calculate lung capacity data of the left and right chests by adding the lung capacity data of the left chest and the lung capacity data of the right chest,
calculate the trunk asymmetry data by adding the chest asymmetry data and the waist portion asymmetry data, and
calculate the spine score by dividing the lung capacity data of the left and right chests by the trunk asymmetry data.

39. A wearable measuring device comprising:
a first stretch sensor unit configured to detect movement of a left trunk of a patient;
a second stretch sensor unit configured to detect movement of a right trunk of the patient; and
a first near field communication unit configured to transmit data output from the first and second stretch sensor units to a patient terminal.

40. The wearable measuring device of claim 39, wherein the first stretch sensor unit is provided at a portion of an object worn on the patient, which interacts with the left trunk, and
the second stretch sensor unit is provided at a portion of the object worn on the patient, which interacts with the right trunk.

41. The wearable measuring device of claim 40, wherein the first and second stretch sensor units are installed at a chest band surrounding a chest on a top worn by the patient.

42. The wearable measuring device of claim 41, wherein the first stretch sensor unit is further configured to output, to the first near field communication unit, first stretching amount data indicating a left chest stretching amount according to movement of a left chest of the patient, and
the second stretch sensor unit is further configured to output, to the first near field communication unit, second stretching amount data indicating a right chest stretching amount according to movement of a right chest of the patient.

43. The wearable measuring device of claim 42, wherein the patient terminal is configured to obtain the left chest stretching amount based on the first stretching amount data and obtain the right chest stretching amount based on the second stretching amount data.

44. The wearable measuring device of claim 43, wherein the wearable measuring device further comprises:
a first stimulator configured to stimulate a left body portion of the patient; and
a second stimulator configured to stimulate a right boy portion of the patient,
wherein the patient terminal is further configured to:
control the first stimulate to operate when the left chest stretching amount is smaller than a pre-set reference chest stretching amount, and
control the second stimulator to operate when the right chest stretching amount is smaller than the pre-set reference chest stretching amount.

45. The wearable measuring device of claim 43, wherein the wearable device further comprises:
a first stimulator configured to stimulate a left body portion of the patient; and
a second stimulator configured to stimulate a right body portion of the patient,
wherein the patient terminal is further configured to:
calculate a first difference between the left chest stretching amount and the right chest stretching amount,
control the first stimulator to operate when the calculated first difference is greater than a pre-set first reference value and the left chest stretching amount is smaller than the right chest stretching amount, and
control the second stimulator to operate when the calculated first difference is greater than the pre-set first reference value and the right chest stretching amount is smaller than the left chest stretching amount.

46. The wearable measuring device of claim 43, further comprising:
a third stretch sensor unit configured to detect movement of a left waist portion of the patient;
a fourth stretch sensor unit configured to detect movement of a right waist portion of the patient; and
an auxiliary near field communication unit configured to transmit data output from the third and fourth stretch sensor units to the patient terminal.

47. The wearable measuring device of claim 46, wherein the third stretch sensor unit is provided at a portion of the object worn on the patient, which interacts with the left waist portion, and
a fourth stretch sensor unit is provided at a portion of the object worn on the patient, which interacts with the right waist portion.

48. The wearable measuring device of claim 47, wherein the third and fourth stretch sensor units are installed at a waist band surrounding a waist portion on clothing worn by the patient.

49. The wearable measuring device of claim 48, wherein the third stretch sensor unit is further configured to output, to the auxiliary near field communication unit, third stretching amount data indicating a left waist portion stretching amount by movement of the left waist portion of the patient, and
the fourth stretch sensor unit is further configured to output, to the auxiliary near field communication unit, fourth stretching amount data indicating a right waist portion stretching amount by movement of the right waist portion of the patient.

50. The wearable measuring device of claim 49, wherein the patient terminal is further configured to obtain the left waist portion stretching amount based on the third stretching amount data, and obtain the right waist portion stretching amount based on the fourth stretching amount data.

51. The wearable measuring device of claim 50, further comprising:
a first stimulator configured to stimulate a left body portion of the patient; and
a second stimulator configured to stimulate a right body portion of the patient,
wherein the patient terminal is further configured to:
calculate a second difference between the left waist portion stretching amount and the right waist portion stretching amount,
control the first stimulator to operate when the calculated second difference is greater than a pre-set second reference value and the left waist portion stretching amount is smaller than the right waist portion stretching amount, and
control the second stimulator to operate when the calculated second difference is greater than the pre-set second reference value and the right waist portion stretching amount is smaller than the left waist portion stretching amount.

52. The wearable measuring device of claim 50, further comprising:
a first stimulator configured to stimulate a left body portion of the patient; and
a second stimulator configured to stimulate a right body portion of the patient,
wherein the patient terminal is further configured to:
calculate a third difference between the left chest stretching amount the left waist portion stretching amount,
control the first stimulator to operate when the calculated third difference is outside a pre-set reference range,
calculate a fourth difference between the right chest stretching amount and the right waist portion stretching amount, and
control the second stimulator to operate when the calculated fourth difference is outside the pre-set reference range.

53. An application for executing a spine diagnosing method comprising:
receiving, from a wearable measuring device, first stretching amount data indicating a left chest stretching amount of a patient and second stretching amount data indicating a right chest stretching amount of the patient;
obtaining the left and right chest stretching amounts respectively from the first and second stretching amount data;
controlling a first stimulator of the wearable measuring device to operate when the left chest stretching amount is smaller than a pre-set reference chest stretching amount, the first simulator configured to stimulate a left body portion of the patient; and
controlling a second stimulator of the wearable measuring device to operate when the right chest stretching amount is smaller than the pre-set reference chest stretching amount, the second stimulator configured to stimulate a right body portion of the patient.

54. The application of claim 53, wherein the spine diagnosing method further comprises:
calculating a first difference between the left chest stretching amount and the right chest stretching amount;
controlling the first stimulator to operate when the calculated first difference is greater than a pre-set first reference value and the left chest stretching amount is smaller than the right chest stretching amount; and
controlling the second stimulator to operate when the calculated first difference is greater than the pre-set first reference value and the right chest stretching amount is smaller than the left chest stretching amount.

55. The application of claim 53, wherein the spine diagnosing method further comprises:
receiving, from the wearable measuring device, third stretching amount data indicating a left waist portion stretching amount of the patient and fourth stretching amount data indicating a right waist portion stretching amount of the patient;
obtaining the left and right waist portion stretching amounts respectively from the third and fourth stretching amount data;
calculating a second difference between the left waist portion stretching amount and the right waist portion stretching amount;
controlling the first stimulator to operate when the calculated second difference is greater than a pre-set second reference value and the left waist portion stretching amount is smaller than the right waist portion stretching amount; and
controlling the second stimulator to operate when the calculated second difference is greater than the pre-set second reference value and the right waist portion stretching amount is smaller than the left waist portion stretching amount.

56. The application of claim 55, wherein the spine diagnosing method further comprises:
calculating a third difference between the left chest stretching amount and the left waist portion stretching amount;
controlling the first stimulator to operate when the calculated third difference is outside a pre-set reference range;
calculating a fourth difference between the right chest stretching amount and the right waist portion stretching amount; and
controlling the second stimulator to operate when the calculated fourth difference is outside the pre-set reference range.
